# EUROPEAN PATENT APPLICATION

(11) **EP 3 339 450 A2**
(43) Date of publication of application: **27.06.2018**
(21) Application number: 18150727.8
(22) Date of filing: 07.03.2014
(51) Int. Cl.: C12Q 1/6886

(54) **MOLECULAR MARKERS IN BLADDER CANCER**

(30) Priority: 08.03.2013 WO PCT/EP2013/054777
(62) Divisional of application: 14708310.9
(71) Applicant: MDxHealth Research B.V., 6525 GA Nijmegen (NL)
(72) Inventor: Smit, Franciscus Petrus, 6546 RN Nijmegen (NL); Hessels, Daphne, 6546 RN Nijmegen (NL); Schalken, Jacobus A., 6524 LH Nijmegen (NL)
(74) Representative: van Kooij, Adriaan

(57) **Abstract**

The present invention relates methods for establishing the presence, or absence, of a bladder tumour and/or classification of the tumor according to the aggressiveness and/or establishing the prediction of prognosis and disease outcome for a human individual suffering from bladder cancer. Specifically, the present invention relates to methods for establishing the presence, or absence, of a bladder tumour in a human individual comprising: determining the expression of one or more genes chosen from the group consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and transcript cluster 2526896 in a biological sample (tissue or bodyfluid) originating from said human individual; establishing up regulation of expression of said one or more genes as compared to expression of said respective one or more genes in a sample originating from said human individual not comprising tumour cells or tissue.

SEQUENCE LISTING

<110> MDxHealth Research B.V.

<120> MOLECULAR MARKERS IN BLADDER CANCER

<130> 4/2WL16/11A

<141> 2014-03-07

<150> PCT/EP2013/054777
<151> 2013-03-08

<160> 34

<170> BiSSAP 1.2

<210> 1
<211> 2175
<212> DNA
<213> Homo sapiens

<220>
<221> source
<222> 1..2175
<223> /mol_type="unassigned DNA"
      /note="INHBA"
      /organism="Homo sapiens"

<400> 1
agtacagtat aaaacttcac agtgccaata ccatgaagag gagctcagac agctcttacc      60

acatgataca agagccggct ggtggaagag tggggaccag aaagagaatt tgctgaagag     120

gagaaggaaa aaaaaaacac caaaaaaaaa aataaaaaaa tccacacaca caaaaaaacc     180

tgcgcgtgag gggggaggaa aagcagggcc ttttaaaaag gcaatcacaa caacttttgc     240

tgccaggatg cccttgcttt ggctgagagg atttctgttg gcaagttgct ggattatagt     300

gaggagttcc cccaccccag gatccgaggg gcacagcgcg gcccccgact gtccgtcctg     360

tgcgctggcc gccctcccaa aggatgtacc caactctcag ccagagatgg tggaggccgt     420

caagaagcac attttaaaca tgctgcactt gaagaagaga cccgatgtca cccagccggt     480

acccaaggcg gcgcttctga acgcgatcag aaagcttcat gtgggcaaag tcggggagaa     540

cgggtatgtg gagatagagg atgacattgg aaggagggca gaaatgaatg aacttatgga     600

gcagacctcg gagatcatca cgtttgccga gtcaggaaca gccaggaaga cgctgcactt     660

cgagatttcc aaggaaggca gtgacctgtc agtggtggag cgtgcagaag tctggctctt     720

cctaaaagtc cccaaggcca acaggaccag gaccaaagtc accatccgcc tcttccagca     780

gcagaagcac ccgcagggca gcttggacac aggggaagag gccgaggaag tgggcttaaa     840

gggggagagg agtgaactgt tgctctctga aaaagtagta gacgctcgga agagcacctg     900

gcatgtcttc cctgtctcca gcagcatcca gcggttgctg gaccagggca agagctccct     960

ggacgttcgg attgcctgtg agcagtgcca ggagagtggc gccagcttgg ttctcctggg    1020

caagaagaag aagaaagaag aggaggggga agggaaaaag aagggcggag gtgaaggtgg 1080

ggcaggagca gatgaggaaa aggagcagtc gcacagacct ttcctcatgc tgcaggcccg 1140

gcagtctgaa gaccaccctc atcgccggcg tcggcggggc ttggagtgtg atggcaaggt 1200

caacatctgc tgtaagaaac agttctttgt cagtttcaag gacatcggct ggaatgactg 1260

gatcattgct ccctctggct atcatgccaa ctactgcgag ggtgagtgcc cgagccatat 1320

agcaggcacg tccgggtcct cactgtcctt ccactcaaca gtcatcaacc actaccgcat 1380

gcggggccat agcccctttg ccaacctcaa atcgtgctgt gtgcccacca agctgagacc 1440

catgtccatg ttgtactatg atgatggtca aaacatcatc aaaaaggaca ttcagaacat 1500

gatcgtggag gagtgtgggt gctcatagag ttgcccagcc cagggggaaa gggagcaaga 1560

gttgtccaga gaagacagtg gcaaaatgaa gaaattttta aggtttctga gttaaccaga 1620

aaaatagaaa ttaaaaacaa aacaaaaaaa aaaacaaaaa aaaacaaaag taaattaaaa 1680

acaaaacctg atgaaacaga tgaaggaaga tgtggaaaaa atccttagcc agggctcaga 1740

gatgaagcag tgaaagagac aggaattggg agggaaaggg agaatggtgt accctttatt 1800

tcttctgaaa tcacactgat gacatcagtt gtttaaacgg ggtattgtcc tttcccccct 1860

tgaggttccc ttgtgagcct tgaatcaacc aatctagtct gcagtagtgt ggactagaac 1920

aacccaaata gcatctagaa agccatgagt ttgaaagggc ccatcacagg cactttccta 1980

cccaattacc caggtcataa ggtatgtctg tgtgacactt atctctgtgt atatcagcat 2040

acacacacac acacacacac acacacacac acacaggcat ttccacacat tacatatata 2100

cacatactgg taaaagaaca atcgtgtgca ggtggtcaca cttccttttt ctgtaccact 2160

tttgcaacaa aacaa 2175

<210> 2
<211> 426
<212> PRT
<213> Homo sapiens

<400> 2
Met Pro Leu Leu Trp Leu Arg Gly Phe Leu Leu Ala Ser Cys Trp Ile
1               5                   10                  15
Ile Val Arg Ser Ser Pro Thr Pro Gly Ser Glu Gly His Ser Ala Ala
            20                  25                  30
Pro Asp Cys Pro Ser Cys Ala Leu Ala Ala Leu Pro Lys Asp Val Pro
        35                  40                  45
Asn Ser Gln Pro Glu Met Val Glu Ala Val Lys Lys His Ile Leu Asn
    50                  55                  60
Met Leu His Leu Lys Lys Arg Pro Asp Val Thr Gln Pro Val Pro Lys
65                  70                  75                  80
Ala Ala Leu Leu Asn Ala Ile Arg Lys Leu His Val Gly Lys Val Gly
                85                  90                  95
Glu Asn Gly Tyr Val Glu Ile Glu Asp Asp Ile Gly Arg Arg Ala Glu
            100                 105                 110

Met Asn Glu Leu Met Glu Gln Thr Ser Glu Ile Ile Thr Phe Ala Glu
        115                 120                 125
Ser Gly Thr Ala Arg Lys Thr Leu His Phe Glu Ile Ser Lys Glu Gly
    130                 135                 140
Ser Asp Leu Ser Val Val Glu Arg Ala Glu Val Trp Leu Phe Leu Lys
145                 150                 155                 160
Val Pro Lys Ala Asn Arg Thr Arg Thr Lys Val Thr Ile Arg Leu Phe
                165                 170                 175
Gln Gln Gln Lys His Pro Gln Gly Ser Leu Asp Thr Gly Glu Glu Ala
            180                 185                 190
Glu Glu Val Gly Leu Lys Gly Glu Arg Ser Glu Leu Leu Leu Ser Glu
        195                 200                 205
Lys Val Val Asp Ala Arg Lys Ser Thr Trp His Val Phe Pro Val Ser
    210                 215                 220
Ser Ser Ile Gln Arg Leu Leu Asp Gln Gly Lys Ser Ser Leu Asp Val
225                 230                 235                 240
Arg Ile Ala Cys Glu Gln Cys Gln Glu Ser Gly Ala Ser Leu Val Leu
                245                 250                 255
Leu Gly Lys Lys Lys Lys Lys Glu Glu Glu Gly Glu Gly Lys Lys Lys
            260                 265                 270
Gly Gly Gly Glu Gly Gly Ala Gly Ala Asp Glu Glu Lys Glu Gln Ser
        275                 280                 285
His Arg Pro Phe Leu Met Leu Gln Ala Arg Gln Ser Glu Asp His Pro
    290                 295                 300
His Arg Arg Arg Arg Arg Gly Leu Glu Cys Asp Gly Lys Val Asn Ile
305                 310                 315                 320
Cys Cys Lys Lys Gln Phe Phe Val Ser Phe Lys Asp Ile Gly Trp Asn
                325                 330                 335
Asp Trp Ile Ile Ala Pro Ser Gly Tyr His Ala Asn Tyr Cys Glu Gly
            340                 345                 350
Glu Cys Pro Ser His Ile Ala Gly Thr Ser Gly Ser Ser Leu Ser Phe
        355                 360                 365
His Ser Thr Val Ile Asn His Tyr Arg Met Arg Gly His Ser Pro Phe
    370                 375                 380
Ala Asn Leu Lys Ser Cys Cys Val Pro Thr Lys Leu Arg Pro Met Ser
385                 390                 395                 400
Met Leu Tyr Tyr Asp Asp Gly Gln Asn Ile Ile Lys Lys Asp Ile Gln
                405                 410                 415
Asn Met Ile Val Glu Glu Cys Gly Cys Ser
            420                 425

<210> 3
<211> 1279
<212> DNA
<213> Homo sapiens

<220>
<221> source
<222> 1..1279
<223> /mol_type="unassigned DNA"
      /organism="Homo sapiens"

<400> 3
gggagggaga gaggcgcgcg ggtgaaaggc gcattgatgc agcctgcggc ggcctcggag      60

cgcggcggag ccagacgctg accacgttcc tctcctcggt ctcctccgcc tccagctccg     120

cgctgcccgg cagccgggag ccatgcgacc ccagggcccc gccgcctccc cgcagcggct     180

ccgcggcctc ctgctgctcc tgctgctgca gctgcccgcg ccgtcgagcg cctctgagat     240

ccccaagggg aagcaaaagg cgcagctccg gcagagggag gtggtggacc tgtataatgg     300

aatgtgctta caagggccag caggagtgcc tggtcgagac gggagccctg gggccaatgg 360

cattccgggt acacctggga tcccaggtcg ggatggattc aaaggagaaa agggggaatg 420

tctgagggaa agctttgagg agtcctggac acccaactac aagcagtgtt catggagttc 480

attgaattat ggcatagatc ttgggaaaat tgcggagtgt acatttacaa agatgcgttc 540

aaatagtgct ctaagagttt tgttcagtgg ctcacttcgg ctaaaatgca gaaatgcatg 600

ctgtcagcgt tggtatttca cattcaatgg agctgaatgt tcaggacctc ttcccattga 660

agctataatt tatttggacc aaggaagccc tgaaatgaat tcaacaatta atattcatcg 720

cacttcttct gtggaaggac tttgtgaagg aattggtgct ggattagtgg atgttgctat 780

ctgggttggt acttgttcag attacccaaa aggagatgct tctactggat ggaattcagt 840

ttctcgcatc attattgaag aactaccaaa ataaatgctt taattttcat ttgctacctc 900

tttttttatt atgccttgga atggttcact taaatgacat tttaaataag tttatgtata 960

catctgaatg aaaagcaaag ctaaatatgt ttacagacca aagtgtgatt tcacactgtt 1020

tttaaatcta gcattattca ttttgcttca atcaaaagtg gtttcaatat tttttttagt 1080

tggttagaat actttcttca tagtcacatt ctctcaacct ataatttgga atattgttgt 1140

ggtcttttgt tttttctctt agtatagcat ttttaaaaaa atataaaagc taccaatctt 1200

tgtacaattt gtaaatgtta agaatttttt ttatatctgt taaataaaaa ttatttccaa 1260

caaccttaat atctttaaa 1279

<210> 4
<211> 243
<212> PRT
<213> Homo sapiens

<400> 4
Met Arg Pro Gln Gly Pro Ala Ala Ser Pro Gln Arg Leu Arg Gly Leu
1               5                   10                  15
Leu Leu Leu Leu Leu Leu Gln Leu Pro Ala Pro Ser Ser Ala Ser Glu
            20                  25                  30
Ile Pro Lys Gly Lys Gln Lys Ala Gln Leu Arg Gln Arg Glu Val Val
        35                  40                  45
Asp Leu Tyr Asn Gly Met Cys Leu Gln Gly Pro Ala Gly Val Pro Gly
    50                  55                  60
Arg Asp Gly Ser Pro Gly Ala Asn Gly Ile Pro Gly Thr Pro Gly Ile
65                  70                  75                  80
Pro Gly Arg Asp Gly Phe Lys Gly Glu Lys Gly Glu Cys Leu Arg Glu
                85                  90                  95
Ser Phe Glu Glu Ser Trp Thr Pro Asn Tyr Lys Gln Cys Ser Trp Ser
            100                 105                 110
Ser Leu Asn Tyr Gly Ile Asp Leu Gly Lys Ile Ala Glu Cys Thr Phe
        115                 120                 125
Thr Lys Met Arg Ser Asn Ser Ala Leu Arg Val Leu Phe Ser Gly Ser
    130                 135                 140
Leu Arg Leu Lys Cys Arg Asn Ala Cys Cys Gln Arg Trp Tyr Phe Thr
145                 150                 155                 160
Phe Asn Gly Ala Glu Cys Ser Gly Pro Leu Pro Ile Glu Ala Ile Ile

165                 170                 175
Tyr Leu Asp Gln Gly Ser Pro Glu Met Asn Ser Thr Ile Asn Ile His
            180                 185                 190
Arg Thr Ser Ser Val Glu Gly Leu Cys Glu Gly Ile Gly Ala Gly Leu
        195                 200                 205
Val Asp Val Ala Ile Trp Val Gly Thr Cys Ser Asp Tyr Pro Lys Gly
    210                 215                 220
Asp Ala Ser Thr Gly Trp Asn Ser Val Ser Arg Ile Ile Ile Glu Glu
225                 230                 235                 240
Leu Pro Lys

<210> 5
<211> 1867
<212> DNA
<213> Homo sapiens

<220>
<221> source
<222> 1..1867
<223> /mol_type="unassigned DNA"
      /organism="Homo sapiens"

<400> 5
cacatagctc agttcccata aaagggctgg tttgccgcgt cggggagtgg agtgggacag      60

gtatataaag gaagtacagg gcctggggaa gaggccctgt ctaggtagct ggcaccagga     120

gccgtgggca agggaagagg ccacaccctg ccctgctctg ctgcagccag aatgggtgtg     180

aaggcgtctc aaacaggctt tgtggtcctg gtgctgctcc agtgctgctc tgcatacaaa     240

ctggtctgct actacaccag ctggtcccag taccgggaag gcgatgggag ctgcttccca     300

gatgcccttg accgcttcct ctgtacccac atcatctaca gctttgccaa tataagcaac     360

gatcacatcg acacctggga gtggaatgat gtgacgctct acggcatgct caacacactc     420

aagaacagga accccaacct gaagactctc ttgtctgtcg gaggatggaa ctttgggtct     480

caaagatttt ccaagatagc ctccaacacc cagagtcgcc ggactttcat caagtcagta     540

ccgccatttc tgcgcaccca tggctttgat gggctggacc ttgcctggct ctaccctgga     600

cggagagaca aacagcattt taccacccta atcaaggaaa tgaaggccga atttataaag     660

gaagcccagc cagggaaaaa gcagctcctg ctcagcgcag cactgtctgc ggggaaggtc     720

accattgaca gcagctatga cattgccaag atatcccaac acctggattt cattagcatc     780

atgacctacg attttcatgg agcctggcgt gggaccacag gccatcacag tcccctgttc     840

cgaggtcagg aggatgcaag tcctgacaga ttcagcaaca ctgactatgc tgtggggtac     900

atgttgaggc tgggggctcc tgccagtaag ctggtgatgg gcatccccac cttcgggagg     960

agcttcactc tggcttcttc tgagactggt gttggagccc caatctcagg accgggaatt    1020

ccaggccggt tcaccaagga ggcagggacc cttgcctact atgagatctg tgacttcctc    1080

cgcggagcca cagtccatag aatcctcggc cagcaggtcc cctatgccac caagggcaac    1140

cagtgggtag gatacgacga ccaggaaagc gtcaaaagca aggtgcagta cctgaaggac    1200

aggcagctgg cgggcgccat ggtatgggcc ctggacctgg atgacttcca gggctccttc 1260

tgcggccagg atctgcgctt ccctctcacc aatgccatca aggatgcact cgctgcaacg 1320

tagccctctg ttctgcacac agcacggggg ccaaggatgc cccgtccccc tctggctcca 1380

gctggccggg agcctgatca cctgccctgc tgagtcccag gctgagcctc agtctccctc 1440

ccttggggcc tatgcagagg tccacaacac acagatttga gctcagccct ggtgggcaga 1500

gaggtaggga tggggctgtg gggatagtga ggcatcgcaa tgtaagactc gggattagta 1560

cacacttgtt gattaatgga aatgtttaca gatccccaag cctggcaagg gaatttcttc 1620

aactccctgc cccccagccc tccttatcaa aggacaccat tttggcaagc tctatcacca 1680

aggagccaaa catcctacaa gacacagtga ccatactaat tataccccct gcaaagccca 1740

gcttgaaacc ttcacttagg aacgtaatcg tgtcccctat cctacttccc cttcctaatt 1800

ccacagctgc tcaataaagt acaagagctt aacagtgaaa aaaaaaaaaa aaaaaaaaaa 1860

aaaaaaa 1867

<210> 6
<211> 383
<212> PRT
<213> Homo sapiens

<400> 6
Met Gly Val Lys Ala Ser Gln Thr Gly Phe Val Val Leu Val Leu Leu
1               5                   10                  15
Gln Cys Cys Ser Ala Tyr Lys Leu Val Cys Tyr Tyr Thr Ser Trp Ser
            20                  25                  30
Gln Tyr Arg Glu Gly Asp Gly Ser Cys Phe Pro Asp Ala Leu Asp Arg
        35                  40                  45
Phe Leu Cys Thr His Ile Ile Tyr Ser Phe Ala Asn Ile Ser Asn Asp
    50                  55                  60
His Ile Asp Thr Trp Glu Trp Asn Asp Val Thr Leu Tyr Gly Met Leu
65                  70                  75                  80
Asn Thr Leu Lys Asn Arg Asn Pro Asn Leu Lys Thr Leu Leu Ser Val
                85                  90                  95
Gly Gly Trp Asn Phe Gly Ser Gln Arg Phe Ser Lys Ile Ala Ser Asn
            100                 105                 110
Thr Gln Ser Arg Arg Thr Phe Ile Lys Ser Val Pro Pro Phe Leu Arg
        115                 120                 125
Thr His Gly Phe Asp Gly Leu Asp Leu Ala Trp Leu Tyr Pro Gly Arg
    130                 135                 140
Arg Asp Lys Gln His Phe Thr Thr Leu Ile Lys Glu Met Lys Ala Glu
145                 150                 155                 160
Phe Ile Lys Glu Ala Gln Pro Gly Lys Lys Gln Leu Leu Leu Ser Ala
                165                 170                 175
Ala Leu Ser Ala Gly Lys Val Thr Ile Asp Ser Ser Tyr Asp Ile Ala
            180                 185                 190
Lys Ile Ser Gln His Leu Asp Phe Ile Ser Ile Met Thr Tyr Asp Phe
        195                 200                 205
His Gly Ala Trp Arg Gly Thr Thr Gly His His Ser Pro Leu Phe Arg
    210                 215                 220
Gly Gln Glu Asp Ala Ser Pro Asp Arg Phe Ser Asn Thr Asp Tyr Ala
225                 230                 235                 240

Val Gly Tyr Met Leu Arg Leu Gly Ala Pro Ala Ser Lys Leu Val Met
            245                 250                 255
Gly Ile Pro Thr Phe Gly Arg Ser Phe Thr Leu Ala Ser Ser Glu Thr
            260                 265                 270
Gly Val Gly Ala Pro Ile Ser Gly Pro Gly Ile Pro Gly Arg Phe Thr
        275                 280                 285
Lys Glu Ala Gly Thr Leu Ala Tyr Tyr Glu Ile Cys Asp Phe Leu Arg
    290                 295                 300
Gly Ala Thr Val His Arg Ile Leu Gly Gln Gln Val Pro Tyr Ala Thr
305                 310                 315                 320
Lys Gly Asn Gln Trp Val Gly Tyr Asp Asp Gln Glu Ser Val Lys Ser
                325                 330                 335
Lys Val Gln Tyr Leu Lys Asp Arg Gln Leu Ala Gly Ala Met Val Trp
            340                 345                 350
Ala Leu Asp Leu Asp Asp Phe Gln Gly Ser Phe Cys Gly Gln Asp Leu
        355                 360                 365
Arg Phe Pro Leu Thr Asn Ala Ile Lys Asp Ala Leu Ala Ala Thr
    370                 375                 380

<210> 7
<211> 3307
<212> DNA
<213> Homo sapiens

<220>
<221> source
<222> 1..3307
<223> /mol_type="unassigned DNA"
      /organism="Homo sapiens"

<400> 7
caccttctgc actgctcatc tgggcagagg aagcttcaga aagctgccaa ggcaccatct      60

ccaggaactc ccagcacgca gaatccatct gagaatatgc tgccacaaat accctttttg     120

ctgctagtat ccttgaactt ggttcatgga gtgttttacg ctgaacgata ccaaatgccc     180

acaggcataa aaggcccact acccaacacc aagacacagt tcttcattcc ctacaccata     240

aagagtaaag gtatagcagt aagaggagag caaggtactc ctggtccacc aggccctgct     300

ggacctcgag ggcacccagg tccttctgga ccaccaggaa aaccaggcta cggaagtcct     360

ggactccaag gagagccagg gttgccagga ccaccgggac catcagctgt agggaaacca     420

ggtgtgccag gactcccagg aaaaccagga gagagaggac catatggacc aaaaggagat     480

gttggaccag ctggcctacc aggaccccgg ggcccaccag gaccacctgg aatccctgga     540

ccggctggaa tttctgtgcc aggaaaacct ggacaacagg gacccacagg agccccagga     600

cccaggggct ttcctggaga aaagggtgca ccaggagtcc ctggtatgaa tggacagaaa     660

ggggaaatgg gatatggtgc tcctggtcgt ccaggtgaga ggggtcttcc aggccctcag     720

ggtcccacag gaccatctgg ccctcctgga gtgggaaaaa gaggtgaaaa tggggttcca     780

ggacagccag gcatcaaagg tgatagaggt tttccgggag aaatgggacc aattggccca     840

ccaggtcccc aaggccctcc tggggaacga gggccagaag gcattggaaa gccaggagct     900

gctggagccc caggccagcc agggattcca ggaacaaaag gtctccctgg ggctccagga     960

atagctgggc ccccagggcc tcctggcttt gggaaaccag gcttgccagg cctgaaggga 1020

gaaagaggac ctgctggcct tcctgggggt ccaggtgcca aaggggaaca agggccagca 1080

ggtcttcctg ggaagccagg tctgactgga ccccctggga atatgggacc ccaaggacca 1140

aaaggcatcc cgggtagcca tggtctccca ggccctaaag gtgagacagg gccagctggg 1200

cctgcaggat accctggggc taagggtgaa aggggttccc ctgggtcaga tggaaaacca 1260

gggtacccag gaaaaccagg tctcgatggt cctaagggta acccagggtt accaggtcca 1320

aaaggtgatc ctggagttgg aggacctcct ggtctcccag gccctgtggg cccagcagga 1380

gcaaagggaa tgcccggaca caatggagag gctggcccaa gaggtgcccc tggaatacca 1440

ggtactagag gccctattgg gccaccaggc attccaggat tccctgggtc taaaggggat 1500

ccaggaagtc ccggtcctcc tggcccagct ggcatagcaa ctaagggcct caatggaccc 1560

accgggccac cagggcctcc aggtccaaga ggccactctg gagagcctgg tcttccaggg 1620

ccccctgggc ctccaggccc accaggtcaa gcagtcatgc ctgagggttt tataaaggca 1680

ggccaaaggc ccagtctttc tgggacccct cttgttagtg ccaaccaggg ggtaacagga 1740

atgcctgtgt ctgcttttac tgttattctc tccaaagctt acccagcaat aggaactccc 1800

ataccatttg ataaaatttt gtataacagg caacagcatt atgacccaag gactggaatc 1860

tttacttgtc agataccagg aatatactat ttttcatacc acgtgcatgt gaaagggact 1920

catgtttggg taggcctgta taagaatggc acccctgtaa tgtacaccta tgatgaatac 1980

accaaaggct acctggatca ggcttcaggg agtgccatca tcgatctcac agaaaatgac 2040

caggtgtggc tccagcttcc caatgccgag tcaaatggcc tatactcctc tgagtatgtc 2100

cactcctctt tctcaggatt cctagtggct ccaatgtgag tacacacaga gctaatctaa 2160

atcttgtgct agaaaaagca ttctctaact ctaccccacc ctacaaaatg catatggagg 2220

taggctgaaa agaatgtaat ttttattttc tgaaatacag atttgagcta tcagaccaac 2280

aaaccttccc cctgaaaagt gagcagcaac gtaaaaacgt atgtgaagcc tctcttgaat 2340

ttctagttag caatcttaag gctctttaag gttttctcca atattaaaaa atatcaccaa 2400

agaagtcctg ctatgttaaa aacaaacaac aaaaaacaaa caacaaaaaa aaaattaaaa 2460

aaaaaaacag aaatagagct ctaagttatg tgaaatttga tttgagaaac tcggcatttc 2520

ctttttaaaa aagcctgttt ctaactatga atatgagaac ttctaggaaa catccaggag 2580

gtatcatata actttgtaga acttaaatac ttgaatattc aaatttaaaa gacactgtat 2640

cccctaaaat atttctgatg gtgcactact ctgaggcctg tatggcccct ttcatcaata 2700

tctattcaaa tatacaggtg catatatact tgttaaagct cttatataaa aaagccccaa 2760

aatattgaag ttcatctgaa atgcaaggtg ctttcatcaa tgaacctttt caaacttttc 2820

tatgattgca gagaagcttt ttatataccc agcataactt ggaaacaggt atctgaccta 2880

ttcttattta gttaacacaa gtgtgattaa tttgatttct ttaattcctt attgaatctt 2940

atgtgatatg attttctgga tttacagaac attagcacat gtaccttgtg cctcccattc 3000

aagtgaagtt ataatttaca ctgagggttt caaaattcga ctagaagtgg agatatatta 3060

tttatttatg cactgtactg tatttttata ttgctgttta aaacttttaa gctgtgcctc 3120

acttattaaa gcacaaaatg ttttacctac tccttattta cgacgcaata aaataacatc 3180

aatagatttt taggctgaat taatttgaaa gcagcaattt gctgttctca accattcttt 3240

caaggctttt cattgttcaa agttaataaa aaagtaggac aataaagtga aaaaaaaaaa 3300

aaaaaaa 3307

<210> 8
<211> 680
<212> PRT
<213> Homo sapiens

<400> 8

<210> 9
<211> 2788
<212> DNA
<213> Homo sapiens

<220>
<221> source
<222> 1..2788
<223> /mol_type="unassigned DNA"
      /organism="Homo sapiens"

<400> 9
   aagaacgccc ccaaaatctg tttctaattt tacagaaatc ttttgaaact tggcacggta       60
   ttcaaaagtc cgtggaaaga aaaaaacctt gtcctggctt cagcttccaa ctacaaagac 120
   agacttggtc cttttcaacg gttttcacag atccagtgac ccacgctctg aagacagaat 180
   tagctaactt tcaaaaacat ctggaaaaat gaagacttgg gtaaaaatcg tatttggagt 240
   tgccacctct gctgtgcttg ccttattggt gatgtgcatt gtcttacgcc cttcaagagt 300
   tcataactct gaagaaaata caatgagagc actcacactg aaggatattt taaatggaac 360
   attttcttat aaaacatttt ttccaaactg gatttcagga caagaatatc ttcatcaatc 420
   tgcagataac aatatagtac tttataatat tgaaacagga caatcatata ccattttgag 480
   taatagaacc atgaaaagtg tgaatgcttc aaattacggc ttatcacctg atcggcaatt 540
   tgtatatcta gaaagtgatt attcaaagct ttggagatac tcttacacag caacatatta 600
   catctatgac cttagcaatg gagaatttgt aagaggaaat gagcttcctc gtccaattca 660
   gtatttatgc tggtcgcctg ttgggagtaa attagcatat gtctatcaaa acaatatcta 720
   tttgaaacaa agaccaggag atccaccttt tcaaataaca tttaatggaa gagaaaataa 780
   aatatttaat ggaatcccag actgggttta tgaagaggaa atgcttgcta caaaatatgc 840
   tctctggtgg tctcctaatg gaaaattttt ggcatatgcg gaatttaatg atacggatat 900
   accagttatt gcctattcct attatggcga tgaacaatat cctagaacaa taaatattcc 960
   atacccaaag gctggagcta agaatcccgt tgttcggata tttattatcg ataccactta 1020
   ccctgcgtat gtaggtcccc aggaagtgcc tgttccagca atgatagcct caagtgatta 1080
   ttatttcagt tggctcacgt gggttactga tgaacgagta tgtttgcagt ggctaaaaag 1140
   agtccagaat gtttcggtcc tgtctatatg tgacttcagg gaagactggc agacatggga 1200
   ttgtccaaag acccaggagc atatagaaga aagcagaact ggatgggctg gtggattctt 1260
   tgtttcaaca ccagttttca gctatgatgc catttcgtac tacaaaatat ttagtgacaa 1320
   ggatggctac aaacatattc actatatcaa agacactgtg gaaaatgcta ttcaaattac 1380
   aagtggcaag tgggaggcca taaatatatt cagagtaaca caggattcac tgttttattc 1440
   tagcaatgaa tttgaagaat accctggaag aagaaacatc tacagaatta gcattggaag 1500
   ctatcctcca agcaagaagt gtgttacttg ccatctaagg aaagaaaggt gccaatatta 1560
   cacagcaagt ttcagcgact acgccaagta ctatgcactt gtctgctacg gcccaggcat 1620
   ccccatttcc acccttcatg atggacgcac tgatcaagaa attaaaatcc tggaagaaaa 1680
   caaggaattg gaaaatgctt tgaaaaatat ccagctgcct aaagaggaaa ttaagaaact 1740
   tgaagtagat gaaattactt tatggtacaa gatgattctt cctcctcaat ttgacagatc 1800
   aaagaagtat cccttgctaa ttcaagtgta tggtggtccc tgcagtcaga gtgtaaggtc 1860
   tgtatttgct gttaattgga tatcttatct tgcaagtaag gaagggatgg tcattgcctt 1920
   ggtggatggt cgaggaacag ctttccaagg tgacaaactc ctctatgcag tgtatcgaaa 1980
   gctgggtgtt tatgaagttg aagaccagat tacagctgtc agaaaattca tagaaatggg 2040
   tttcattgat gaaaaaagaa tagccatatg gggctggtcc tatggaggat acgtttcatc 2100
   actggccctt gcatctggaa ctggtctttt caaatgtggt atagcagtgg ctccagtctc 2160
   cagctgggaa tattacgcgt ctgtctacac agagagattc atgggtctcc caacaaagga 2220
   tgataatctt gagcactata agaattcaac tgtgatggca agagcagaat atttcagaaa 2280
   tgtagactat cttctcatcc acggaacagc agatgataat gtgcactttc aaaactcagc 2340
   acagattgct aaagctctgg ttaatgcaca agtggatttc caggcaatgt ggtactctga 2400
   ccagaaccac ggcttatccg gcctgtccac gaaccactta tacacccaca tgacccactt 2460
   cctaaagcag tgtttctctt tgtcagacta aaaacgatgc agatgcaagc ctgtatcaga 2520
   atctgaaaac cttatataaa cccctcagac agtttgctta ttttattttt tatgttgtaa 2580
   aatgctagta taaacaaaca aattaatgtt gttctaaagg ctgttaaaaa aaagatgagg 2640
   actcagaagt tcaagctaaa tattgtttac attttctggt actctgtgaa agaagagaaa 2700
   agggagtcat gcattttgct ttggacacag tgttttatca cctgttcatt tgaagaaaaa 2760
   taataaagtc agaagttcaa aaaaaaaa 2788

<210> 10
<211> 760
<212> PRT
<213> Homo sapiens

<400> 10
Met Lys Thr Trp Val Lys Ile Val Phe Gly Val Ala Thr Ser Ala Val
1               5                   10                  15
Leu Ala Leu Leu Val Met Cys Ile Val Leu Arg Pro Ser Arg Val His
            20                  25                  30
Asn Ser Glu Glu Asn Thr Met Arg Ala Leu Thr Leu Lys Asp Ile Leu
        35                  40                  45
Asn Gly Thr Phe Ser Tyr Lys Thr Phe Phe Pro Asn Trp Ile Ser Gly
    50                  55                  60
Gln Glu Tyr Leu His Gln Ser Ala Asp Asn Asn Ile Val Leu Tyr Asn
65                  70                  75                  80
Ile Glu Thr Gly Gln Ser Tyr Thr Ile Leu Ser Asn Arg Thr Met Lys
                85                  90                  95
Ser Val Asn Ala Ser Asn Tyr Gly Leu Ser Pro Asp Arg Gln Phe Val
            100                 105                 110
Tyr Leu Glu Ser Asp Tyr Ser Lys Leu Trp Arg Tyr Ser Tyr Thr Ala
        115                 120                 125
Thr Tyr Tyr Ile Tyr Asp Leu Ser Asn Gly Glu Phe Val Arg Gly Asn
    130                 135                 140
Glu Leu Pro Arg Pro Ile Gln Tyr Leu Cys Trp Ser Pro Val Gly Ser
145                 150                 155                 160
Lys Leu Ala Tyr Val Tyr Gln Asn Asn Ile Tyr Leu Lys Gln Arg Pro
                165                 170                 175
Gly Asp Pro Pro Phe Gln Ile Thr Phe Asn Gly Arg Glu Asn Lys Ile
            180                 185                 190
Phe Asn Gly Ile Pro Asp Trp Val Tyr Glu Glu Glu Met Leu Ala Thr
        195                 200                 205

Lys Tyr Ala Leu Trp Trp Ser Pro Asn Gly Lys Phe Leu Ala Tyr Ala
    210                 215                 220
Glu Phe Asn Asp Thr Asp Ile Pro Val Ile Ala Tyr Ser Tyr Tyr Gly
225                 230                 235                 240
Asp Glu Gln Tyr Pro Arg Thr Ile Asn Ile Pro Tyr Pro Lys Ala Gly
                245                 250                 255
Ala Lys Asn Pro Val Val Arg Ile Phe Ile Ile Asp Thr Thr Tyr Pro
            260                 265                 270
Ala Tyr Val Gly Pro Gln Glu Val Pro Val Pro Ala Met Ile Ala Ser
        275                 280                 285
Ser Asp Tyr Tyr Phe Ser Trp Leu Thr Trp Val Thr Asp Glu Arg Val
    290                 295                 300
Cys Leu Gln Trp Leu Lys Arg Val Gln Asn Val Ser Val Leu Ser Ile
305                 310                 315                 320
Cys Asp Phe Arg Glu Asp Trp Gln Thr Trp Asp Cys Pro Lys Thr Gln
                325                 330                 335
Glu His Ile Glu Glu Ser Arg Thr Gly Trp Ala Gly Gly Phe Phe Val
            340                 345                 350
Ser Thr Pro Val Phe Ser Tyr Asp Ala Ile Ser Tyr Tyr Lys Ile Phe
        355                 360                 365
Ser Asp Lys Asp Gly Tyr Lys His Ile His Tyr Ile Lys Asp Thr Val
    370                 375                 380
Glu Asn Ala Ile Gln Ile Thr Ser Gly Lys Trp Glu Ala Ile Asn Ile
385                 390                 395                 400
Phe Arg Val Thr Gln Asp Ser Leu Phe Tyr Ser Ser Asn Glu Phe Glu
                405                 410                 415
Glu Tyr Pro Gly Arg Arg Asn Ile Tyr Arg Ile Ser Ile Gly Ser Tyr
            420                 425                 430
Pro Pro Ser Lys Lys Cys Val Thr Cys His Leu Arg Lys Glu Arg Cys
        435                 440                 445
Gln Tyr Tyr Thr Ala Ser Phe Ser Asp Tyr Ala Lys Tyr Tyr Ala Leu
    450                 455                 460
Val Cys Tyr Gly Pro Gly Ile Pro Ile Ser Thr Leu His Asp Gly Arg
465                 470                 475                 480
Thr Asp Gln Glu Ile Lys Ile Leu Glu Glu Asn Lys Glu Leu Glu Asn
                485                 490                 495
Ala Leu Lys Asn Ile Gln Leu Pro Lys Glu Glu Ile Lys Lys Leu Glu
            500                 505                 510
Val Asp Glu Ile Thr Leu Trp Tyr Lys Met Ile Leu Pro Pro Gln Phe
        515                 520                 525
Asp Arg Ser Lys Lys Tyr Pro Leu Leu Ile Gln Val Tyr Gly Gly Pro
    530                 535                 540
Cys Ser Gln Ser Val Arg Ser Val Phe Ala Val Asn Trp Ile Ser Tyr
545                 550                 555                 560
Leu Ala Ser Lys Glu Gly Met Val Ile Ala Leu Val Asp Gly Arg Gly
                565                 570                 575
Thr Ala Phe Gln Gly Asp Lys Leu Leu Tyr Ala Val Tyr Arg Lys Leu
            580                 585                 590
Gly Val Tyr Glu Val Glu Asp Gln Ile Thr Ala Val Arg Lys Phe Ile
        595                 600                 605
Glu Met Gly Phe Ile Asp Glu Lys Arg Ile Ala Ile Trp Gly Trp Ser
    610                 615                 620
Tyr Gly Gly Tyr Val Ser Ser Leu Ala Leu Ala Ser Gly Thr Gly Leu
625                 630                 635                 640
Phe Lys Cys Gly Ile Ala Val Ala Pro Val Ser Ser Trp Glu Tyr Tyr
                645                 650                 655
Ala Ser Val Tyr Thr Glu Arg Phe Met Gly Leu Pro Thr Lys Asp Asp
            660                 665                 670
Asn Leu Glu His Tyr Lys Asn Ser Thr Val Met Ala Arg Ala Glu Tyr
        675                 680                 685
Phe Arg Asn Val Asp Tyr Leu Leu Ile His Gly Thr Ala Asp Asp Asn
    690                 695                 700
Val His Phe Gln Asn Ser Ala Gln Ile Ala Lys Ala Leu Val Asn Ala

705                 710                 715                 720
Gln Val Asp Phe Gln Ala Met Trp Tyr Ser Asp Gln Asn His Gly Leu
                725                 730                 735
Ser Gly Leu Ser Thr Asn His Leu Tyr Thr His Met Thr His Phe Leu
            740                 745                 750
Lys Gln Cys Phe Ser Leu Ser Asp
        755                 760

<210> 11
<211> 307
<212> DNA
<213> Homo sapiens

<220>
<221> source
<222> 1..307
<223> /mol_type="unassigned DNA"
      /organism="Homo sapiens"

<400> 11
tggtactgca cccatagata ctgtcacctc tactctttct tccaatcacc attagcagat      60

gccacaggat tcctacttct gaaagttttt gggccctgca ggtggaagac tggagaagcc     120

aataaagttt aaggctacat tttattccat ccacaaattt ggtgaaggag gaaatgttta     180

caattctgcc atgccatgaa taggagtttt ccaccgggtg tacactgctg ttaacaaggt     240

gtaaatactt gtccagtaaa gagaccgtac ggtactgctg atggacgtcc caacacaatg     300

ccagatg                                                               307


<210> 12
<211> 2541
<212> DNA
<213> Homo sapiens

<220>
<221> source
<222> 1..2541
<223> /mol_type="unassigned DNA"
      /organism="Homo sapiens"

<400> 12
gatacaaaca gtgatggaaa acacatgagc agtaacaagt tttaatcttg ctcctcagta      60

ctaacatgga ctaatctgtg ggagcagttt attccagtat cacccagggt gcagccacac     120

caggactgtg ttgaagggtg ttttttttct tttaaatgta atacctcctc atcttttctt     180

cttacacagt gtctgagaac atttacatta tagataagta gtacatggtg gataacttct     240

acttttagga ggactactct cttctgacag tcctagactg gtcttctaca ctaagacacc     300

atgaaggagt atgtgctcct attattcctg gctttgtgct ctgccaaacc cttctttagc     360

ccttcacaca tcgcactgaa gaatatgatg ctgaaggata tggaagacac agatgatgat     420

gatgatgatg atgatgatga tgatgatgat gaggacaact ctctttttcc aacaagagag     480

ccaagaagcc atttttttcc atttgatctg tttccaatgt gtccatttgg atgtcagtgc     540

tattcacgag ttgtacattg ctcagattta ggtttgacct cagtcccaac caacattcca 600

tttgatactc gaatgcttga tcttcaaaac aataaaatta aggaaatcaa agaaaatgat 660

tttaaaggac tcacttcact ttatggtctg atcctgaaca acaacaagct aacgaagatt 720

cacccaaaag cctttctaac cacaaagaag ttgcgaaggc tgtatctgtc ccacaatcaa 780

ctaagtgaaa taccacttaa tcttcccaaa tcattagcag aactcagaat tcatgaaaat 840

aaagttaaga aaatacaaaa ggacacattc aaaggaatga atgctttaca cgttttggaa 900

atgagtgcaa accctcttga taataatggg atagagccag gggcatttga aggggtgacg 960

gtgttccata tcagaattgc agaagcaaaa ctgacctcag ttcctaaagg cttaccacca 1020

actttattgg agcttcactt agattataat aaaatttcaa cagtggaact tgaggatttt 1080

aaacgataca aagaactaca aaggctgggc ctaggaaaca acaaaatcac agatatcgaa 1140

aatgggagtc ttgctaacat accacgtgtg agagaaatac atttggaaaa caataaacta 1200

aaaaaaatcc cttcaggatt accagagttg aaatacctcc agataatctt ccttcattct 1260

aattcaattg caagagtggg agtaaatgac ttctgtccaa cagtgccaaa gatgaagaaa 1320

tctttataca gtgcaataag tttattcaac aacccggtga aatactggga aatgcaacct 1380

gcaacatttc gttgtgtttt gagcagaatg agtgttcagc ttgggaactt tggaatgtaa 1440

taattagtaa ttggtaatgt ccatttaata taagattcaa aaatccctac atttggaata 1500

cttgaactct attaataatg gtagtattat atatacaagc aaatatctat tctcaagtgg 1560

taagtccact gacttatttt atgacaagaa atttcaacgg aattttgcca aactattgat 1620

acataagggt tgagagaaac aagcatctat tgcagtttct ttttgcgtac aaatgatctt 1680

acataaatct catgcttgac cattcctttc ttcataacaa aaaagtaaga tattcggtat 1740

ttaacacttt gttatcaagc atattttaaa aagaactgta ctgtaaatgg aatgcttgac 1800

ttagcaaaat ttgtgctctt tcatttgctg ttagaaaaac agaattaaca aagacagtaa 1860

tgtgaagagt gcattacact attcttattc tttagtaact tgggtagtac tgtaatattt 1920

ttaatcatct taaagtatga tttgatataa tcttattgaa attaccttat catgtcttag 1980

agcccgtctt tatgtttaaa actaatttct taaaataaag ccttcagtaa atgttcatta 2040

ccaacttgat aaatgctact cataagagct ggtttggggc tatagcatat gctttttttt 2100

ttttaattat tacctgattt aaaaatctct gtaaaaacgt gtagtgtttc ataaaatctg 2160

taactcgcat tttaatgatc cgctattata agcttttaat agcatgaaaa ttgttaggct 2220

atataacatt gccacttcaa ctctaaggaa tatttttgag atatcccttt ggaagacctt 2280

gcttggaaga gcctggacac taacaattct acaccaaatt gtctcttcaa atacgtatgg 2340

actggataac tctgagaaac acatctagta taactgaata agcagagcat caaattaaac 2400

agacagaaac cgaaagctct atataaatgc tcagagttct ttatgtattt cttattggca 2460

ttcaacatat gtaaaatcag aaaacaggga aattttcatt aaaaatattg gtttgaaata 2520

aaaaaaaaaa aaaaaaaaaa a 2541

<210> 13
<211> 379
<212> PRT
<213> Homo sapiens

<400> 13
Met Lys Glu Tyr Val Leu Leu Leu Phe Leu Ala Leu Cys Ser Ala Lys
1               5                   10                  15
Pro Phe Phe Ser Pro Ser His Ile Ala Leu Lys Asn Met Met Leu Lys
            20                  25                  30
Asp Met Glu Asp Thr Asp Asp Asp Asp Asp Asp Asp Asp Asp Asp Asp
        35                  40                  45
Asp Asp Glu Asp Asn Ser Leu Phe Pro Thr Arg Glu Pro Arg Ser His
    50                  55                  60
Phe Phe Pro Phe Asp Leu Phe Pro Met Cys Pro Phe Gly Cys Gln Cys
65                  70                  75                  80
Tyr Ser Arg Val Val His Cys Ser Asp Leu Gly Leu Thr Ser Val Pro
                85                  90                  95
Thr Asn Ile Pro Phe Asp Thr Arg Met Leu Asp Leu Gln Asn Asn Lys
            100                 105                 110
Ile Lys Glu Ile Lys Glu Asn Asp Phe Lys Gly Leu Thr Ser Leu Tyr
        115                 120                 125
Gly Leu Ile Leu Asn Asn Asn Lys Leu Thr Lys Ile His Pro Lys Ala
    130                 135                 140
Phe Leu Thr Thr Lys Lys Leu Arg Arg Leu Tyr Leu Ser His Asn Gln
145                 150                 155                 160
Leu Ser Glu Ile Pro Leu Asn Leu Pro Lys Ser Leu Ala Glu Leu Arg
                165                 170                 175
Ile His Glu Asn Lys Val Lys Lys Ile Gln Lys Asp Thr Phe Lys Gly
            180                 185                 190
Met Asn Ala Leu His Val Leu Glu Met Ser Ala Asn Pro Leu Asp Asn
        195                 200                 205
Asn Gly Ile Glu Pro Gly Ala Phe Glu Gly Val Thr Val Phe His Ile
    210                 215                 220
Arg Ile Ala Glu Ala Lys Leu Thr Ser Val Pro Lys Gly Leu Pro Pro
225                 230                 235                 240
Thr Leu Leu Glu Leu His Leu Asp Tyr Asn Lys Ile Ser Thr Val Glu
                245                 250                 255
Leu Glu Asp Phe Lys Arg Tyr Lys Glu Leu Gln Arg Leu Gly Leu Gly
            260                 265                 270
Asn Asn Lys Ile Thr Asp Ile Glu Asn Gly Ser Leu Ala Asn Ile Pro
        275                 280                 285
Arg Val Arg Glu Ile His Leu Glu Asn Asn Lys Leu Lys Lys Ile Pro
    290                 295                 300
Ser Gly Leu Pro Glu Leu Lys Tyr Leu Gln Ile Ile Phe Leu His Ser
305                 310                 315                 320
Asn Ser Ile Ala Arg Val Gly Val Asn Asp Phe Cys Pro Thr Val Pro
                325                 330                 335
Lys Met Lys Lys Ser Leu Tyr Ser Ala Ile Ser Leu Phe Asn Asn Pro
            340                 345                 350
Val Lys Tyr Trp Glu Met Gln Pro Ala Thr Phe Arg Cys Val Leu Ser
        355                 360                 365
Arg Met Ser Val Gln Leu Gly Asn Phe Gly Met
    370                 375

<210> 14

<211> 608
<212> DNA
<213> Homo sapiens

<220>
<221> source
<222> 1..608
<223> /mol_type="unassigned DNA"
      /organism="Homo sapiens"

<400> 14
agaggtataa aaatcagata gctcagcact tcgctccaaa ggagctgaag gagagaggat      60

tccactgcgt gtatgttttc tctttcacgt ctcacttttg ttctctgcca tcaggaaatg     120

cagtagacat ttgtcaccag cagctctttg gagacttcaa tgttccttcc tcccaggatc     180

cgaaagggga cttcagacac ggagaatcta agacaccagg agctcagaag aatggagtct     240

caagacatca ggcaaatacc gaagagatca gagccaagtt ctaaagggca gccatttccc     300

aaatgtcaac caagagcgaa gccaagagaa ttatgatttc ctggctatac aatgacagga     360

aaagcttgat caaagctgga gtgcaagtta aaaagtgtaa ggtactcaag tgctagggtc     420

tgtgtccaag cccaatcaat tgatcattag ttgtttttaa cttcaacgtt tcttttgcaa     480

cagcacctct tccttaaagt agcaatctta gcagactgcc accttacatg atgcatctta     540

ttctccaagg acaatgaaga ctgaactatc gcacattacc taagaaagat gggaattgac     600

atgcacat                                                              608


<210> 15
<211> 4955
<212> DNA
<213> Homo sapiens

<220>
<221> source
<222> 1..4955
<223> /mol_type="unassigned DNA"
      /organism="Homo sapiens"

<400> 15
gcttaaaaaa gcacagggag atcgcgggca gctttgcagt cgctgccttc tcgcgcctga      60

ccatgcaccc ctgcatcttc ctgctgggcc acaggcgagc gctttatttc tggagctgag     120

ggctaaaact tttttgactt ttcttctcct caacatctga atcatgccat gtgcccagag     180

gagctggctt gcaaaccttt ccgtggtggc tcagctcctt aactttgggg cgctttgcta     240

tgggagacag cctcagccag gcccggttcg cttcccggac aggaggcaag agcattttat     300

caagggcctg ccagaatacc acgtggtggg tccagtccga gtagatgcca gtgggcattt     360

tttgtcatat ggcttgcact atcccatcac gagcagcagg aggaagagag atttggatgg     420

ctcagaggac tgggtgtact acagaatttc tcacgaggag aaggacctgt tttttaactt     480

gacggtcaat caaggatttc tttccaatag ctacatcatg gagaagagat atgggaacct     540

ctcccatgtt aagatgatgg cttcctctgc ccccctctgc catctcagtg gcacggttct 600

acagcagggc accagagttg ggacggcagc cctcagtgcc tgccatggac tgactggatt 660

tttccaacta ccacatggag actttttcat tgaacccgtg aagaagcatc cactggttga 720

gggagggtac cacccgcaca tcgtttacag gaggcagaaa gttccagaaa ccaaggagcc 780

aacctgtgga ttaaaggaca gtgttaacat ctcccagaag caagagctat ggcgggagaa 840

gtgggagagg cacaacttgc caagcagaag cctctctcgg cgttccatca gcaaggagag 900

atgggtggag acactggtgg tggccgacac aaagatgatt gaataccatg ggagtgagaa 960

tgtggagtcc tacatcctca ccatcatgaa catggtcact gggttgttcc ataacccaag 1020

cattggcaat gcaattcaca ttgttgtggt tcggctcatt ctactcgaag aagaagagca 1080

aggactgaaa atagttcacc atgcagaaaa gacactgtct agcttctgca agtggcagaa 1140

gagtatcaat cccaagagtg acctcaatcc tgttcatcac gacgtggctg tccttctcac 1200

cagaaaggac atctgtgctg gtttcaatcg cccctgcgag accctgggcc tgtctcacct 1260

ttcaggaatg tgtcagcctc accgcagttg taacatcaat gaagattcgg gactccctct 1320

ggctttcaca attgcccatg agctaggaca cagcttcggc atccagcatg atgggaaaga 1380

aaatgactgt gagcctgtgg gcagacatcc gtacatcatg tcccgccagc tccagtacga 1440

tcccactccg ctgacatggt ccaagtgcag cgaggagtac atcacccgct tcttggaccg 1500

aggctggggg ttctgtcttg atgacatacc taaaaagaaa ggcttgaagt ccaaggtcat 1560

tgcccccgga gtgatctatg atgttcacca ccagtgccag ctacaatatg gacccaatgc 1620

taccttctgc caggaagtag aaaacgtctg ccagacactg tggtgctccg tgaagggctt 1680

ttgtcgctct aagctggacg ctgctgcaga tggaactcaa tgtggtgaga agaagtggtg 1740

tatggcaggc aagtgcatca cagtggggaa gaaaccagag agcattcctg gaggctgggg 1800

ccgctggtca ccctggtccc actgttccag gacctgtggg gctggagtcc agagcgcaga 1860

gaggctctgc aacaaccccg agccaaagtt tggagggaaa tattgcactg gagaaagaaa 1920

acgctatcgc ttgtgcaacg tccacccctg tcgctcagag gcaccaacat ttcggcagat 1980

gcagtgcagt gaatttgaca ctgttcccta caagaatgaa ctctaccact ggtttcccat 2040

ttttaaccca gcacatcctt gtgagctcta ctgccgaccc atagatggcc agttttctga 2100

gaaaatgctg gatgctgtca ttgatggtac cccttgcttt gaaggcggca acagcagaaa 2160

tgtctgtatt aatggcatat gtaagatggt tggctgtgac tatgagatcg attccaatgc 2220

caccgaggat cgctgcggtg tgtgcctggg agatggctct tcctgccaga ctgtgagaaa 2280

gatgtttaag cagaaggaag gatctggtta tgttgacatt gggctcattc caaaaggagc 2340

aagggacata agagtgatgg aaattgaggg agctggaaac ttcctggcca tcaggagtga 2400

agatcctgaa aaatattacc tgaatggagg gtttattatc cagtggaacg ggaactataa 2460

gctggcaggg actgtctttc agtatgacag gaaaggagac ctggaaaagc tgatggccac 2520

aggtcccacc aatgagtctg tgtggatcca gcttctattc caggtgacta accctggcat 2580

caagtatgag tacacaatcc agaaagatgg ccttgacaat gatgttgagc agcagatgta 2640

cttctggcag tacggccact ggacagagtg cagtgtgacc tgcgggacag gtatccgccg 2700

ccaaactgcc cattgcataa agaagggccg cgggatggtg aaagctacat tctgtgaccc 2760

agaaacacag cccaatggga gacagaagaa gtgccatgaa aaggcttgtc cacccaggtg 2820

gtgggcaggg gagtgggaag catgctcggc gacatgcggg ccccacgggg agaagaagcg 2880

aaccgtgctg tgcatccaga ccatggtctc tgacgagcag gctctcccgc ccacagactg 2940

ccagcacctg ctgaagccca agaccctcct ttcctgcaac agagacatcc tgtgcccctc 3000

ggactggaca gtgggcaact ggagtgagtg ttctgtttcc tgtggtggtg gagtgcggat 3060

tcgcagtgtc acatgtgcca agaaccatga tgaaccttgc gatgtgacaa ggaaacccaa 3120

cagccgagct ctgtgtggcc tccagcaatg cccttctagc cggagagttc tgaaaccaaa 3180

caaaggcact atttccaatg gaaaaaaccc accaacacta aagcccgtcc ctccacctac 3240

atccaggccc agaatgctga ccacacccac agggcctgag tctatgagca caagcactcc 3300

agcaatcagc agccctagtc ctaccacagc ctccaaagaa ggagacctgg gtgggaaaca 3360

gtggcaagat agctcaaccc aacctgagct gagctctcgc tatctcattt ccactggaag 3420

cacttcccag cccatcctca cttcccaatc cttgagcatt cagccaagtg aggaaaatgt 3480

ttccagttca gatactggtc ctacctcgga gggaggcctt gtagctacaa caacaagtgg 3540

ttctggcttg tcatcttccc gcaaccctat cacttggcct gtgactccat tttacaatac 3600

cttgaccaaa ggtccagaaa tggagattca cagtggctca ggggaagaaa gagaacagcc 3660

tgaggacaaa gatgaaagca atcctgtaat atggaccaag atcagagtac ctggaaatga 3720

cgctccagtg gaaagtacag aaatgccact tgcacctcca ctaacaccag atctcagcag 3780

ggagtcctgg tggccaccct tcagcacagt aatggaagga ctgctcccca gccaaaggcc 3840

cactacttcc gaaactggga cacccagagt tgaggggatg gttactgaaa agccagccaa 3900

cactctgctc cctctgggag gagaccacca gccagaaccc tcaggaaaga cggcaaaccg 3960

taaccacctg aaacttccaa acaacatgaa ccaaacaaaa agttctgaac cagtcctgac 4020

tgaggaggat gcaacaagtc tgattactga gggctttttg ctaaatgcct ccaattacaa 4080

gcagctcaca aacggccacg gctctgcaca ctggatcgtc ggaaactgga gcgagtgctc 4140

caccacatgt ggcctggggg cctactggag aagggtggag tgcagcaccc agatggattc 4200

tgactgtgcg gccatccaga gacctgaccc tgcaaaaaga tgccacctcc gtccctgtgc 4260

tggctggaaa gtgggaaact ggagcaagtg ctccagaaac tgcagtgggg gcttcaagat 4320

acgcgagatt cagtgcgtgg acagccggga ccaccggaac ctgaggccat ttcactgcca 4380

gttcctggcc ggcattcctc ccccattgag catgagctgt aacccggagc cctgtgaggc 4440

gtggcaggtg gagccttgga gccagtgctc caggtcctgt ggaggtggag ttcaggagag 4500

aggagtgttc tgtccaggag gcctctgtga ttggacaaaa agacccacat ccaccatgtc 4560

ttgcaatgag cacctgtgct gtcactgggc cactgggaac tgggacctgt gttccacttc 4620

ctgtggaggt ggctttcaga agaggactgt ccaatgtgtg ccctcagagg gcaataaaac 4680

tgaagaccaa gaccaatgtc tatgtgatca caaacccaga cctccagaat tcaaaaaatg 4740

caaccagcag gcctgcaaga aaagtgccga tttactttgc actaaggaca aactgtcagc 4800

cagtttctgc cagacactga aagccatgaa gaaatgttct gtgcccaccg tgagggctga 4860

gtgctgcttc tcgtgtcccc agacacacat cacacacacc caaaggcaaa gaaggcaacg 4920

gttgctccaa aagtcaaaag aactctaagc ccaaa 4955

<210> 16
<211> 1594
<212> PRT
<213> Homo sapiens

<400> 16
Met Pro Cys Ala Gln Arg Ser Trp Leu Ala Asn Leu Ser Val Val Ala
1               5                   10                  15
Gln Leu Leu Asn Phe Gly Ala Leu Cys Tyr Gly Arg Gln Pro Gln Pro
            20                  25                  30
Gly Pro Val Arg Phe Pro Asp Arg Arg Gln Glu His Phe Ile Lys Gly
        35                  40                  45
Leu Pro Glu Tyr His Val Val Gly Pro Val Arg Val Asp Ala Ser Gly
    50                  55                  60
His Phe Leu Ser Tyr Gly Leu His Tyr Pro Ile Thr Ser Ser Arg Arg
65                  70                  75                  80
Lys Arg Asp Leu Asp Gly Ser Glu Asp Trp Val Tyr Tyr Arg Ile Ser
                85                  90                  95
His Glu Glu Lys Asp Leu Phe Phe Asn Leu Thr Val Asn Gln Gly Phe
            100                 105                 110
Leu Ser Asn Ser Tyr Ile Met Glu Lys Arg Tyr Gly Asn Leu Ser His
        115                 120                 125
Val Lys Met Met Ala Ser Ser Ala Pro Leu Cys His Leu Ser Gly Thr
    130                 135                 140
Val Leu Gln Gln Gly Thr Arg Val Gly Thr Ala Ala Leu Ser Ala Cys
145                 150                 155                 160
His Gly Leu Thr Gly Phe Phe Gln Leu Pro His Gly Asp Phe Phe Ile
                165                 170                 175
Glu Pro Val Lys Lys His Pro Leu Val Glu Gly Gly Tyr His Pro His
            180                 185                 190
Ile Val Tyr Arg Arg Gln Lys Val Pro Glu Thr Lys Glu Pro Thr Cys
        195                 200                 205
Gly Leu Lys Asp Ser Val Asn Ile Ser Gln Lys Gln Glu Leu Trp Arg
    210                 215                 220
Glu Lys Trp Glu Arg His Asn Leu Pro Ser Arg Ser Leu Ser Arg Arg
225                 230                 235                 240
Ser Ile Ser Lys Glu Arg Trp Val Glu Thr Leu Val Val Ala Asp Thr
                245                 250                 255

Lys Met Ile Glu Tyr His Gly Ser Glu Asn Val Glu Ser Tyr Ile Leu
            260                 265                 270
Thr Ile Met Asn Met Val Thr Gly Leu Phe His Asn Pro Ser Ile Gly
        275                 280                 285
Asn Ala Ile His Ile Val Val Val Arg Leu Ile Leu Leu Glu Glu Glu
    290                 295                 300
Glu Gln Gly Leu Lys Ile Val His His Ala Glu Lys Thr Leu Ser Ser
305                 310                 315                 320
Phe Cys Lys Trp Gln Lys Ser Ile Asn Pro Lys Ser Asp Leu Asn Pro
                325                 330                 335
Val His His Asp Val Ala Val Leu Leu Thr Arg Lys Asp Ile Cys Ala
            340                 345                 350
Gly Phe Asn Arg Pro Cys Glu Thr Leu Gly Leu Ser His Leu Ser Gly
        355                 360                 365
Met Cys Gln Pro His Arg Ser Cys Asn Ile Asn Glu Asp Ser Gly Leu
    370                 375                 380
Pro Leu Ala Phe Thr Ile Ala His Glu Leu Gly His Ser Phe Gly Ile
385                 390                 395                 400
Gln His Asp Gly Lys Glu Asn Asp Cys Glu Pro Val Gly Arg His Pro
                405                 410                 415
Tyr Ile Met Ser Arg Gln Leu Gln Tyr Asp Pro Thr Pro Leu Thr Trp
            420                 425                 430
Ser Lys Cys Ser Glu Glu Tyr Ile Thr Arg Phe Leu Asp Arg Gly Trp
        435                 440                 445
Gly Phe Cys Leu Asp Asp Ile Pro Lys Lys Lys Gly Leu Lys Ser Lys
    450                 455                 460
Val Ile Ala Pro Gly Val Ile Tyr Asp Val His His Gln Cys Gln Leu
465                 470                 475                 480
Gln Tyr Gly Pro Asn Ala Thr Phe Cys Gln Glu Val Glu Asn Val Cys
                485                 490                 495
Gln Thr Leu Trp Cys Ser Val Lys Gly Phe Cys Arg Ser Lys Leu Asp
            500                 505                 510
Ala Ala Ala Asp Gly Thr Gln Cys Gly Glu Lys Lys Trp Cys Met Ala
        515                 520                 525
Gly Lys Cys Ile Thr Val Gly Lys Lys Pro Glu Ser Ile Pro Gly Gly
    530                 535                 540
Trp Gly Arg Trp Ser Pro Trp Ser His Cys Ser Arg Thr Cys Gly Ala
545                 550                 555                 560
Gly Val Gln Ser Ala Glu Arg Leu Cys Asn Asn Pro Glu Pro Lys Phe
                565                 570                 575
Gly Gly Lys Tyr Cys Thr Gly Glu Arg Lys Arg Tyr Arg Leu Cys Asn
            580                 585                 590
Val His Pro Cys Arg Ser Glu Ala Pro Thr Phe Arg Gln Met Gln Cys
        595                 600                 605
Ser Glu Phe Asp Thr Val Pro Tyr Lys Asn Glu Leu Tyr His Trp Phe
    610                 615                 620
Pro Ile Phe Asn Pro Ala His Pro Cys Glu Leu Tyr Cys Arg Pro Ile
625                 630                 635                 640
Asp Gly Gln Phe Ser Glu Lys Met Leu Asp Ala Val Ile Asp Gly Thr
                645                 650                 655
Pro Cys Phe Glu Gly Gly Asn Ser Arg Asn Val Cys Ile Asn Gly Ile
            660                 665                 670
Cys Lys Met Val Gly Cys Asp Tyr Glu Ile Asp Ser Asn Ala Thr Glu
        675                 680                 685
Asp Arg Cys Gly Val Cys Leu Gly Asp Gly Ser Ser Cys Gln Thr Val
    690                 695                 700
Arg Lys Met Phe Lys Gln Lys Glu Gly Ser Gly Tyr Val Asp Ile Gly
705                 710                 715                 720
Leu Ile Pro Lys Gly Ala Arg Asp Ile Arg Val Met Glu Ile Glu Gly
                725                 730                 735
Ala Gly Asn Phe Leu Ala Ile Arg Ser Glu Asp Pro Glu Lys Tyr Tyr
            740                 745                 750
Leu Asn Gly Gly Phe Ile Ile Gln Trp Asn Gly Asn Tyr Lys Leu Ala

755                 760                 765
Gly Thr Val Phe Gln Tyr Asp Arg Lys Gly Asp Leu Glu Lys Leu Met
    770                 775                 780
Ala Thr Gly Pro Thr Asn Glu Ser Val Trp Ile Gln Leu Leu Phe Gln
785                 790                 795                 800
Val Thr Asn Pro Gly Ile Lys Tyr Glu Tyr Thr Ile Gln Lys Asp Gly
                805                 810                 815
Leu Asp Asn Asp Val Glu Gln Gln Met Tyr Phe Trp Gln Tyr Gly His
            820                 825                 830
Trp Thr Glu Cys Ser Val Thr Cys Gly Thr Gly Ile Arg Arg Gln Thr
        835                 840                 845
Ala His Cys Ile Lys Lys Gly Arg Gly Met Val Lys Ala Thr Phe Cys
    850                 855                 860
Asp Pro Glu Thr Gln Pro Asn Gly Arg Gln Lys Lys Cys His Glu Lys
865                 870                 875                 880
Ala Cys Pro Pro Arg Trp Trp Ala Gly Glu Trp Glu Ala Cys Ser Ala
                885                 890                 895
Thr Cys Gly Pro His Gly Glu Lys Lys Arg Thr Val Leu Cys Ile Gln
            900                 905                 910
Thr Met Val Ser Asp Glu Gln Ala Leu Pro Pro Thr Asp Cys Gln His
        915                 920                 925
Leu Leu Lys Pro Lys Thr Leu Leu Ser Cys Asn Arg Asp Ile Leu Cys
    930                 935                 940
Pro Ser Asp Trp Thr Val Gly Asn Trp Ser Glu Cys Ser Val Ser Cys
945                 950                 955                 960
Gly Gly Gly Val Arg Ile Arg Ser Val Thr Cys Ala Lys Asn His Asp
                965                 970                 975
Glu Pro Cys Asp Val Thr Arg Lys Pro Asn Ser Arg Ala Leu Cys Gly
            980                 985                 990
Leu Gln Gln Cys Pro Ser Ser Arg Arg Val Leu Lys Pro Asn Lys Gly
        995                 1000                1005
Thr Ile Ser Asn Gly Lys Asn Pro Pro Thr Leu Lys Pro Val Pro Pro
    1010                1015                1020
Pro Thr Ser Arg Pro Arg Met Leu Thr Thr Pro Thr Gly Pro Glu Ser
1025                1030                1035                1040
Met Ser Thr Ser Thr Pro Ala Ile Ser Ser Pro Ser Pro Thr Thr Ala
                1045                1050                1055
Ser Lys Glu Gly Asp Leu Gly Gly Lys Gln Trp Gln Asp Ser Ser Thr
            1060                1065                1070
Gln Pro Glu Leu Ser Ser Arg Tyr Leu Ile Ser Thr Gly Ser Thr Ser
        1075                1080                1085
Gln Pro Ile Leu Thr Ser Gln Ser Leu Ser Ile Gln Pro Ser Glu Glu
    1090                1095                1100
Asn Val Ser Ser Ser Asp Thr Gly Pro Thr Ser Glu Gly Gly Leu Val
1105                1110                1115                1120
Ala Thr Thr Thr Ser Gly Ser Gly Leu Ser Ser Ser Arg Asn Pro Ile
                1125                1130                1135
Thr Trp Pro Val Thr Pro Phe Tyr Asn Thr Leu Thr Lys Gly Pro Glu
            1140                1145                1150
Met Glu Ile His Ser Gly Ser Gly Glu Glu Arg Glu Gln Pro Glu Asp
        1155                1160                1165
Lys Asp Glu Ser Asn Pro Val Ile Trp Thr Lys Ile Arg Val Pro Gly
    1170                1175                1180
Asn Asp Ala Pro Val Glu Ser Thr Glu Met Pro Leu Ala Pro Pro Leu
1185                1190                1195                1200
Thr Pro Asp Leu Ser Arg Glu Ser Trp Trp Pro Pro Phe Ser Thr Val
                1205                1210                1215
Met Glu Gly Leu Leu Pro Ser Gln Arg Pro Thr Thr Ser Glu Thr Gly
            1220                1225                1230
Thr Pro Arg Val Glu Gly Met Val Thr Glu Lys Pro Ala Asn Thr Leu
        1235                1240                1245
Leu Pro Leu Gly Gly Asp His Gln Pro Glu Pro Ser Gly Lys Thr Ala
    1250                1255                1260

Asn Arg Asn His Leu Lys Leu Pro Asn Asn Met Asn Gln Thr Lys Ser
1265                1270                1275                1280
Ser Glu Pro Val Leu Thr Glu Glu Asp Ala Thr Ser Leu Ile Thr Glu
                1285                1290                1295
Gly Phe Leu Leu Asn Ala Ser Asn Tyr Lys Gln Leu Thr Asn Gly His
            1300                1305                1310
Gly Ser Ala His Trp Ile Val Gly Asn Trp Ser Glu Cys Ser Thr Thr
        1315                1320                1325
Cys Gly Leu Gly Ala Tyr Trp Arg Arg Val Glu Cys Ser Thr Gln Met
    1330                1335                1340
Asp Ser Asp Cys Ala Ala Ile Gln Arg Pro Asp Pro Ala Lys Arg Cys
1345                1350                1355                1360
His Leu Arg Pro Cys Ala Gly Trp Lys Val Gly Asn Trp Ser Lys Cys
                1365                1370                1375
Ser Arg Asn Cys Ser Gly Gly Phe Lys Ile Arg Glu Ile Gln Cys Val
            1380                1385                1390
Asp Ser Arg Asp His Arg Asn Leu Arg Pro Phe His Cys Gln Phe Leu
        1395                1400                1405
Ala Gly Ile Pro Pro Pro Leu Ser Met Ser Cys Asn Pro Glu Pro Cys
    1410                1415                1420
Glu Ala Trp Gln Val Glu Pro Trp Ser Gln Cys Ser Arg Ser Cys Gly
1425                1430                1435                1440
Gly Gly Val Gln Glu Arg Gly Val Phe Cys Pro Gly Gly Leu Cys Asp
                1445                1450                1455
Trp Thr Lys Arg Pro Thr Ser Thr Met Ser Cys Asn Glu His Leu Cys
            1460                1465                1470
Cys His Trp Ala Thr Gly Asn Trp Asp Leu Cys Ser Thr Ser Cys Gly
        1475                1480                1485
Gly Gly Phe Gln Lys Arg Thr Val Gln Cys Val Pro Ser Glu Gly Asn
    1490                1495                1500
Lys Thr Glu Asp Gln Asp Gln Cys Leu Cys Asp His Lys Pro Arg Pro
1505                1510                1515                1520
Pro Glu Phe Lys Lys Cys Asn Gln Gln Ala Cys Lys Lys Ser Ala Asp
                1525                1530                1535
Leu Leu Cys Thr Lys Asp Lys Leu Ser Ala Ser Phe Cys Gln Thr Leu
            1540                1545                1550
Lys Ala Met Lys Lys Cys Ser Val Pro Thr Val Arg Ala Glu Cys Cys
        1555                1560                1565
Phe Ser Cys Pro Gln Thr His Ile Thr His Thr Gln Arg Gln Arg Arg
    1570                1575                1580
Gln Arg Leu Leu Gln Lys Ser Lys Glu Leu
1585                1590

<210> 17
<211> 3676
<212> DNA
<213> Homo sapiens

<220>
<221> source
<222> 1..3676
<223> /mol_type="unassigned DNA"
      /organism="Homo sapiens"

<400> 17
gagagacgag ggcagcggag gaggcgagga gcgccgggta ccgggccggg ggagccgcgg      60

gctctcgggg aagagacgga tgatgaacaa gctttacatc gggaacctga gccccgccgt     120

caccgccgac gacctccggc agctctttgg ggacaggaag ctgcccctgg cgggacaggt     180

cctgctgaag tccggctacg ccttcgtgga ctaccccgac cagaactggg ccatccgcgc     240

catcgagacc ctctcgggta aagtggaatt gcatgggaaa atcatggaag ttgattactc 300

agtctctaaa aagctaagga gcaggaaaat tcagattcga aacatccctc ctcacctgca 360

gtgggaggtg ttggatggac ttttggctca atatgggaca gtggagaatg tggaacaagt 420

caacacagac acagaaaccg ccgttgtcaa cgtcacatat gcaacaagag aagaagcaaa 480

aatagccatg gagaagctaa gcgggcatca gtttgagaac tactccttca agatttccta 540

catcccggat gaagaggtga gctccccttc gccccctcag cgagcccagc gtggggacca 600

ctcttcccgg gagcaaggcc acgcccctgg gggcacttct caggccagac agattgattt 660

cccgctgcgg atcctggtcc ccacccagtt tgttggtgcc atcatcggaa aggagggctt 720

gaccataaag aacatcacta agcagaccca gtcccgggta gatatccata gaaaagagaa 780

ctctggagct gcagagaagc ctgtcaccat ccatgccacc ccagagggga cttctgaagc 840

atgccgcatg attcttgaaa tcatgcagaa agaggcagat gagaccaaac tagccgaaga 900

gattcctctg aaaatcttgg cacacaatgg cttggttgga agactgattg gaaaagaagg 960

cagaaatttg aagaaaattg aacatgaaac agggaccaag ataacaatct catctttgca 1020

ggatttgagc atatacaacc cggaaagaac catcactgtg aagggcacag ttgaggcctg 1080

tgccagtgct gagatagaga ttatgaagaa gctgcgtgag gcctttgaaa atgatatgct 1140

ggctgttaac caacaagcca atctgatccc agggttgaac ctcagcgcac ttggcatctt 1200

ttcaacagga ctgtccgtgc tatctccacc agcagggccc cgcggagctc cccccgctgc 1260

cccctaccac cccttcacta cccactccgg atacttctcc agcctgtacc cccatcacca 1320

gtttggcccg ttcccgcatc atcactctta tccagagcag gagattgtga atctcttcat 1380

cccaacccag gctgtgggcg ccatcatcgg gaagaagggg gcacacatca aacagctggc 1440

gagattcgcc ggagcctcta tcaagattgc ccctgcggaa ggcccagacg tcagcgaaag 1500

gatggtcatc atcaccgggc caccggaagc ccagttcaag gcccagggac ggatctttgg 1560

gaaactgaaa gaggaaaact tctttaaccc caaagaagaa gtgaagctgg aagcgcatat 1620

cagagtgccc tcttccacag ctggccgggt gattggcaaa ggtggcaaga ccgtgaacga 1680

actgcagaac ttaaccagtg cagaagtcat cgtgcctcgt gaccaaacgc cagatgaaaa 1740

tgaggaagtg atcgtcagaa ttatcgggca cttctttgct agccagactg cacagcgcaa 1800

gatcagggaa attgtacaac aggtgaagca gcaggagcag aaataccctc agggagtcgc 1860

ctcacagcgc agcaagtgag gctcccacag gcaccagcaa aacaacggat gaatgtagcc 1920

cttccaacac ctgacagaat gagaccaaac gcagccagcc agatcgggag caaaccaaag 1980

accatctgag gaatgagaag tctgcggagg cggccaggga ctctgccgag gccctgagaa 2040

ccccaggggc cgaggagggg cggggaaggt cagccaggtt tgccagaacc accgagcccc 2100

gcctcccgcc ccccagggct tctgcaggct tcagccatcc acttcaccat ccactcggat 2160

ctctcctgaa ctcccacgac gctatccctt ttagttgaac taacataggt gaacgtgttc 2220

aaagccaagc aaaatgcaca ccctttttct gtggcaaatc gtctctgtac atgtgtgtac 2280

atattagaaa gggaagatgt taagatatgt ggcctgtggg ttacacaggg tgcctgcagc 2340

ggtaatatat tttagaaata atatatcaaa taactcaact aactccaatt tttaatcaat 2400

tattaatttt tttttctttt taaagagaaa gcaggctttt ctagacttta aagaataaag 2460

tctttgggag gtctcacggt gtagagagga gctttgaggc cacccgcaca aaattcaccc 2520

agagggaaat ctcgtcggaa ggacactcac ggcagttctg gatcacctgt gtatgtcaac 2580

agaagggata ccgtctcctt gaagaggaaa ctctgtcact cctcatgcct gtctagctca 2640

tacacccatt tctctttgct tcacaggttt taaactggtt ttttgcatac tgctatataa 2700

ttctctgtct ctctctgttt atctctcccc tccctcccct ccccttcttc tccatctcca 2760

ttcttttgaa tttcctcatc cctccatctc aatcccgtat ctacgcaccc cccccccccc 2820

aggcaaagca gtgctctgag tatcacatca cacaaaagga acaaaagcga aacacacaaa 2880

ccagcctcaa cttacacttg gttactcaaa agaacaagag tcaatggtac ttgtcctagc 2940

gttttggaag aggaaaacag gaacccacca aaccaaccaa tcaaccaaac aaagaaaaaa 3000

ttccacaatg aaagaatgta ttttgtcttt ttgcattttg gtgtataagc catcaatatt 3060

cagcaaaatg attcctttct ttaaaaaaaa aaaatgtgga ggaaagtaga aatttaccaa 3120

ggttgttggc ccagggcgtt aaattcacag atttttttaa cgagaaaaac acacagaaga 3180

agctacctca ggtgttttta cctcagcacc ttgctcttgt gtttccctta gagattttgt 3240

aaagctgata gttggagcat ttttttattt ttttaataaa aatgagttgg aaaaaaaata 3300

agatatcaac tgccagcctg gagaaggtga cagtccaagt gtgcaacagc tgttctgaat 3360

tgtcttccgc tagccaagaa cctatatggc cttcttttgg acaaaccttg aaaatgttta 3420

tttaaaaaaa aaaaagatga caaagaaaaa cagagagaga gaatattgga gatgtcctga 3480

attttaatag ggtacgcgcc attagggctt tttgcgctaa aggatgaaca tgtactggtt 3540

tatgtggaca agccattata ccaccagact gcaatgccag tttcctctac tgcaaacagt 3600

gttctgtgac aaaaaaaaaa aaaaaaaaaa gaaaaaaaaa gaaaaaacag aaatatatcc 3660

agctaacaag aaaaaa 3676

<210> 18
<211> 599
<212> PRT
<213> Homo sapiens

<400> 18
Met Met Asn Lys Leu Tyr Ile Gly Asn Leu Ser Pro Ala Val Thr Ala
1               5                   10                  15

Asp Asp Leu Arg Gln Leu Phe Gly Asp Arg Lys Leu Pro Leu Ala Gly
            20                  25                  30
Gln Val Leu Leu Lys Ser Gly Tyr Ala Phe Val Asp Tyr Pro Asp Gln
        35                  40                  45
Asn Trp Ala Ile Arg Ala Ile Glu Thr Leu Ser Gly Lys Val Glu Leu
    50                  55                  60
His Gly Lys Ile Met Glu Val Asp Tyr Ser Val Ser Lys Lys Leu Arg
65                  70                  75                  80
Ser Arg Lys Ile Gln Ile Arg Asn Ile Pro Pro His Leu Gln Trp Glu
                85                  90                  95
Val Leu Asp Gly Leu Leu Ala Gln Tyr Gly Thr Val Glu Asn Val Glu
            100                 105                 110
Gln Val Asn Thr Asp Thr Glu Thr Ala Val Val Asn Val Thr Tyr Ala
        115                 120                 125
Thr Arg Glu Glu Ala Lys Ile Ala Met Glu Lys Leu Ser Gly His Gln
    130                 135                 140
Phe Glu Asn Tyr Ser Phe Lys Ile Ser Tyr Ile Pro Asp Glu Glu Val
145                 150                 155                 160
Ser Ser Pro Ser Pro Pro Gln Arg Ala Gln Arg Gly Asp His Ser Ser
                165                 170                 175
Arg Glu Gln Gly His Ala Pro Gly Gly Thr Ser Gln Ala Arg Gln Ile
            180                 185                 190
Asp Phe Pro Leu Arg Ile Leu Val Pro Thr Gln Phe Val Gly Ala Ile
        195                 200                 205
Ile Gly Lys Glu Gly Leu Thr Ile Lys Asn Ile Thr Lys Gln Thr Gln
    210                 215                 220
Ser Arg Val Asp Ile His Arg Lys Glu Asn Ser Gly Ala Ala Glu Lys
225                 230                 235                 240
Pro Val Thr Ile His Ala Thr Pro Glu Gly Thr Ser Glu Ala Cys Arg
                245                 250                 255
Met Ile Leu Glu Ile Met Gln Lys Glu Ala Asp Glu Thr Lys Leu Ala
            260                 265                 270
Glu Glu Ile Pro Leu Lys Ile Leu Ala His Asn Gly Leu Val Gly Arg
        275                 280                 285
Leu Ile Gly Lys Glu Gly Arg Asn Leu Lys Lys Ile Glu His Glu Thr
    290                 295                 300
Gly Thr Lys Ile Thr Ile Ser Ser Leu Gln Asp Leu Ser Ile Tyr Asn
305                 310                 315                 320
Pro Glu Arg Thr Ile Thr Val Lys Gly Thr Val Glu Ala Cys Ala Ser
                325                 330                 335
Ala Glu Ile Glu Ile Met Lys Lys Leu Arg Glu Ala Phe Glu Asn Asp
            340                 345                 350
Met Leu Ala Val Asn Gln Gln Ala Asn Leu Ile Pro Gly Leu Asn Leu
        355                 360                 365
Ser Ala Leu Gly Ile Phe Ser Thr Gly Leu Ser Val Leu Ser Pro Pro
    370                 375                 380
Ala Gly Pro Arg Gly Ala Pro Pro Ala Ala Pro Tyr His Pro Phe Thr
385                 390                 395                 400
Thr His Ser Gly Tyr Phe Ser Ser Leu Tyr Pro His His Gln Phe Gly
                405                 410                 415
Pro Phe Pro His His His Ser Tyr Pro Glu Gln Glu Ile Val Asn Leu
            420                 425                 430
Phe Ile Pro Thr Gln Ala Val Gly Ala Ile Ile Gly Lys Lys Gly Ala
        435                 440                 445
His Ile Lys Gln Leu Ala Arg Phe Ala Gly Ala Ser Ile Lys Ile Ala
    450                 455                 460
Pro Ala Glu Gly Pro Asp Val Ser Glu Arg Met Val Ile Ile Thr Gly
465                 470                 475                 480
Pro Pro Glu Ala Gln Phe Lys Ala Gln Gly Arg Ile Phe Gly Lys Leu
                485                 490                 495
Lys Glu Glu Asn Phe Phe Asn Pro Lys Glu Glu Val Lys Leu Glu Ala
            500                 505                 510
His Ile Arg Val Pro Ser Ser Thr Ala Gly Arg Val Ile Gly Lys Gly

515                 520                 525
Gly Lys Thr Val Asn Glu Leu Gln Asn Leu Thr Ser Ala Glu Val Ile
    530                 535                 540
Val Pro Arg Asp Gln Thr Pro Asp Glu Asn Glu Glu Val Ile Val Arg
545                 550                 555                 560
Ile Ile Gly His Phe Phe Ala Ser Gln Thr Ala Gln Arg Lys Ile Arg
                565                 570                 575
Glu Ile Val Gln Gln Val Lys Gln Gln Glu Gln Lys Tyr Pro Gln Gly
            580                 585                 590
Val Ala Ser Gln Arg Ser Lys
        595

<210> 19
<211> 2418
<212> DNA
<213> Homo sapiens

<220>
<221> source
<222> 1..2418
<223> /mol_type="unassigned DNA"
      /organism="Homo sapiens"

<400> 19
gcaaacctta agctgaatga acaacttttc ttctcttgaa tatatcttaa cgccaaattt      60

tgagtgcttt tttgttaccc atcctcatat gtcccagcta gaaagaatcc tgggttggag     120

ctactgcatg ttgattgttt tgtttttcct tttggctgtt cattttggtg gctactataa     180

ggaaatctaa cacaaacagc aactgttttt tgttgtttac ttttgcatct ttacttgtgg     240

agctgtggca agtcctcata tcaaatacag aacatgatct tcctcctgct aatgttgagc     300

ctggaattgc agcttcacca gatagcagct ttattcacag tgacagtccc taaggaactg     360

tacataatag agcatggcag caatgtgacc ctggaatgca actttgacac tggaagtcat     420

gtgaaccttg gagcaataac agccagtttg caaaaggtgg aaaatgatac atccccacac     480

cgtgaaagag ccactttgct ggaggagcag ctgcccctag ggaaggcctc gttccacata     540

cctcaagtcc aagtgaggga cgaaggacag taccaatgca taatcatcta tggggtcgcc     600

tgggactaca agtacctgac tctgaaagtc aaagcttcct acaggaaaat aaacactcac     660

atcctaaagg ttccagaaac agatgaggta gagctcacct gccaggctac aggttatcct     720

ctggcagaag tatcctggcc aaacgtcagc gttcctgcca acaccagcca ctccaggacc     780

cctgaaggcc tctaccaggt caccagtgtt ctgcgcctaa agccaccccc tggcagaaac     840

ttcagctgtg tgttctggaa tactcacgtg agggaactta ctttggccag cattgacctt     900

caaagtcaga tggaacccag gacccatcca acttggctgc ttcacatttt catccccttc     960

tgcatcattg ctttcatttt catagccaca gtgatagccc taagaaaaca actctgtcaa    1020

aagctgtatt cttcaaaaga cacaacaaaa agacctgtca ccacaacaaa gagggaagtg    1080

aacagtgcta tctgaacctg tggtcttggg agccagggtg acctgatatg acatctaaag    1140

aagcttctgg actctgaaca agaattcggt ggcctgcaga gcttgccatt tgcacttttc    1200

aaatgccttt ggatgaccca gcactttaat ctgaaacctg caacaagact agccaacacc 1260

tggccatgaa acttgcccct tcactgatct ggactcacct ctggagccta tggctttaag 1320

caagcactac tgcactttac agaattaccc cactggatcc tggacccaca gaattccttc 1380

aggatccttc ttgctgccag actgaaagca aaaggaatta tttcccctca agttttctaa 1440

gtgatttcca aaagcagagg tgtgtggaaa tttccagtaa cagaaacaga tgggttgcca 1500

atagagttat tttttatcta tagcttcctc tgggtactag aagaggctat tgagactatg 1560

agctcacaga cagggcttcg cacaaactca aatcataatt gacatgtttt atggattact 1620

ggaatcttga tagcataatg aagttgttct aattaacaga gagcatttaa atatacacta 1680

agtgcacaaa ttgtggagta aagtcatcaa gctctgtttt tgaggtctaa gtcacaaagc 1740

atttgtttta acctgtaatg gcaccatgtt taatggtggt tttttttttg aactacatct 1800

ttcctttaaa aattattggt ttctttttat ttgtttttac cttagaaatc aattatatac 1860

agtcaaaaat atttgatatg ctcatacgtt gtatctgcag caatttcaga taagtagcta 1920

aaatggccaa agccccaaac taagcctcct tttctggccc tcaatatgac tttaaatttg 1980

acttttcagt gcctcagttt gcacatctgt aatacagcaa tgctaagtag tcaaggcctt 2040

tgataattgg cactatggaa atcctgcaag atcccactac atatgtgtgg agcagaaggg 2100

taactcggct acagtaacag cttaattttg ttaaatttgt tctttatact ggagccatga 2160

agctcagagc attagctgac ccttgaacta ttcaaatggg cacattagct agtataacag 2220

acttacatag gtgggcctaa agcaagctcc ttaactgagc aaaatttggg gcttatgaga 2280

atgaaagggt gtgaaattga ctaacagaca aatcatacat ctcagtttct caattctcat 2340

gtaaatcaga gaatgccttt aaagaataaa actcaattgt tattcttcaa cgttctttat 2400

atattctact tttgggta 2418

<210> 20
<211> 273
<212> PRT
<213> Homo sapiens

<400> 20
Met Ile Phe Leu Leu Leu Met Leu Ser Leu Glu Leu Gln Leu His Gln
1               5                   10                  15
Ile Ala Ala Leu Phe Thr Val Thr Val Pro Lys Glu Leu Tyr Ile Ile
            20                  25                  30
Glu His Gly Ser Asn Val Thr Leu Glu Cys Asn Phe Asp Thr Gly Ser
        35                  40                  45
His Val Asn Leu Gly Ala Ile Thr Ala Ser Leu Gln Lys Val Glu Asn
    50                  55                  60
Asp Thr Ser Pro His Arg Glu Arg Ala Thr Leu Leu Glu Glu Gln Leu
65                  70                  75                  80
Pro Leu Gly Lys Ala Ser Phe His Ile Pro Gln Val Gln Val Arg Asp
                85                  90                  95

Glu Gly Gln Tyr Gln Cys Ile Ile Ile Tyr Gly Val Ala Trp Asp Tyr
            100                 105                 110
Lys Tyr Leu Thr Leu Lys Val Lys Ala Ser Tyr Arg Lys Ile Asn Thr
        115                 120                 125
His Ile Leu Lys Val Pro Glu Thr Asp Glu Val Glu Leu Thr Cys Gln
    130                 135                 140
Ala Thr Gly Tyr Pro Leu Ala Glu Val Ser Trp Pro Asn Val Ser Val
145                 150                 155                 160
Pro Ala Asn Thr Ser His Ser Arg Thr Pro Glu Gly Leu Tyr Gln Val
                165                 170                 175
Thr Ser Val Leu Arg Leu Lys Pro Pro Pro Gly Arg Asn Phe Ser Cys
            180                 185                 190
Val Phe Trp Asn Thr His Val Arg Glu Leu Thr Leu Ala Ser Ile Asp
        195                 200                 205
Leu Gln Ser Gln Met Glu Pro Arg Thr His Pro Thr Trp Leu Leu His
    210                 215                 220
Ile Phe Ile Pro Phe Cys Ile Ile Ala Phe Ile Phe Ile Ala Thr Val
225                 230                 235                 240
Ile Ala Leu Arg Lys Gln Leu Cys Gln Lys Leu Tyr Ser Ser Lys Asp
                245                 250                 255
Thr Thr Lys Arg Pro Val Thr Thr Thr Lys Arg Glu Val Asn Ser Ala
            260                 265                 270
Ile

<210> 21
<211> 2974
<212> DNA
<213> Homo sapiens

<220>
<221> source
<222> 1..2974
<223> /mol_type="unassigned DNA"
      /organism="Homo sapiens"

<400> 21
gcggccgagg gggagcccgc gccgcggctg cagctgccaa gggagcgttc cgagcccacg      60

tcaggggagg tgtcgggata aatagggtcc cgcaatggcc gtggctggct gcgctccgag     120

ctgcggagtc cgggactgga gctgcccggg cgggttcgcg ccccgaaggc tgagagctgg     180

cgctgctcgt gccctgtgtg ccagacggcg gagctccgcg gccggacccc gcggccccgc     240

tttgctgccg actggagttt gggggaagaa actctcctgc gccccagagg atttcttcct     300

cggcgaaggg acagcgaaag atgagggtgg caggaagaga agggcgcttt ctgtctgccg     360

gggtcgcagc gcgagagggc agtgccatgt tcctctccat cctagtggcg ctgtgcctgt     420

ggctgcacct ggcgctgggc gtgcgcggcg cgccctgcga ggcggtgcgc atccctatgt     480

gccggcacat gccctggaac atcacgcgga tgcccaacca cctgcaccac agcacgcagg     540

agaacgccat cctggccatc gagcagtacg aggagctggt ggacgtgaac tgcagcgccg     600

tgctgcgctt cttcctctgt gccatgtacg cgcccatttg caccctggag ttcctgcacg     660

accctatcaa gccgtgcaag tcggtgtgcc aacgcgcgcg cgacgactgc gagcccctca     720

tgaagatgta caaccacagc tggcccgaaa gcctggcctg cgacgagctg cctgtctatg     780

accgtggcgt gtgcatctcg cctgaagcca tcgtcacgga cctcccggag gatgttaagt 840

ggatagacat cacaccagac atgatggtac aggaaaggcc tcttgatgtt gactgtaaac 900

gcctaagccc cgatcggtgc aagtgtaaaa aggtgaagcc aactttggca acgtatctca 960

gcaaaaacta cagctatgtt attcatgcca aaataaaagc tgtgcagagg agtggctgca 1020

atgaggtcac aacggtggtg gatgtaaaag agatcttcaa gtcctcatca cccatccctc 1080

gaactcaagt cccgctcatt acaaattctt cttgccagtg tccacacatc ctgccccatc 1140

aagatgttct catcatgtgt tacgagtggc gctcaaggat gatgcttctt gaaaattgct 1200

tagttgaaaa atggagagat cagcttagta aaagatccat acagtgggaa gagaggctgc 1260

aggaacagcg gagaacagtt caggacaaga agaaaacagc cgggcgcacc agtcgtagta 1320

atccccccaa accaaaggga aagcctcctg ctcccaaacc agccagtccc aagaagaaca 1380

ttaaaactag gagtgcccag aagagaacaa acccgaaaag agtgtgagct aactagtttc 1440

caaagcggag acttccgact tccttacagg atgaggctgg gcattgcctg ggacagccta 1500

tgtaaggcca tgtgcccctt gccctaacaa ctcactgcag tgctcttcat agacacatct 1560

tgcagcattt ttcttaaggc tatgcttcag tttttctttg taagccatca caagccatag 1620

tggtaggttt gccctttggt acagaaggtg agttaaagct ggtggaaaag gcttattgca 1680

ttgcattcag agtaacctgt gtgcatactc tagaagagta gggaaaataa tgcttgttac 1740

aattcgacct aatatgtgca ttgtaaaata aatgccatat ttcaaacaaa acacgtaatt 1800

tttttacagt atgttttatt accttttgat atctgttgtt gcaatgttag tgatgtttta 1860

aaatgtgatc gaaaatataa tgcttctaag aaggaacagt agtggaatga atgtctaaaa 1920

gatctttatg tgtttatggt ctgcagaagg atttttgtga tgaaagggga ttttttgaaa 1980

aatctagaga agtagcatat ggaaaactat aatgtgtctt ttttacaatg acttcagctc 2040

tgtttttagc tagaaactct aaaaacaaaa ataataataa agaaaaataa ataaaaagga 2100

gaggcagaca atgtctggat tcctgttttt tggttacctg atttcatgat catgatgctt 2160

cttgtcaaca ccctcttaag cagcaccaga aacagtgagt ttgtctgtac cattaggagt 2220

taggtactaa ttagttggct aatgctcaag tattttatac ccacaagaga ggtatgtcac 2280

tcatcttact tcccaggaca tccaccctga gaataatttg acaagcttaa aaatggcctt 2340

catgtgagtg ccaaattttg ttttcttcat ttaaatattt tctttgccta aatacatgtg 2400

agaggagtta aatataaatg tacagagagg aaagttgagg ttccacctct gaaatgagaa 2460

ttacttgaca gttgggatac tttaatcaga aaaaaagaac ttatcttgca gcattttatc 2520

aacaaatttc ataattgtgg acaattggag gcatttattt taaaaaacaa ttttattggc 2580

cttttgctaa cacagtaagc atgtattctc tataaggcat tcaataaatg cacaacgccc 2640

aaaggaaata aaatcctatc taatcctact ctccactaca cagaggtaat cactattagt 2700

attttggcat attattctcc aggtgtttct tatgcactta taaaatgatt tgaacaaata 2760

aaactaggaa cctgctatac atgtgtttca taacctgcct cctttgcttg gccctttatt 2820

gagataagtt ttcctgtcaa gaaagcagaa accatctcat ttctaacagc tgtgttatat 2880

tccatagtat gcattactca acaaactgtt gtgctattgg atacttaggt ggtttcttca 2940

ctgacaatac tgaataaaca tctcaatagt caaa 2974

<210> 22
<211> 346
<212> PRT
<213> Homo sapiens

<400> 22

<210> 23
<211> 2450
<212> DNA
<213> Homo sapiens

<220>
<221> source
<222> 1..2450
<223> /mol_type="unassigned DNA"
      /organism="Homo sapiens"

<400> 23
atatttcata cctttctaga aactgggtgt gatctcactg ttggtaaagc ccagcccttc      60

ccaacctgca agctcacctt ccaggactgg gcccagccca tgctctccat atataagctg     120

ctgccccgag cctgattcct agtcctgctt ctcttccctc tctcctccag cctctcacac     180

tctcctcagc tctctcatct cctggaacca tggccagcac atccaccacc atcaggagcc     240

acagcagcag ccgccggggt ttcagtgcca actcagccag gctccctggg gtcagccgct     300

ctggcttcag cagcgtctcc gtgtcccgct ccaggggcag tggtggcctg ggtggtgcat     360

gtggaggagc tggctttggc agccgcagtc tgtatggcct ggggggctcc aagaggatct     420

ccattggagg gggcagctgt gccatcagtg gcggctatgg cagcagagcc ggaggcagct     480

atggctttgg tggcgccggg agtggatttg gtttcggtgg tggagccggc attggctttg     540

gtctgggtgg tggagccggc cttgctggtg gctttggggg ccctggcttc cctgtgtgcc     600

cccctggagg catccaagag gtcaccgtca accagagtct cctgactccc ctcaacctgc     660

aaatcgatcc caccatccag cgggtgcggg ctgaggagcg tgaacagatc aagaccctca     720

acaacaagtt tgcctccttc atcgacaagg tgcggttcct ggagcagcag aacaaggttc     780

tggaaacaaa gtggaccctg ctgcaggagc agggcaccaa gactgtgagg cagaacctgg     840

agccgttgtt cgagcagtac atcaacaacc tcaggaggca gctggacagc attgtcgggg     900

aacggggccg cctggactca gagctcagag gcatgcagga cctggtggag gacttcaaga     960

acaaatatga ggatgaaatc aacaagcgca cagcagcaga gaatgaattt gtgactctga    1020

agaaggatgt ggatgctgcc tacatgaaca aggttgaact gcaagccaag gcagacactc    1080

tcacagacga gatcaacttc ctgagagcct tgtatgatgc agagctgtcc cagatgcaga    1140

cccacatctc agacacatct gtggtgctgt ccatggacaa caaccgcaac ctggacctgg    1200

acagcatcat cgctgaggtc aaggcccaat atgaggagat tgctcagaga agccgggctg    1260

aggctgagtc ctggtaccag accaagtacg aggagctgca ggtcacagca ggcagacatg    1320

gggacgacct gcgcaacacc aagcaggaga ttgctgagat caaccgcatg atccagaggc    1380

tgagatctga gatcgaccac gtcaagaagc agtgcgccaa cctgcaggcc gccattgctg    1440

atgctgagca gcgtggggag atggccctca aggatgccaa gaacaagctg gaagggctgg    1500

aggatgccct gcagaaggcc aagcaggacc tggcccggct gctgaaggag taccaggagc 1560

tgatgaatgt caagctggcc ctggacgtgg agatcgccac ctaccgcaag ctgctggagg 1620

gtgaggagtg caggctgaat ggcgaaggcg ttggacaagt caacatctct gtggtgcagt 1680

ccaccgtctc cagtggctat ggcggtgcca gtggtgtcgg cagtggctta ggcctgggtg 1740

gaggaagcag ctactcctat ggcagtggtc ttggcgttgg aggtggcttc agttccagca 1800

gtggcagagc cattgggggt ggcctcagct ctgttggagg cggcagttcc accatcaagt 1860

acaccaccac ctcctcctcc agcaggaaga gctataagca ctaaagtgcg tctgctagct 1920

ctcggtccca cagtcctcag gcccctctct ggctgcagag ccctctcctc aggttgcctt 1980

tcctctcctg gcctccagtc tcccctgctg tcccaggtag agctgggtat ggatgcttag 2040

tgccctcact tcttctctct ctctctatac catctgagca cccattgctc accatcagat 2100

caacctctga ttttacatca tgatgtaatc accactggag cttcactgtt actaaattat 2160

taatttcttg cctccagtgt tctatctctg aggctgagca ttataagaaa atgacctctg 2220

ctccttttca ttgcagaaaa ttgccagggg cttatttcag aacaacttcc acttactttc 2280

cactggctct caaactctct aacttataag tgttgtgaac ccccacccag gcagtatcca 2340

tgaaagcaca agtgactagt cctatgatgt acaaagcctg tatctctgtg atgatttctg 2400

tgctcttcgc tgtttgcaat tgctaaataa agcagattta taatacaata 2450

<210> 24
<211> 564
<212> PRT
<213> Homo sapiens

<400> 24

<210> 25
<211> 3685
<212> DNA
<213> Homo sapiens

<220>
<221> source
<222> 1..3685
<223> /mol_type="unassigned DNA"
      /organism="Homo sapiens"

<400> 25
agtggactca cgcaggcgca ggagactaca cttcccagga actccgggcc gcgttgttcg        60

ctggtacctc cttctgactt ccggtattgc tgcggtctgt agggccaatc gggagcctgg 120

aattgctttc ccggcgctct gattggtgca ttcgactagg ctgcctgggt tcaaaatttc 180

aacgatactg aatgagtccc gcggcgggtt ggctcgcgct tcgttgtcag atctgaggcg 240

aggctaggtg agccgtggga agaaaagagg gagcagctag ggcgcgggtc tccctcctcc 300

cggagtttgg aacggctgaa gttcaccttc cagcccctag cgccgttcgc gccgctaggc 360

ctggcttctg aggcggttgc ggtgctcggt cgccgcctag gcggggcagg gtgcgagcag 420

gggcttcggg ccacgcttct cttggcgaca ggattttgct gtgaagtccg tccgggaaac 480

ggaggaaaaa aagagttgcg ggaggctgtc ggctaataac ggttcttgat acatatttgc 540

cagacttcaa gatttcagaa aaggggtgaa agagaagatt gcaactttga gtcagacctg 600

taggcctgat agactgatta aaccacagaa ggtgacctgc tgagaaaagt ggtacaaata 660

ctgggaaaaa cctgctcttc tgcgttaagt gggagacaat gtcacaagtt aaaagctctt 720

attcctatga tgccccctcg gatttcatca atttttcatc cttggatgat gaaggagata 780

ctcaaaacat agattcatgg tttgaggaga aggccaattt ggagaataag ttactgggga 840

agaatggaac tggagggctt tttcagggca aaactccttt gagaaaggct aatcttcagc 900

aagctattgt cacacctttg aaaccagttg acaacactta ctacaaagag gcagaaaaag 960

aaaatcttgt ggaacaatcc attccgtcaa atgcttgttc ttccctggaa gttgaggcag 1020

ccatatcaag aaaaactcca gcccagcctc agagaagatc tcttaggctt tctgctcaga 1080

aggatttgga acagaaagaa aagcatcatg taaaaatgaa agccaagaga tgtgccactc 1140

ctgtaatcat cgatgaaatt ctaccctcta agaaaatgaa agtttctaac aacaaaaaga 1200

agccagagga agaaggcagt gctcatcaag atactgctga aaagaatgca tcttccccag 1260

agaaagccaa gggtagacat actgtgcctt gtatgccacc tgcaaagcag aagtttctaa 1320

aaagtactga ggagcaagag ctggagaaga gtatgaaaat gcagcaagag gtggtggaga 1380

tgcggaaaaa gaatgaagaa ttcaagaaac ttgctctggc tggaataggg caacctgtga 1440

agaaatcagt gagccaggtc accaaatcag ttgacttcca cttccgcaca gatgagcgaa 1500

tcaaacaaca tcctaagaac caggaggaat ataaggaagt gaactttaca tctgaactac 1560

gaaagcatcc ttcatctcct gcccgagtga ctaagggatg taccattgtt aagcctttca 1620

acctgtccca aggaaagaaa agaacatttg atgaaacagt ttctacatat gtgccccttg 1680

cacagcaagt tgaagacttc cataaacgaa cccctaacag atatcatttg aggagcaaga 1740

aggatgatat taacctgtta ccctccaaat cttctgtgac caagatttgc agagacccac 1800

agactcctgt actgcaaacc aaacaccgtg cacgggctgt gacctgcaaa agtacagcag 1860

agctggaggc tgaggagctc gagaaattgc aacaatacaa attcaaagca cgtgaacttg 1920

atcccagaat acttgaaggt gggcccatct tgcccaagaa accacctgtg aaaccaccca 1980

ccgagcctat tggctttgat ttggaaattg agaaaagaat ccaggagcga gaatcaaaga 2040

agaaaacaga ggatgaacac tttgaatttc attccagacc ttgccctact aagattttgg 2100

aagatgttgt gggtgttcct gaaaagaagg tacttccaat caccgtcccc aagtcaccag 2160

cctttgcatt gaagaacaga attcgaatgc ccaccaaaga agatgaggaa gaggacgaac 2220

cggtagtgat aaaagctcaa cctgtgccac attatggggt gccttttaag ccccaaatcc 2280

cagaggcaag aactgtggaa atatgccctt tctcgtttga ttctcgagac aaagaacgtc 2340

agttacagaa ggagaagaaa ataaaagaac tgcagaaagg ggaggtgccc aagttcaagg 2400

cacttccctt gcctcatttt gacaccatta acctgccaga gaagaaggta aagaatgtga 2460

cccagattga acctttctgc ttggagactg acagaagagg tgctctgaag gcacagactt 2520

ggaagcacca gctggaagaa gaactgagac agcagaaaga agcagcttgt ttcaaggctc 2580

gtccaaacac cgtcatctct caggagccct ttgttcccaa gaaagagaag aaatcagttg 2640

ctgagggcct ttctggttct ctagttcagg aaccttttca gctggctact gagaagagag 2700

ccaaagagcg gcaggagctg gagaagagaa tggctgaggt agaagcccag aaagcccagc 2760

agttggagga ggccagacta caggaggaag agcagaaaaa agaggagctg gccaggctac 2820

ggagagaact ggtgcataag gcaaatccaa tacgcaagta ccagggtctg gagataaagt 2880

caagtgacca gcctctgact gtgcctgtat ctcccaaatt ctccactcga ttccactgct 2940

aaactcagct gtgagctgcg gataccgccc ggcaatggga cctgctctta acctcaaacc 3000

taggaccgtc ttgctttgtc attgggcatg gagagaaccc atttctccag acttttacct 3060

acccgtgcct gagaaagcat acttgacaac tgtggactcc agttttgttg agaattgttt 3120

tcttacatta ctaaggctaa taatgagatg taactcatga atgtctcgat tagactccat 3180

gtagttactt cctttaaacc atcagccggc cttttatatg ggtcttcact ctgactagaa 3240

tttagtctct gtgtcagcac agtgtaatct ctattgctat tgccccttac gactctcacc 3300

ctctccccac tttttttaaa aattttaacc agaaaataaa gatagttaaa tcctaagata 3360

gagattaagt catggtttaa atgaggaaca atcagtaaat cagattctgt cctcttctct 3420

gcataccgtg aatttatagt taaggatccc tttgctgtga gggtagaaaa cctcaccaac 3480

tgcaccagtg aggaagaaga ctgcgtggat tcatggggag cctcacagca gccacgcagc 3540

aggctctggg tggggctgcc gttaaggcac gttctttcct tactggtgct gataacaaca 3600

gggaaccgtg cagtgtgcat tttaagacct ggcctggaat aaatacgttt tgtctttccc 3660

tcaaaaaaaa aaaaaaaaaa aaaaa 3685

<210> 26
<211> 747

<212> PRT
<213> Homo sapiens

<400> 26
Met Ser Gln Val Lys Ser Ser Tyr Ser Tyr Asp Ala Pro Ser Asp Phe
1               5                   10                  15
Ile Asn Phe Ser Ser Leu Asp Asp Glu Gly Asp Thr Gln Asn Ile Asp
            20                  25                  30
Ser Trp Phe Glu Glu Lys Ala Asn Leu Glu Asn Lys Leu Leu Gly Lys
        35                  40                  45
Asn Gly Thr Gly Gly Leu Phe Gln Gly Lys Thr Pro Leu Arg Lys Ala
    50                  55                  60
Asn Leu Gln Gln Ala Ile Val Thr Pro Leu Lys Pro Val Asp Asn Thr
65                  70                  75                  80
Tyr Tyr Lys Glu Ala Glu Lys Glu Asn Leu Val Glu Gln Ser Ile Pro
                85                  90                  95
Ser Asn Ala Cys Ser Ser Leu Glu Val Glu Ala Ala Ile Ser Arg Lys
            100                 105                 110
Thr Pro Ala Gln Pro Gln Arg Arg Ser Leu Arg Leu Ser Ala Gln Lys
        115                 120                 125
Asp Leu Glu Gln Lys Glu Lys His His Val Lys Met Lys Ala Lys Arg
    130                 135                 140
Cys Ala Thr Pro Val Ile Ile Asp Glu Ile Leu Pro Ser Lys Lys Met
145                 150                 155                 160
Lys Val Ser Asn Asn Lys Lys Lys Pro Glu Glu Glu Gly Ser Ala His
                165                 170                 175
Gln Asp Thr Ala Glu Lys Asn Ala Ser Ser Pro Glu Lys Ala Lys Gly
            180                 185                 190
Arg His Thr Val Pro Cys Met Pro Pro Ala Lys Gln Lys Phe Leu Lys
        195                 200                 205
Ser Thr Glu Glu Gln Glu Leu Glu Lys Ser Met Lys Met Gln Gln Glu
    210                 215                 220
Val Val Glu Met Arg Lys Lys Asn Glu Glu Phe Lys Lys Leu Ala Leu
225                 230                 235                 240
Ala Gly Ile Gly Gln Pro Val Lys Lys Ser Val Ser Gln Val Thr Lys
                245                 250                 255
Ser Val Asp Phe His Phe Arg Thr Asp Glu Arg Ile Lys Gln His Pro
            260                 265                 270
Lys Asn Gln Glu Glu Tyr Lys Glu Val Asn Phe Thr Ser Glu Leu Arg
        275                 280                 285
Lys His Pro Ser Ser Pro Ala Arg Val Thr Lys Gly Cys Thr Ile Val
    290                 295                 300
Lys Pro Phe Asn Leu Ser Gln Gly Lys Lys Arg Thr Phe Asp Glu Thr
305                 310                 315                 320
Val Ser Thr Tyr Val Pro Leu Ala Gln Gln Val Glu Asp Phe His Lys
                325                 330                 335
Arg Thr Pro Asn Arg Tyr His Leu Arg Ser Lys Lys Asp Asp Ile Asn
            340                 345                 350
Leu Leu Pro Ser Lys Ser Ser Val Thr Lys Ile Cys Arg Asp Pro Gln
        355                 360                 365
Thr Pro Val Leu Gln Thr Lys His Arg Ala Arg Ala Val Thr Cys Lys
    370                 375                 380
Ser Thr Ala Glu Leu Glu Ala Glu Glu Leu Glu Lys Leu Gln Gln Tyr
385                 390                 395                 400
Lys Phe Lys Ala Arg Glu Leu Asp Pro Arg Ile Leu Glu Gly Gly Pro
                405                 410                 415
Ile Leu Pro Lys Lys Pro Pro Val Lys Pro Pro Thr Glu Pro Ile Gly
            420                 425                 430
Phe Asp Leu Glu Ile Glu Lys Arg Ile Gln Glu Arg Glu Ser Lys Lys
        435                 440                 445
Lys Thr Glu Asp Glu His Phe Glu Phe His Ser Arg Pro Cys Pro Thr
    450                 455                 460

Lys Ile Leu Glu Asp Val Val Gly Val Pro Glu Lys Lys Val Leu Pro
465                 470                 475                 480
Ile Thr Val Pro Lys Ser Pro Ala Phe Ala Leu Lys Asn Arg Ile Arg
                485                 490                 495
Met Pro Thr Lys Glu Asp Glu Glu Glu Asp Glu Pro Val Val Ile Lys
            500                 505                 510
Ala Gln Pro Val Pro His Tyr Gly Val Pro Phe Lys Pro Gln Ile Pro
        515                 520                 525
Glu Ala Arg Thr Val Glu Ile Cys Pro Phe Ser Phe Asp Ser Arg Asp
    530                 535                 540
Lys Glu Arg Gln Leu Gln Lys Glu Lys Lys Ile Lys Glu Leu Gln Lys
545                 550                 555                 560
Gly Glu Val Pro Lys Phe Lys Ala Leu Pro Leu Pro His Phe Asp Thr
                565                 570                 575
Ile Asn Leu Pro Glu Lys Lys Val Lys Asn Val Thr Gln Ile Glu Pro
            580                 585                 590
Phe Cys Leu Glu Thr Asp Arg Arg Gly Ala Leu Lys Ala Gln Thr Trp
        595                 600                 605
Lys His Gln Leu Glu Glu Glu Leu Arg Gln Gln Lys Glu Ala Ala Cys
    610                 615                 620
Phe Lys Ala Arg Pro Asn Thr Val Ile Ser Gln Glu Pro Phe Val Pro
625                 630                 635                 640
Lys Lys Glu Lys Lys Ser Val Ala Glu Gly Leu Ser Gly Ser Leu Val
                645                 650                 655
Gln Glu Pro Phe Gln Leu Ala Thr Glu Lys Arg Ala Lys Glu Arg Gln
            660                 665                 670
Glu Leu Glu Lys Arg Met Ala Glu Val Glu Ala Gln Lys Ala Gln Gln
        675                 680                 685
Leu Glu Glu Ala Arg Leu Gln Glu Glu Glu Gln Lys Lys Glu Glu Leu
    690                 695                 700
Ala Arg Leu Arg Arg Glu Leu Val His Lys Ala Asn Pro Ile Arg Lys
705                 710                 715                 720
Tyr Gln Gly Leu Glu Ile Lys Ser Ser Asp Gln Pro Leu Thr Val Pro
                725                 730                 735
Val Ser Pro Lys Phe Ser Thr Arg Phe His Cys
            740                 745

<210> 27
<211> 1566
<212> DNA
<213> Homo sapiens

<220>
<221> source
<222> 1..1566
<223> /mol_type="unassigned DNA"
      /organism="Homo sapiens"

<400> 27
gggccgccca atggggcgca agcgacgcgg tatttgaatc ctggaacaag gctacagcgt      60

cgaagatccc cagcgctgcg ggctcggaga gcagtcctaa cggcgcctcg tacgctagtg     120

tcctcccttt tcagtccgcg tccctccctg ggccgggctg gcactcttgc cttccccgtc     180

cctcatggcg ctgctccgac gcccgacggt gtccagtgat ttggagaata ttgacacagg     240

agttaattct aaagttaaga gtcatgtgac tattaggcga actgttttag aagaaattgg     300

aaatagagtt acaaccagag cagcacaagt agctaagaaa gctcagaaca ccaaagttcc     360

agttcaaccc accaaaacaa caaatgtcaa caaacaactg aaacctactg cttctgtcaa     420

accagtacag atggaaaagt tggctccaaa gggtccttct cccacacctg aggatgtctc 480

catgaaggaa gagaatctct gccaagcttt ttctgatgcc ttgctctgca aaatcgagga 540

cattgataac gaagattggg agaaccctca gctctgcagt gactacgtta aggatatcta 600

tcagtatctc aggcagctgg aggttttgca gtccataaac ccacatttct tagatggaag 660

agatataaat ggacgcatgc gtgccatcct agtggattgg ctggtacaag tccactccaa 720

gtttaggctt ctgcaggaga ctctgtacat gtgcgttggc attatggatc gatttttaca 780

ggttcagcca gtttcccgga agaagcttca attagttggg attactgctc tgctcttggc 840

ttccaagtat gaggagatgt tttctccaaa tattgaagac tttgtttaca tcacagacaa 900

tgcttatacc agttcccaaa tccgagaaat ggaaactcta attttgaaag aattgaaatt 960

tgagttgggt cgacccttgc cactacactt cttaaggcga gcatcaaaag ccggggaggt 1020

tgatgttgaa cagcacactt tagccaagta tttgatggag ctgactctca tcgactatga 1080

tatggtgcat tatcatcctt ctaaggtagc agcagctgct tcctgcttgt ctcagaaggt 1140

tctaggacaa ggaaaatgga acttaaagca gcagtattac acaggataca cagagaatga 1200

agtattggaa gtcatgcagc acatggccaa gaatgtggtg aaagtaaatg aaaacttaac 1260

taaattcatc gccatcaaga ataagtatgc aagcagcaaa ctcctgaaga tcagcatgat 1320

ccctcagctg aactcaaaag ccgtcaaaga ccttgcctcc ccactgatag gaaggtccta 1380

ggctgccgtg gcccctgggg atgtgtgctt cattgtgccc tttttcttat tggtttagaa 1440

ctcttgattt tgtacatagt cctctggtct atctcatgaa acctcttctc agaccagttt 1500

tctaaacata tattgaggaa aaataaagcg attggttttt cttaaggtaa aaaaaaaaaa 1560

aaaaaa 1566

<210> 28
<211> 398
<212> PRT
<213> Homo sapiens

<400> 28
Met Ala Leu Leu Arg Arg Pro Thr Val Ser Ser Asp Leu Glu Asn Ile
1               5                   10                  15
Asp Thr Gly Val Asn Ser Lys Val Lys Ser His Val Thr Ile Arg Arg
            20                  25                  30
Thr Val Leu Glu Glu Ile Gly Asn Arg Val Thr Thr Arg Ala Ala Gln
        35                  40                  45
Val Ala Lys Lys Ala Gln Asn Thr Lys Val Pro Val Gln Pro Thr Lys
    50                  55                  60
Thr Thr Asn Val Asn Lys Gln Leu Lys Pro Thr Ala Ser Val Lys Pro
65                  70                  75                  80
Val Gln Met Glu Lys Leu Ala Pro Lys Gly Pro Ser Pro Thr Pro Glu
                85                  90                  95
Asp Val Ser Met Lys Glu Glu Asn Leu Cys Gln Ala Phe Ser Asp Ala
            100                 105                 110
Leu Leu Cys Lys Ile Glu Asp Ile Asp Asn Glu Asp Trp Glu Asn Pro

115                 120                 125
Gln Leu Cys Ser Asp Tyr Val Lys Asp Ile Tyr Gln Tyr Leu Arg Gln
    130                 135                 140
Leu Glu Val Leu Gln Ser Ile Asn Pro His Phe Leu Asp Gly Arg Asp
145                 150                 155                 160
Ile Asn Gly Arg Met Arg Ala Ile Leu Val Asp Trp Leu Val Gln Val
                165                 170                 175
His Ser Lys Phe Arg Leu Leu Gln Glu Thr Leu Tyr Met Cys Val Gly
            180                 185                 190
Ile Met Asp Arg Phe Leu Gln Val Gln Pro Val Ser Arg Lys Lys Leu
        195                 200                 205
Gln Leu Val Gly Ile Thr Ala Leu Leu Leu Ala Ser Lys Tyr Glu Glu
    210                 215                 220
Met Phe Ser Pro Asn Ile Glu Asp Phe Val Tyr Ile Thr Asp Asn Ala
225                 230                 235                 240
Tyr Thr Ser Ser Gln Ile Arg Glu Met Glu Thr Leu Ile Leu Lys Glu
                245                 250                 255
Leu Lys Phe Glu Leu Gly Arg Pro Leu Pro Leu His Phe Leu Arg Arg
            260                 265                 270
Ala Ser Lys Ala Gly Glu Val Asp Val Glu Gln His Thr Leu Ala Lys
        275                 280                 285
Tyr Leu Met Glu Leu Thr Leu Ile Asp Tyr Asp Met Val His Tyr His
    290                 295                 300
Pro Ser Lys Val Ala Ala Ala Ala Ser Cys Leu Ser Gln Lys Val Leu
305                 310                 315                 320
Gly Gln Gly Lys Trp Asn Leu Lys Gln Gln Tyr Tyr Thr Gly Tyr Thr
                325                 330                 335
Glu Asn Glu Val Leu Glu Val Met Gln His Met Ala Lys Asn Val Val
            340                 345                 350
Lys Val Asn Glu Asn Leu Thr Lys Phe Ile Ala Ile Lys Asn Lys Tyr
        355                 360                 365
Ala Ser Ser Lys Leu Leu Lys Ile Ser Met Ile Pro Gln Leu Asn Ser
    370                 375                 380
Lys Ala Val Lys Asp Leu Ala Ser Pro Leu Ile Gly Arg Ser
385                 390                 395

<210> 29
<211> 4786
<212> DNA
<213> Homo sapiens

<220>
<221> source
<222> 1..4786
<223> /mol_type="unassigned DNA"
      /organism="Homo sapiens"

<400> 29
ctcggcgctg aaattcaaat ttgaacggct gcagaggccg agtccgtcac tggaagccga      60

gaggagagga cagctggttg tgggagagtt cccccgcctc agactcctgg ttttttccag     120

gagacacact gagctgagac tcacttttct cttcctgaat ttgaaccacc gtttccatcg     180

tctcgtagtc cgacgcctgg ggcgatggat ccgtttacgg agaaactgct ggagcgaacc     240

cgtgccaggc gagagaatct tcagagaaaa atggctgaga ggcccacagc agctccaagg     300

tctatgactc atgctaagcg agctagacag ccactttcag aagcaagtaa ccagcagccc     360

ctctctggtg gtgaagagaa atcttgtaca aaaccatcgc catcaaaaaa acgctgttct     420

gacaacactg aagtagaagt ttctaacttg gaaaataaac aaccagttga gtcgacatct     480

gcaaaatctt gttctccaag tcctgtgtct cctcaggtgc agccacaagc agcagatacc 540

atcagtgatt ctgttgctgt cccggcatca ctgctgggca tgaggagagg gctgaactca 600

agattggaag caactgcagc ctcctcagtt aaaacacgta tgcaaaaact tgcagagcaa 660

cggcgccgtt gggataatga tgatatgaca gatgacattc ctgaaagctc actcttctca 720

ccaatgccat cagaggaaaa ggctgcttcc cctcccagac ctctgctttc aaatgcctcg 780

gcaactccag ttggcagaag gggccgtctg gccaatcttg ctgcaactat ttgctcctgg 840

gaagatgatg taaatcactc atttgcaaaa caaaacagtg tacaagaaca gcctggtacc 900

gcttgtttat ccaaattttc ctctgcaagt ggagcatctg ctaggatcaa tagcagcagt 960

gttaagcagg aagctacatt ctgttcccaa agggatggcg atgcctcttt gaataaagcc 1020

ctatcctcaa gtgctgatga tgcgtctttg gttaatgcct caatttccag ctctgtgaaa 1080

gctacttctc cagtgaaatc tactacatct atcactgatg ctaaaagttg tgagggacaa 1140

aatcctgagc tacttccaaa aactcctatt agtcctctga aaacgggggt atcgaaacca 1200

attgtgaagt caactttatc ccagacagtt ccatccaagg gagaattaag tagagaaatt 1260

tgtctgcaat ctcaatctaa agacaaatct acgacaccag gaggaacagg aattaagcct 1320

ttcctggaac gctttggaga gcgttgtcaa gaacatagca aagaaagtcc agctcgtagc 1380

acaccccaca gaacccccat tattactcca aatacaaagg ccatccaaga aagattattc 1440

aagcaagaca catcttcatc tactacccat ttagcacaac agctcaagca ggaacgtcaa 1500

aaagaactag catgtcttcg tggccgattt gacaagggca atatatggag tgcagaaaaa 1560

ggcggaaact caaaaagcaa acaactagaa accaaacagg aaactcactg tcagagcact 1620

cccctcaaaa aacaccaagg tgtttcaaaa actcagtcac ttccagtaac agaaaaggtg 1680

accgaaaacc agataccagc caaaaattct agtacagaac ctaaaggttt cactgaatgc 1740

gaaatgacga aatctagccc tttgaaaata acattgtttt tagaagagga caaatcctta 1800

aaagtaacat cagacccaaa ggttgagcag aaaattgaag tgatacgtga aattgagatg 1860

agtgtggatg atgatgatat caatagttcg aaagtaatta atgacctctt cagtgatgtc 1920

ctagaggaag gtgaactaga tatggagaag agccaagagg agatggatca agcattagca 1980

gaaagcagcg aagaacagga agatgcactg aatatctcct caatgtcttt acttgcacca 2040

ttggcacaaa cagttggtgt ggtaagtcca gagagtttag tgtccacacc tagactggaa 2100

ttgaaagaca ccagcagaag tgatgaaagt ccaaaaccag gaaaattcca aagaactcgt 2160

gtccctcgag ctgaatctgg tgatagcctt ggttctgaag atcgtgatct tctttacagc 2220

attgatgcat atagatctca aagattcaaa gaaacagaac gtccatcaat aaagcaggtg 2280

attgttcgga aggaagatgt tacttcaaaa ctggatgaaa aaaataatgc ctttccttgt 2340

caagttaata tcaaacagaa aatgcaggaa ctcaataacg aaataaatat gcaacagaca 2400

gtgatctatc aagctagcca ggctcttaac tgctgtgttg atgaagaaca tggaaaaggg 2460

tccctagaag aagctgaagc agaaagactt cttctaattg caactgggaa gagaacactt 2520

ttgattgatg aattgaataa attgaagaac gaaggacctc agaggaagaa taaggctagt 2580

ccccaaagtg aatttatgcc atccaaagga tcagttactt tgtcagaaat ccgcttgcct 2640

ctaaaagcag attttgtctg cagtacggtt cagaaaccag atgcagcaaa ttactattac 2700

ttaattatac taaaagcagg agctgaaaat atggtagcca caccattagc aagtacttca 2760

aactctctta acggtgatgc tctgacattc actactacat ttactctgca agatgtatcc 2820

aatgactttg aaataaatat tgaagtttac agcttggtgc aaaagaaaga tccctcaggc 2880

cttgataaga agaaaaaaac atccaagtcc aaggctatta ctccaaagcg actcctcaca 2940

tctataacca caaaaagcaa cattcattct tcagtcatgg ccagtccagg aggtcttagt 3000

gctgtgcgaa ccagcaactt cgcccttgtt ggatcttaca cattatcatt gtcttcagta 3060

ggaaatacta agtttgttct ggacaaggtc ccctttttat cttctttgga aggtcatatt 3120

tatttaaaaa taaaatgtca agtgaattcc agtgttgaag aaagaggttt tctaaccata 3180

tttgaagatg ttagtggttt tggtgcctgg catcgaagat ggtgtgttct ttctggaaac 3240

tgtatatctt attggactta tccagatgat gagaaacgca agaatcccat aggaaggata 3300

aatctggcta attgtaccag tcgtcagata gaaccagcca acagagaatt ttgtgcaaga 3360

cgcaacactt ttgaattaat tactgtccga ccacaaagag aagatgaccg agagactctt 3420

gtcagccaat gcagggacac actctgtgtt accaagaact ggctgtctgc agatactaaa 3480

gaagagcggg atctctggat gcaaaaactc aatcaagttc ttgttgatat tcgcctctgg 3540

caacctgatg cttgctacaa acctattgga aagccttaaa ccgggaaatt tccatgctat 3600

ctagaggttt ttgatgtcat cttaagaaac acacttaaga gcatcagatt tactgattgc 3660

attttatgct ttaagtacga aagggtttgt gccaatattc actacgtatt atgcagtatt 3720

tatatctttt gtatgtaaaa ctttaactga tttctgtcat tcatcaatga gtagaagtaa 3780

atacattata gttgattttg ctaaatctta atttaaaagc ctcattttcc tagaaatcta 3840

attattcagt tattcatgac aatatttttt taaaagtaag aaattctgag ttgtcttctt 3900

ggagctgtag gtcttgaagc agcaacgtct ttcaggggtt ggagacagaa acccattctc 3960

caatctcagt agttttttcg aaaggctgtg atcatttatt gatcgtgata tgacttgtta 4020

ctagggtact gaaaaaaatg tctaaggcct ttacagaaac atttttagta atgaggatga 4080

gaactttttc aaatagcaaa tatatattgg cttaaagcat gaggctgtct tcagaaaagt 4140

gatgtggaca taggaggcaa tgtgtgagac ttgggggttc aatattttat atagaagagt 4200

taataagcac atggtttaca tttactcagc tactatatat gcagtgtggt gcacattttc 4260

acagaattct ggcttcatta agatcattat ttttgctgcg tagcttacag acttagcata 4320

ttagtttttt ctactcctac aagtgtaaat tgaaaaatct ttatattaaa aaagtaaact 4380

gttatgaagc tgctatgtac taataatact ttgcttgcca aagtgtttgg gttttgttgt 4440

tgtttgtttg tttgtttgtt tttggttcat gaacaacagt gtctagaaac ccattttgaa 4500

agtggaaaat tattaagtca cctatcacct ttaaacgcct ttttttaaaa ttataaaata 4560

ttgtaaagca gggtctcaac ttttaaatac actttgaact tcttctctga attattaaag 4620

ttctttatga cctcatttat aaacactaaa ttctgtcacc tcctgtcatt ttatttttta 4680

ttcattcaaa tgtatttttt cttgtgcata ttataaaaat atattttatg agctcttact 4740

caaataaata cctgtaaatg tctaaaggaa aaaaaaaaaa aaaaaa 4786

<210> 30
<211> 1124
<212> PRT
<213> Homo sapiens

<400> 30

<210> 31
<211> 3665
<212> DNA
<213> Homo sapiens

<220>
<221> source
<222> 1..3665
<223> /mol_type="unassigned DNA"
      /organism="Homo sapiens"

<400> 31
tttcaaacag cggaacaaac tgaaagctcc ggtgccagac cccacccccg gccccggccc      60

gggaccccct cccctcccgg gatcccccgg ggttcccacc ccgcccgcac cgccggggac      120

ccggccggtc cggcgcgagc ccccgtccgg ggccctggct cggcccccag gttggaggag      180

cccggagccc gccttcggag ctacggccta acggcggcgg cgactgcagt ctggagggtc      240

cacacttgtg attctcaatg gagagtgaaa acgcagattc ataatgaaaa ctagcccccg 300

tcggccactg attctcaaaa gacggaggct gccccttcct gttcaaaatg ccccaagtga 360

aacatcagag gaggaaccta agagatcccc tgcccaacag gagtctaatc aagcagaggc 420

ctccaaggaa gtggcagagt ccaactcttg caagtttcca gctgggatca agattattaa 480

ccaccccacc atgcccaaca cgcaagtagt ggccatcccc aacaatgcta atattcacag 540

catcatcaca gcactgactg ccaagggaaa agagagtggc agtagtgggc ccaacaaatt 600

catcctcatc agctgtgggg gagccccaac tcagcctcca ggactccggc ctcaaaccca 660

aaccagctat gatgccaaaa ggacagaagt gaccctggag accttgggac caaaacctgc 720

agctagggat gtgaatcttc ctagaccacc tggagccctt tgcgagcaga aacgggagac 780

ctgtgcagat ggtgaggcag caggctgcac tatcaacaat agcctatcca acatccagtg 840

gcttcgaaag atgagttctg atggactggg ctcccgcagc atcaagcaag agatggagga 900

aaaggagaat tgtcacctgg agcagcgaca ggttaaggtt gaggagcctt cgagaccatc 960

agcgtcctgg cagaactctg tgtctgagcg gccaccctac tcttacatgg ccatgataca 1020

attcgccatc aacagcactg agaggaagcg catgactttg aaagacatct atacgtggat 1080

tgaggaccac tttccctact ttaagcacat tgccaagcca ggctggaaga actccatccg 1140

ccacaacctt tccctgcacg acatgtttgt ccgggagacg tctgccaatg gcaaggtctc 1200

cttctggacc attcacccca gtgccaaccg ctacttgaca ttggaccagg tgtttaagcc 1260

actggaccca gggtctccac aattgcccga gcacttggaa tcacagcaga aacgaccgaa 1320

tccagagctc cgccggaaca tgaccatcaa aaccgaactc cccctgggcg cacggcggaa 1380

gatgaagcca ctgctaccac gggtcagctc atacctggta cctatccagt tcccggtgaa 1440

ccagtcactg gtgttgcagc cctcggtgaa ggtgccattg cccctggcgg cttccctcat 1500

gagctcagag cttgcccgcc atagcaagcg agtccgcatt gcccccaagg tttttgggga 1560

acaggtggtg tttggttaca tgagtaagtt ctttagtggc gatctgcgag attttggtac 1620

acccatcacc agcttgttta attttatctt tctttgttta tcagtgctgc tagctgagga 1680

ggggatagct cctctttctt ctgcaggacc agggaaagag gagaaactcc tgtttggaga 1740

agggttttct cctttgcttc cagttcagac tatcaaggag gaagaaatcc agcctgggga 1800

ggaaatgcca cacttagcga gacccatcaa agtggagagc cctcccttgg aagagtggcc 1860

ctccccggcc ccatctttca aagaggaatc atctcactcc tgggaggatt cgtcccaatc 1920

tcccacccca agacccaaga agtcctacag tgggcttagg tccccaaccc ggtgtgtctc 1980

ggaaatgctt gtgattcaac acagggagag gagggagagg agccggtctc ggaggaaaca 2040

gcatctactg cctccctgtg tggatgagcc ggagctgctc ttctcagagg ggcccagtac 2100

ttcccgctgg gccgcagagc tcccgttccc agcagactcc tctgaccctg cctcccagct 2160

cagctactcc caggaagtgg gaggaccttt taagacaccc attaaggaaa cgctgcccat 2220

ctcctccacc ccgagcaaat ctgtcctccc cagaacccct gaatcctgga ggctcacgcc 2280

cccagccaaa gtagggggac tggatttcag cccagtacaa acctcccagg gtgcctctga 2340

ccccttgcct gaccccctgg ggctgatgga tctcagcacc actcccttgc aaagtgctcc 2400

cccccttgaa tcaccgcaaa ggctcctcag ttcagaaccc ttagacctca tctccgtccc 2460

ctttggcaac tcttctccct cagatataga cgtccccaag ccaggctccc cggagccaca 2520

ggtttctggc cttgcagcca atcgttctct gacagaaggc ctggtcctgg acacaatgaa 2580

tgacagcctc agcaagatcc tgctggacat cagctttcct ggcctggacg aggacccact 2640

gggccctgac aacatcaact ggtcccagtt tattcctgag ctacagtaga gccctgccct 2700

tgcccctgtg ctcaagctgt ccaccatccc gggcactcca aggctcagtg caccccaagc 2760

ctctgagtga ggacagcagg cagggactgt tctgctcctc atagctccct gctgcctgat 2820

tatgcaaaag tagcagtcac accctagcca ctgctgggac cttgtgttcc ccaagagtat 2880

ctgattcctc tgctgtccct gccaggagct gaagggtggg aacaacaaag gcaatggtga 2940

aaagagatta ggaacccccc agcctgtttc cattctctgc ccagcagtct cttaccttcc 3000

ctgatctttg cagggtggtc cgtgtaaata gtataaattc tccaaattat cctctaatta 3060

taaatgtaag cttatttcct tagatcatta tccagagact gccagaaggt gggtaggatg 3120

acctggggtt tcaattgact tctgttcctt gcttttagtt ttgatagaag ggaagacctg 3180

cagtgcacgg tttcttccag gctgaggtac ctggatcttg ggttcttcac tgcagggacc 3240

cagacaagtg gatctgcttg ccagagtcct ttttgcccct ccctgccacc tccccgtgtt 3300

tccaagtcag ctttcctgca agaagaaatc ctggttaaaa aagtcttttg tattgggtca 3360

ggagttgaat ttggggtggg aggatggatg caactgaagc agagtgtggg tgcccagatg 3420

tgcgctatta gatgtttctc tgataatgtc cccaatcata ccagggagac tggcattgac 3480

gagaactcag gtggaggctt gagaaggccg aaagggcccc tgacctgcct ggcttcctta 3540

gcttgcccct cagctttgca aagagccacc ctaggcccca gctgaccgca tgggtgtgag 3600

ccagcttgag aacactaact actcaataaa agcgaaggtg gacatgaaaa aaaaaaaaaa 3660

aaaaa 3665

<210> 32
<211> 801
<212> PRT
<213> Homo sapiens

<400> 32
Met Lys Thr Ser Pro Arg Arg Pro Leu Ile Leu Lys Arg Arg Arg Leu

1               5                   10                  15
Pro Leu Pro Val Gln Asn Ala Pro Ser Glu Thr Ser Glu Glu Glu Pro
            20                  25                  30
Lys Arg Ser Pro Ala Gln Gln Glu Ser Asn Gln Ala Glu Ala Ser Lys
        35                  40                  45
Glu Val Ala Glu Ser Asn Ser Cys Lys Phe Pro Ala Gly Ile Lys Ile
    50                  55                  60
Ile Asn His Pro Thr Met Pro Asn Thr Gln Val Val Ala Ile Pro Asn
65                  70                  75                  80
Asn Ala Asn Ile His Ser Ile Ile Thr Ala Leu Thr Ala Lys Gly Lys
                85                  90                  95
Glu Ser Gly Ser Ser Gly Pro Asn Lys Phe Ile Leu Ile Ser Cys Gly
            100                 105                 110
Gly Ala Pro Thr Gln Pro Pro Gly Leu Arg Pro Gln Thr Gln Thr Ser
        115                 120                 125
Tyr Asp Ala Lys Arg Thr Glu Val Thr Leu Glu Thr Leu Gly Pro Lys
    130                 135                 140
Pro Ala Ala Arg Asp Val Asn Leu Pro Arg Pro Pro Gly Ala Leu Cys
145                 150                 155                 160
Glu Gln Lys Arg Glu Thr Cys Ala Asp Gly Glu Ala Ala Gly Cys Thr
                165                 170                 175
Ile Asn Asn Ser Leu Ser Asn Ile Gln Trp Leu Arg Lys Met Ser Ser
            180                 185                 190
Asp Gly Leu Gly Ser Arg Ser Ile Lys Gln Glu Met Glu Glu Lys Glu
        195                 200                 205
Asn Cys His Leu Glu Gln Arg Gln Val Lys Val Glu Glu Pro Ser Arg
    210                 215                 220
Pro Ser Ala Ser Trp Gln Asn Ser Val Ser Glu Arg Pro Pro Tyr Ser
225                 230                 235                 240
Tyr Met Ala Met Ile Gln Phe Ala Ile Asn Ser Thr Glu Arg Lys Arg
                245                 250                 255
Met Thr Leu Lys Asp Ile Tyr Thr Trp Ile Glu Asp His Phe Pro Tyr
            260                 265                 270
Phe Lys His Ile Ala Lys Pro Gly Trp Lys Asn Ser Ile Arg His Asn
        275                 280                 285
Leu Ser Leu His Asp Met Phe Val Arg Glu Thr Ser Ala Asn Gly Lys
    290                 295                 300
Val Ser Phe Trp Thr Ile His Pro Ser Ala Asn Arg Tyr Leu Thr Leu
305                 310                 315                 320
Asp Gln Val Phe Lys Pro Leu Asp Pro Gly Ser Pro Gln Leu Pro Glu
                325                 330                 335
His Leu Glu Ser Gln Gln Lys Arg Pro Asn Pro Glu Leu Arg Arg Asn
            340                 345                 350
Met Thr Ile Lys Thr Glu Leu Pro Leu Gly Ala Arg Arg Lys Met Lys
        355                 360                 365
Pro Leu Leu Pro Arg Val Ser Ser Tyr Leu Val Pro Ile Gln Phe Pro
    370                 375                 380
Val Asn Gln Ser Leu Val Leu Gln Pro Ser Val Lys Val Pro Leu Pro
385                 390                 395                 400
Leu Ala Ala Ser Leu Met Ser Ser Glu Leu Ala Arg His Ser Lys Arg
                405                 410                 415
Val Arg Ile Ala Pro Lys Val Phe Gly Glu Gln Val Val Phe Gly Tyr
            420                 425                 430
Met Ser Lys Phe Phe Ser Gly Asp Leu Arg Asp Phe Gly Thr Pro Ile
        435                 440                 445
Thr Ser Leu Phe Asn Phe Ile Phe Leu Cys Leu Ser Val Leu Leu Ala
    450                 455                 460
Glu Glu Gly Ile Ala Pro Leu Ser Ser Ala Gly Pro Gly Lys Glu Glu
465                 470                 475                 480
Lys Leu Leu Phe Gly Glu Gly Phe Ser Pro Leu Leu Pro Val Gln Thr
                485                 490                 495
Ile Lys Glu Glu Glu Ile Gln Pro Gly Glu Glu Met Pro His Leu Ala
            500                 505                 510

Arg Pro Ile Lys Val Glu Ser Pro Pro Leu Glu Glu Trp Pro Ser Pro
        515                 520                 525
Ala Pro Ser Phe Lys Glu Glu Ser Ser His Ser Trp Glu Asp Ser Ser
    530                 535                 540
Gln Ser Pro Thr Pro Arg Pro Lys Lys Ser Tyr Ser Gly Leu Arg Ser
545                 550                 555                 560
Pro Thr Arg Cys Val Ser Glu Met Leu Val Ile Gln His Arg Glu Arg
                565                 570                 575
Arg Glu Arg Ser Arg Ser Arg Arg Lys Gln His Leu Leu Pro Pro Cys
            580                 585                 590
Val Asp Glu Pro Glu Leu Leu Phe Ser Glu Gly Pro Ser Thr Ser Arg
        595                 600                 605
Trp Ala Ala Glu Leu Pro Phe Pro Ala Asp Ser Ser Asp Pro Ala Ser
    610                 615                 620
Gln Leu Ser Tyr Ser Gln Glu Val Gly Gly Pro Phe Lys Thr Pro Ile
625                 630                 635                 640
Lys Glu Thr Leu Pro Ile Ser Ser Thr Pro Ser Lys Ser Val Leu Pro
                645                 650                 655
Arg Thr Pro Glu Ser Trp Arg Leu Thr Pro Pro Ala Lys Val Gly Gly
            660                 665                 670
Leu Asp Phe Ser Pro Val Gln Thr Ser Gln Gly Ala Ser Asp Pro Leu
        675                 680                 685
Pro Asp Pro Leu Gly Leu Met Asp Leu Ser Thr Thr Pro Leu Gln Ser
    690                 695                 700
Ala Pro Pro Leu Glu Ser Pro Gln Arg Leu Leu Ser Ser Glu Pro Leu
705                 710                 715                 720
Asp Leu Ile Ser Val Pro Phe Gly Asn Ser Ser Pro Ser Asp Ile Asp
                725                 730                 735
Val Pro Lys Pro Gly Ser Pro Glu Pro Gln Val Ser Gly Leu Ala Ala
            740                 745                 750
Asn Arg Ser Leu Thr Glu Gly Leu Val Leu Asp Thr Met Asn Asp Ser
        755                 760                 765
Leu Ser Lys Ile Leu Leu Asp Ile Ser Phe Pro Gly Leu Asp Glu Asp
    770                 775                 780
Pro Leu Gly Pro Asp Asn Ile Asn Trp Ser Gln Phe Ile Pro Glu Leu
785                 790                 795                 800
Gln

<210> 33
<211> 1697
<212> DNA
<213> Homo sapiens

<220>
<221> source
<222> 1..1697
<223> /mol_type="unassigned DNA"
      /organism="Homo sapiens"

<400> 33
gaggcgtaag ccaggcgtgt taaagccggt cggaactgct ccggagggca cgggctccgt      60

aggcaccaac tgcaaggacc cctccccctg cgggcgctcc catggcacag ttcgcgttcg     120

agagtgacct gcactcgctg cttcagctgg atgcacccat ccccaatgca ccccctgcgc     180

gctggcagcg caaagccaag gaagccgcag gcccggcccc ctcacccatg cgggccgcca     240

accgatccca cagcgccggc aggactccgg gccgaactcc tggcaaatcc agttccaagg     300

ttcagaccac tcctagcaaa cctggcggtg accgctatat cccccatcgc agtgctgccc     360

agatggaggt ggccagcttc ctcctgagca aggagaacca gcctgaaaac agccagacgc 420

ccaccaagaa ggaacatcag aaagcctggg ctttgaacct gaacggtttt gatgtagagg 480

aagccaagat ccttcggctc agtggaaaac cacaaaatgc gccagagggt tatcagaaca 540

gactgaaagt actctacagc caaaaggcca ctcctggctc cagccggaag acctgccgtt 600

acattccttc cctgccagac cgtatcctgg atgcgcctga aatccgaaat gactattacc 660

tgaaccttgt ggattggagt tctgggaatg tactggccgt ggcactggac aacagtgtgt 720

acctgtggag tgcaagctct ggtgacatcc tgcagctttt gcaaatggag cagcctgggg 780

aatatatatc ctctgtggcc tggatcaaag agggcaacta cttggctgtg ggcaccagca 840

gtgctgaggt gcagctatgg gatgtgcagc agcagaaacg gcttcgaaat atgaccagtc 900

actctgcccg agtgggctcc ctaagctgga acagctatat cctgtccagt ggttcacgtt 960

ctggccacat ccaccaccat gatgttcggg tagcagaaca ccatgtggcc acactgagtg 1020

gccacagcca ggaagtgtgt gggctgcgct gggccccaga tggacgacat ttggccagtg 1080

gtggtaatga taacttggtc aatgtgtggc ctagtgctcc tggagagggt ggctgggttc 1140

ctctgcagac attcacccag catcaagggg ctgtcaaggc cgtagcatgg tgtccctggc 1200

agtccaatgt cctggcaaca ggagggggca ccagtgatcg acacattcgc atctggaatg 1260

tgtgctctgg ggcctgtctg agtgccgtgg atgcccattc ccaggtgtgc tccatcctct 1320

ggtctcccca ttacaaggag ctcatctcag gccatggctt tgcacagaac cagctagtta 1380

tttggaagta cccaaccatg gccaaggtgg ctgaactcaa aggtcacaca tcccgggtcc 1440

tgagtctgac catgagccca gatggggcca cagtggcatc cgcagcagca gatgagaccc 1500

tgaggctatg gcgctgtttt gagttggacc ctgcgcggcg gcgggagcgg gagaaggcca 1560

gtgcagccaa aagcagcctc atccaccaag gcatccgctg aagaccaacc catcacctca 1620

gttgtttttt atttttctaa taaagtcatg tctcccttca tgtttttttt ttaaaaaaaa 1680

aaaaaaaaaa aaaaaaa 1697

<210> 34
<211> 499
<212> PRT
<213> Homo sapiens

<400> 34
Met Ala Gln Phe Ala Phe Glu Ser Asp Leu His Ser Leu Leu Gln Leu
1               5                   10                  15
Asp Ala Pro Ile Pro Asn Ala Pro Pro Ala Arg Trp Gln Arg Lys Ala
            20                  25                  30
Lys Glu Ala Ala Gly Pro Ala Pro Ser Pro Met Arg Ala Ala Asn Arg
        35                  40                  45
Ser His Ser Ala Gly Arg Thr Pro Gly Arg Thr Pro Gly Lys Ser Ser
    50                  55                  60
Ser Lys Val Gln Thr Thr Pro Ser Lys Pro Gly Gly Asp Arg Tyr Ile

65                  70                  75                  80
Pro His Arg Ser Ala Ala Gln Met Glu Val Ala Ser Phe Leu Leu Ser
                85                  90                  95
Lys Glu Asn Gln Pro Glu Asn Ser Gln Thr Pro Thr Lys Lys Glu His
            100                 105                 110
Gln Lys Ala Trp Ala Leu Asn Leu Asn Gly Phe Asp Val Glu Glu Ala
        115                 120                 125
Lys Ile Leu Arg Leu Ser Gly Lys Pro Gln Asn Ala Pro Glu Gly Tyr
    130                 135                 140
Gln Asn Arg Leu Lys Val Leu Tyr Ser Gln Lys Ala Thr Pro Gly Ser
145                 150                 155                 160
Ser Arg Lys Thr Cys Arg Tyr Ile Pro Ser Leu Pro Asp Arg Ile Leu
                165                 170                 175
Asp Ala Pro Glu Ile Arg Asn Asp Tyr Tyr Leu Asn Leu Val Asp Trp
            180                 185                 190
Ser Ser Gly Asn Val Leu Ala Val Ala Leu Asp Asn Ser Val Tyr Leu
        195                 200                 205
Trp Ser Ala Ser Ser Gly Asp Ile Leu Gln Leu Leu Gln Met Glu Gln
    210                 215                 220
Pro Gly Glu Tyr Ile Ser Ser Val Ala Trp Ile Lys Glu Gly Asn Tyr
225                 230                 235                 240
Leu Ala Val Gly Thr Ser Ser Ala Glu Val Gln Leu Trp Asp Val Gln
                245                 250                 255
Gln Gln Lys Arg Leu Arg Asn Met Thr Ser His Ser Ala Arg Val Gly
            260                 265                 270
Ser Leu Ser Trp Asn Ser Tyr Ile Leu Ser Ser Gly Ser Arg Ser Gly
        275                 280                 285
His Ile His His His Asp Val Arg Val Ala Glu His His Val Ala Thr
    290                 295                 300
Leu Ser Gly His Ser Gln Glu Val Cys Gly Leu Arg Trp Ala Pro Asp
305                 310                 315                 320
Gly Arg His Leu Ala Ser Gly Gly Asn Asp Asn Leu Val Asn Val Trp
                325                 330                 335
Pro Ser Ala Pro Gly Glu Gly Gly Trp Val Pro Leu Gln Thr Phe Thr
            340                 345                 350
Gln His Gln Gly Ala Val Lys Ala Val Ala Trp Cys Pro Trp Gln Ser
        355                 360                 365
Asn Val Leu Ala Thr Gly Gly Gly Thr Ser Asp Arg His Ile Arg Ile
    370                 375                 380
Trp Asn Val Cys Ser Gly Ala Cys Leu Ser Ala Val Asp Ala His Ser
385                 390                 395                 400
Gln Val Cys Ser Ile Leu Trp Ser Pro His Tyr Lys Glu Leu Ile Ser
                405                 410                 415
Gly His Gly Phe Ala Gln Asn Gln Leu Val Ile Trp Lys Tyr Pro Thr
            420                 425                 430
Met Ala Lys Val Ala Glu Leu Lys Gly His Thr Ser Arg Val Leu Ser
        435                 440                 445
Leu Thr Met Ser Pro Asp Gly Ala Thr Val Ala Ser Ala Ala Ala Asp
    450                 455                 460
Glu Thr Leu Arg Leu Trp Arg Cys Phe Glu Leu Asp Pro Ala Arg Arg
465                 470                 475                 480
Arg Glu Arg Glu Lys Ala Ser Ala Ala Lys Ser Ser Leu Ile His Gln
                485                 490                 495
Gly Ile Arg

## Description

The present invention relates to methods for establishing the presence, or absence, of a bladder tumour and/or establishing the prediction of prognosis and disease outcome for a human individual suffering from bladder cancer. The present invention further relates to the use of expression analysis of the indicated genes, or molecular markers, for establishing the presence, or absence, of a bladder tumour and/or establishing the prediction of prognosis and disease outcome for a human individual suffering from bladder cancer and to kit of parts for establishing the presence, or absence, of a bladder tumour and/or establishing the prediction of prognosis and disease outcome for a human individual suffering from bladder cancer.

Urinary bladder (or bladder) cancer is one of the most common cancers worldwide, with the highest incidence in industrialized countries. In the Western world, the chances of developing this type of cancer is 1 in 26, for women the chance is 1 in 90. Bladder cancer is the 4th most common cancer in men.

Two main histological types of bladder cancer are the urothelial cell carcinomas (UCC) and the squamous cell carcinomas (SCC). The UCCs are the most prevalent in Western and industrialized countries and are related to cigarette smoking and occupational exposure. The squamous cell carcinomas (SCC) are more frequently seen in some Middle Eastern and African countries where the schistosoma haematobium parasite is endemic.

In the Western world, 90% of the bladder tumours are UCCs, 3 to 5% are SCCs, and 1 to 2% are adenocarcinomas. Two third of the patients with UCC can be catogorized into non-muscle invasive bladder cancer (NMIBC) and one third in muscle invasive bladder cancer (MIBC).

In NMIBC, the disease is generally confined to the bladder mucosa (stage Ta, carcinoma in situ (CIS)) or bladder submucosa (stage T1). In MIBC, the patient has a tumour initially invading the detrusor muscle (stage T2), followed by the perivesical fat (stage T3) and the organs surrounding the bladder (stage T4). The management of these two types of UCC differs significantly. The management of NMIBC consists of transurethral resection of the bladder tumour (TURBT). However, after TURBT, 30% to 85% of patients develop recurrences. This high risk of recurrence makes bladder cancer one of the most prevalent human tumours.

Patients with NMIBC can be divided into 3 groups. 20% to 30% of patients have a relatively benign type of UCC, with a low recurrence rate. These low risk tumours do not exhibit progression. 40% to 50% of patients have so-called intermediate risk tumours. These patients often develop a superficial recurrence, but seldom progression. A small group of patients (20% to 30%) has a relatively aggressive superficial tumour at presentation and despite maximum treatment and 70% to 80% of these patients will have recurrent disease. 50% of these patients will develop muscle invasive disease associated with a poor prognosis. Therefore, there is a need to identify the patient group at risk for progression.

The primary treatment for MIBC is cystectomy. Despite this radical treatment, 50% of patients with primary MIBC develop metastases within 2 years after cystectomy and subsequently die of the disease. The 5-year tumour-specific survival of these patients is 55%. In comparison, patients with NMIBC have a 5-year tumour-specific survival of 88-90%. However, patients with MIBC who have a history of NMIBC, the 5-year tumour-specific survival drops to only 28%. These percentages emphasize the need for the identification of patients with a high risk of progression of their NMIBC.

The risk for progression and cancer related death is associated with tumour stage and grade. Currently, staging and grading of the tumour is used for making treatment decisions. Unfortunately, this procedure has led to overtreatment (*e.g*. cystectomy in patients who would have survived without this treatment) or undertreatment (*i.e*. patients with progressive disease dying after TURBT and who would have survived if they underwent cystectomy at an earlier stage). At present no reliable methods are available to accurately predict prognosis of individual patients with bladder cancer. The limited value of the established prognostic markers requires the analysis of new molecular parameters in predicting the prognosis and treatment of bladder cancer patients.

Bladder cancer is a genetic disorder driven by the progressive accumulation of multiple genetic and epigenetic changes. At the molecular level, these genetic changes result in uncontrolled cell proliferation, decreased cell death, invasion, and metastasis. The specific alterations in gene expression that occur as a result of interactions between various cellular pathways determine the biological behavior of the tumor, including growth, recurrence, progression, and metastasis, and may influence patient survival. To detect and monitor cancer and determine the likely prognosis, it is necessary to identify molecular markers of the disease that can be used in the clinic.

Considering the above, there is a need in the art for molecular markers capable of establishing the presence, or absence, of a bladder tumour and/or establishing the prediction of prognosis and disease outcome for a human individual suffering from bladder cancer. A suitable molecular marker preferably fulfils the following criteria:
1) it must be reproducible (intra- and inter- institutional); and
2) it must have an impact on clinical management.

It is an object of the present invention, amongst other objects, to meet at least partially, if not completely, the above stated needs of the art.

According to the present invention, the above object, amongst other objects, is met by bladder tumour markers and methods as outlined in the appended claims.

Specifically, the above object, amongst other objects, is met by an (*in vitro*) method for establishing the presence, or absence, of a bladder tumour in a human individual; or classification of the tumours according to aggressiveness, prediction of prognosis and/or disease outcome for a human individual suffering from bladder cancer comprising:
a) determining the expression of one or more genes chosen from the group consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and transcript cluster 2526896 in a sample originating from said human individual; and
b) establishing up, or down, regulation of expression of said one or more genes as compared to expression of said respective one or more genes in a sample originating from said human individual not comprising tumour cells or tissue, or from an individual, or group of individuals, not suffering from bladder cancer; and
c) establishing the presence, or absence, of a bladder tumour based on the established up- or down regulation of said one or more genes; or establishing the prediction of prognosis and disease outcome for a human individual suffering from bladder cancer based on the established up- or down regulation of said one or more genes.

According to the present invention, establishing the presence, or absence, of bladder cancer in a human individual preferably includes diagnosis, prognosis and/or prediction of disease survival.

It should be noted that the present method, when taken alone, does not suffice to diagnose an individual as suffering from bladder cancer. For this, a trained physician is required capable of taking into account factors not related to the present invention such as disease symptoms, history, pathology, general condition, age, sex, and/or other indicators. The present methods and molecular markers provide the trained physician with additional tools, or aids, to arrive at a reliable diagnosis.

According to the present invention, expression analysis comprises establishing an increased, or decreased, expression of a gene as compared to expression of this gene in non-bladder cancer tissue, i.e., under non-disease conditions.

For example, establishing an increased expression of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, or transcript cluster 2526896, as compared to expression of these genes under non-bladder cancer conditions, allows establishing the presence, or absence, of a bladder tumour in a human individual suspected of suffering from bladder cancer and allows establishing the prediction of prognosis and disease outcome for an individual patient suffering from bladder cancer.

INHBA: Inhibin βA is a ligand in the TGF-β superfamily. INHBA forms a disulphide-linked homodimer known as activin A. In cancer a biological mechanism is suggested that is centered on activin A induced TGF-β signalling.

CTHRC1: collagen triple helix repeat containing-1 is a 30 kDa secreted protein that has the ability to inhibit collagen matrix synthesis. It is typically expressed at epithelial-mesenchymal interfaces. CTHRC1 is a cell-type-specific inhibitor TGF-β. Increased CTHRC1 expression results in morphological cell changes, increased cell proliferation, and decreased apoptosis.

CHI3L1: Chitinase 3-like 1 is a member of the mammalian chitinase family. It has been suggested that CHI3L1 is associated with cancer cell proliferation, differentiation, metastatic potential, and extracellular tissue remodelling, but *in vivo* proofs are yet to be obtained.

COL10A1: controls growth and maturation of endochondral bone. Overexpression of COL10A1 was also found in advanced breast cancer tissue specimen. COL10A1 was identified as a gene with restricted expression in most normal tissues and elevated expression in many diverse tumour types.

FAP: Human fibroblast activation protein alpha is a 97-kDa membrane bound serine protease. FAP was found to be selectively expressed on fibroblasts within the tumour stroma or on tumour-associated fibroblasts in epithelial cancers (e.g. colon cancer, myeloma, esophagal cancer, gastric cancer, breast cancer).

ASPN: asporin is an extracellular matrix protein that belongs to the small leucine-rich repeat proteoglycan family of proteins. Its biological role is unknown, but there is an association between ASPN and various bone and joint diseases, including rheumatoid arthritis. ASPN binds to various growth factors, including TGFβ and BMP2. ASPN was found to be upregulated in invasive ductal and lobular carcinomas.

ADAMTS12 is a desintegrin and metalloprotease with thrombospondin motif. ADAMTS12 transcripts were only detected at significant levels in fetal lung, but not in any other analysed normal tissue. ADAMTS12 could be detected in gastric, colorectal, renal, and pancreatic carcinomas. ADAMTS12 may play roles in pulmonary cells during fetal development or in tumour processes through its proteolytic activity or as a molecule potentially involved in regulation of cell adhesion. In colon carcinomas, the expression of ADAMTS12 in fibroblasts is linked with an antiproliferative effect on tumour cells. It seems that ADAMTS12 is a novel anti-tumour proteinase that plays an important role in inhibiting tumour development in colorectal cancer.

IGF2BP2: Insulin-like growth factor-II mRNA-binding protein 2 (IMP2) belongs to a family of RNA-binding proteins implicated in mRNA localization, turnover and translational control. Translational control and mRNA localization are important mechanisms for control of gene expression in germ cells and during early embryogenesis. Although the fetal expression is prominent, data indicating that the proteins are also present in mature tissues have been accumulating. In colon cancer, IGF2BP2 transcripts were shown to exist in sense:antisense pairs, which may have a direct regulatory function.

PDCD1LG2: Programmed cell death 1 (PD-1) and its ligands, Programmed death ligand 1 (PD-L1) and PD-L2, have an important inhibitory function to play in the regulation of immune homeostasis and in the maintenance of peripheral tolerance. The selective blockade of these inhibitory molecules is an attractive approach to cancer immunotherapy. PD-L1 is upregulated by many human cancers. On the other hand, the role of PD-L2 in modulating immune responses is less clear, and its expression is more restricted compared to PD-L1, thus making it a less obvious target in cancer immunotherapy.

SFRP4: Secreted frizzled-related protein 4 (SFRP4) is a secreted protein with putative inhibitory activity of the Wnt-signaling cascade. Membranous SFRP4 expression predicted for biochemical relapse. In colorectal carcinoma, SFRP4 is upregulated, which is in contrast to other SFRP family members. In ovarian cancers, there is supporting evidence that SFRP4 acts as a tumour suppressor gene via the inhibition of the Wnt signalling pathway. Although the risk of invasive bladder cancer increases with the number of methylated SFRP genes, methylation of *sFRP-4* is not an independent predictor of bladder cancer and therefore an exception.

KRT6A: The keratin 6 (K6 or Krt6) gene family is comprised of three members, K6a, K6b, and K6hf (or Krt75). Only KRT6A is expressed in the mammary gland, and only in a very small fraction of mammary luminal epithelial cells.

TPX2: The microtubule-associated protein TPX2 (Xklp2) has been reported to be crucial for mitotic spindle which can bind to tubulin and induce microtubule polymerization. TPX2 mRNA is closely linked to increased or abnormal cell proliferation in malignant salivary gland tumours, breast cancer, endometrioid adenocarcinoma, neuroblastoma, pancreatic cancer, ovarian cancer and cervical cancer. An increased expression of TPX2 might reflect an advanced loss of cell cycle inhibitory mechanisms resulting in more aggressive tumours.

CCNB2: Cyclin B2 is a member of the cyclin protein family. Cyclins B1 and B2 are particularly critical for the maintenance of the mitotic state. Cyclin B2 has been found to be up-regulated in human tumors, such as colorectal cancer, lung cancer, pituitary cancer. Recently it was shown that circulating CCNB2 in serum was significantly higher in cancer patients than in normal controls. The CCNB2 mRNA level was correlated with cancer stage and metastases status of patients with lung cancer and digestive tract cancer.

ANLN: Anillin is a gene highly expressed in the brain and ubiquitously present in various tissues.ANLN is overexpressed in breast cancer, endometrial carcinomas and gastric cancer. A tumor-progression-related pattern of ANLN expression was found in breast, ovarian, kidney, colorectal, hepatic, lung, endometrial and pancreatic cancer.

FOXM1: The human cell cycle transcription factor Forkhead box M1 is known to play a key role in regulating timely mitotic progression and accurate chromosomal segregation during cell division. Deregulation of FOXM1 has been linked to a majority of human cancers. Up-regulation of FOXM1 precedes malignancy in a number of solid cancers including oral, oesophagus, lung, breast, kidney bladder and uterus cancer. It is an early molecular signal required for aberrant cell cycle and cancer initiation.

CDC20: cell division cycle 20 homolog is a component of the mammalian cell cycle mechanism that activates the anaphase-promoting complex (APC). Its expression is essential for cell division. P53 was found to inhibit tumor cell growth through the indirect regulation of CDC20. CDC20 was found to be upregulated in many types of malignancies like ovarian cancer, bladder cancer, glioblastomas, pancreatic ductal carcinomas. In ovarian cancer and non-small cell lung cancer CDC20 appears to be associated with a poor prognosis. It has been suggested that CDC20 may function as an oncoprotein that promotes the development and progression of human cancers.

According to the present invention, the method as described above is preferably an *ex vivo* or *in vitro* method. In this embodiment, expression analysis of the indicated genes is performed on a sample derived, originating or obtained from an individual suspected of suffering from bladder cancer. Such sample can be a body fluid such as saliva, lymph, blood or urine, or a tissue sample such as a transurethral resection of a bladder tumour (TURBT). Samples of, derived or originating from blood, such as plasma or cells, and urine, such as urine sediments, are preferably contemplated within the context of the present invention as are samples of, derived or originating from TURBT specimens.

According to another preferred embodiment of the present method, determining the expression comprises determining mRNA expression of the said one or more genes.

Expression analysis based on mRNA is generally known in the art and routinely practiced in diagnostic labs world-wide. For example, suitable techniques for mRNA analysis are Northern blot hybridisation and amplification based techniques such as PCR, and especially real time PCR, and NASBA.

According to a particularly preferred embodiment, expression analysis comprises high-throughput DNA array chip analysis not only allowing the simultaneous analysis of multiple samples but also an automatic processing of the data obtained.

According to another preferred embodiment of the present method, determining the expression comprises determining protein levels of the said genes. Suitable techniques are, for example, matrix-assisted laser desorption-ionization time-of-flight mass spectrometer (MALDI-TOF).

According to the present invention, the present method is preferably provided by expression analysis of a number of the present genes selected from the group consisting of two or more, three or more, four or more, five or more, six or more, seven or more, eight or more, nine or more, ten or more, eleven or more, twelve or more, thirteen or more, fourteen or more, fifteen or more, sixteen or more, seventeen or more or eighteen of the genes chosen from the group consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896.

Preferred combinations within the context of the present invention are CCNB2 in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896, such as in combination with CDC20 and, preferably further in combination with PDCD1LG2, more preferably further in combination with INHBA, i.e. the combination at least comprising CCNB2, CDC20, PDCD1LG2 and INHBA. The latter panel of four markers provides a prediction of 0.991 (95%CI: 0.977-1.000). Within the present group of combinations with CCNB2, the preferred samples are urine or urine derived samples such as urine sediments.

Other preferred combinations within the context of the present invention are FAP in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896 such as in combination with CDC20 and, preferably, further in combination with INHBA, more preferably further in combination with IGF2BP2, i.e. the combination at least comprising FAP, CDC20, INHBA and IGF2BP2. Within the present group of combinations with FAP, the preferred samples are tissue or tissue derived samples such as biopses. The Area Under the Curve (AUC) for the combination of IGF2BP2+FAP+CHI3L1+CDC20 expression is 0.955 (95%CI: 0.929-0.980).

According to a most preferred embodiment of the above methods, the present invention relates to methods, wherein establishing the presence, or absence, of a tumour further comprises establishing suspected metastasis or no metastasis. Establishing whether the bladder tumour identified is capable to metastasize, is likely to metastasize, or has metastasized, is inherently a valuable tool for a trained physician to develop an individualised treatment protocol.

In case of metastasis, the survival rate of a patient is generally directly correlated with the point in time on which the metastasis is identified, detected or established. The earlier in time the treatment commences, the higher the survival rates. Additionally, if a tumour is not capable of metastasis, is not likely to metastasize, or has not metastasized, the patient needs not to be subjected to, or can be spared of, treatments severely affecting the quality of life.

Establishing the presence, or absence, of a tumour, according to another preferred embodiment, can further comprise establishing whether a NMIBC wil, or is likely to, progress into MIBC.

Considering the diagnostic- and/or prognostic value of the present markers, the present invention also relates to the use of expression analysis of one or more genes selected from the group consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and transcript cluster 2526896 for establishing the presence, or absence, of a bladder tumour or establishing the prediction of prognosis and disease outcome for an individual patient suffering from bladder cancer.

The present use, for reasons indicated above, is preferably an *ex vivo* or *in vitro* use and, preferably, involves the use of two or more, three or more, four or more, five or more, six or more, seven or more, eight or more, nine or more, ten or more, eleven or more, twelve or more, thirteen or more, fourteen or more, fifteen or more, sixteen or more, seventeen or more and eighteen of the present markers for establishing the presence, or absence of a bladder tumour, and establishing the prediction of prognosis and disease outcome for an individual patient suffering from bladder cancer.

Preferred combinations within the context of the present use are CCNB2 in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896, such as in combination with CDC20 and, preferably further in combination with PDCD1LG2, more preferably further in combination with INHBA, i.e. the combination at least comprising CCNB2, CDC20, PDCD1LG2 and INHBA. The latter panel of four markers provides a prediction of 0.991 (95%CI: 0.977-1.000). Within the present group of combinations with CCNB2, the preferred samples are urine or urine derived samples such as urine sediments.

Other preferred combinations within the context of the present use are FAP in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896 such as in combination with CDC20 and, preferably, further in combination with INHBA, more preferably further in combination with IGF2BP2, i.e. the combination at least comprising FAP, CDC20, INHBA and IGF2BP2. Within the present group of combinations with FAP, the preferred samples are tissue or tissue derived samples such as biopses. The Area Under the Curve (AUC) for the combination of IGF2BP2+FAP+CHI3L1+CDC20 expression is 0.955 (95%CI: 0.929-0.980).

Considering the diagnostic and/or prognostic value of the present genes as biomarkers for bladder cancer, the present invention also relates to a kit of parts for establishing the presence, or absence, of a bladder tumour and establishing the prediction of prognosis and disease outcome for an individual patient suffering from bladder cancer said kit of parts comprises:
- expression analysis means for determining the expression of one or more genes chosen from the group consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and transcript cluster 2526896;
- instructions for use.

Preferred combinations included in the present kits are CCNB2 in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896, such as in combination with CDC20 and, preferably further in combination with PDCD1LG2, more preferably further in combination with INHBA, i.e. the combination at least comprising CCNB2, CDC20, PDCD1LG2 and INHBA.

Other preferred combinations included in the present kits are FAP in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896 such as in combination with CDC20 and, preferably, further in combination with INHBA, more preferably further in combination with IGF2BP2, i.e. the combination at least comprising FAP, CDC20, INHBA and IGF2BP2.

According to a preferred embodiment, the present kit of parts comprises mRNA expression analysis means, preferably for PCR, rtPCR or NASBA.

According to a particularly preferred embodiment, the present kit of parts comprises means for expression analysis of two or more, three or more, four or more, five or more, six or more, seven or more, eight or more, nine or more, ten or more, eleven or more, twelve or more, thirteen or more, fourteen or more, fifteen or more, sixteen or more, seventeen or more or eighteen of the present genes.

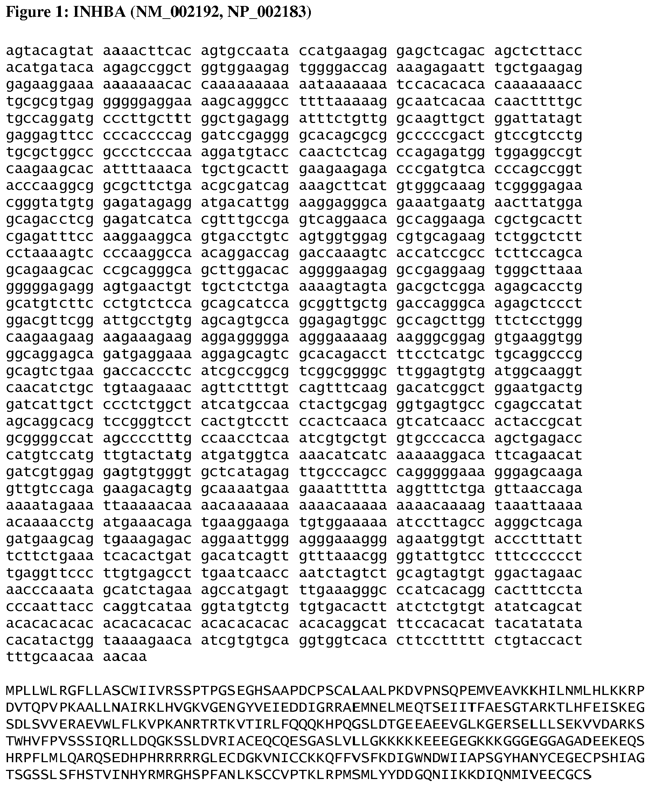

In the present description, reference is made to genes suitable as biomarkers for bladder cancer by referring to their arbitrarily assigned names. Although the skilled person is readily capable of identifying and using the present genes as biomarkers based on the indicated names, the appended figures 1 to 18 provide the cDNA and amino acid sequences of these genes, thereby readily allowing the skilled person to develop expression analysis assays based on analysis techniques commonly known in the art.

Such analysis techniques can, for example, be based on the genomic sequence of the gene or the provided cDNA or amino acid sequences. This sequence information can either be derived from the provided sequences, or can be readily obtained from public databases, for example by using the provided accession numbers.

The present invention will be further elucidated in the following examples of preferred embodiments of the invention. In the examples, reference is made to figures, wherein:
- **Figures 1-18:**: show the cDNA and amino acid sequences of the INHBA gene (NM_002192, NP_002183); the CTHRC1 gene (NM_138455, NP_612464); the CHI3L1 gene (NM_001276, NP_001267); the COL10A1 gene (NM_000493, NP_000484); the FAP gene (NM_004460, NP_004451); the sequence of transcript cluster 2526896 (no assigned mRNA and protein sequences); the ASPN gene (NM_017680, NP_060150); the sequence of transcript cluster 2526893 (no assigned mRNA and protein sequences); the ADAMTS12 gene (NM_030955, NP_112217); the IGF2BP2 gene (NM_006548, NP_006539); the PDCD1LG2 gene (NM_025239, NP_079515); the SFRP4 gene (NM_003014, NP_003005); the KRT6A gene (NM_005554, NP_005545); the TPX2 gene (NM_012112, NP_036244); the CCNB2 gene (NM_004701, NP_004692); the ANLN gene (NM_018685, NP_061155); the FOXM1 gene (NM_202002, NP_973731) and the CDC20 gene (NM_001255, NP_001246), respectively;
- **Figure 19:**: shows boxplots for the identified five best performing individual biomarkers that could distinguish NBl from BCa (NMIBC+MIBC) in tissue;
- **Figure 20:**: shows boxplots for the identified five best performing individual biomarkers that could distinguish MIBC tissue from NMIBC tissue;
- **Figure 21:**: shows boxplots for the identified best performing individual biomarkers for the detection of BCa in urine and/or that could distinguish MIBC from NMIBC in urine;
- **Figure 22:**: shows receiver under Operation Curves (ROC) showing a combination of biomarkers for predicting the occurrence of BCa (NMIBC and MIBC) based on the expression of the markers in urine. The Area Under the Curve (AUC) for the combination of CCNB2+CDC20+PDCD1LG2+INHBA expression in urine is 0.991 (95%CI: 0.977-1.000)
- **Figure 23:**: shows Receiver under Operation Curves (ROC) showing a combination of biomarkers for predicting the occurrence of MIBC based on the expression of the markers in tissue. The Area Under the Curve (AUC) for the combination of IGF2BP2+FAP+CHI3L1+CDC20 expression in tissue is 0.955(95%CI: 0.929-0.980).

Below the present invention will be further illustrated by examples of preferred embodiments of the present invention.

### EXAMPLES

### Example 1:

To identify markers for bladder cancer, the gene expression profile (GeneChip® Human Exon 1.0 ST arrays, Affymetrix) of samples from patients with and without bladder cancer were used. The expression analysis was performed according to standard protocols.

Briefly, tissue was obtained after radical cystectomy from patients with bladder cancer. The tissues were snap frozen and cryostat sections were hematoxylin-eosin (H.E.) stained for classification by a pathologist.

Malignant- and and non-malignant areas were dissected and total RNA was extracted with TRIpure® (Roche, Indianapolis, IN, CA, USA) following manufacturer's instructions. Total RNA was purified with the Qiagen RNeasy mini kit (Qiagen, Valencia, CA, USA). The integrity of the RNA was checked by electrophoresis using the Agilent 2100 Bioanalyzer.

From the purified total RNA, 1 µg was used for the GeneChip® Whole Transcript (WT) Sense Target Labeling Assay. (Affymetrix, Santa Clara, CA, USA). Using a random hexamer incorporating a T7 promoter, double-stranded cDNA was synthesized.

Then, cRNA was generated from the double-stranded cDNA template through an *in vitro* transcription reaction and purified using the Affymetrix sample clean-up module. Single-stranded cDNA was regenerated through a random-primed reverse transcription using a dNTP mix containing dUTP. The RNA was hydrolyzed with RNaseH and the cDNA was purified. Subsequently, the cDNA was fragmented by incubation with a mixture of UDG (uracil DNA glycosylase) and APE1 (apurinic/apyrimidinic endonuclease 1) restriction endonucleases and, finally, end-labeled via a terminal transferase reaction incorporating a biotinylated dideoxynucleotide. Of the fragmented, biotinylated cDNA, 5.5 µg was added to a hybridization mixture, loaded on a GeneChip® Human Exon 1.0 ST array and hybridized for 16 hours at 45 °C and 60 rpm.

Using the GeneChip® Human Exon 1.0 ST array, genes are indirectly measured by exon analysis which measurements can be combined into transcript clusters measurements. There are more than 300,000 transcript clusters on the array, of which 90,000 contain more than one exon. Of these 90,000 there are more than 17,000 high confidence (CORE) genes which are used in the default analysis. In total there are more than 5.5 million features per array.

Following hybridization, the array was washed and stained according to the Affymetrix protocol. The stained array was scanned at 532 nm using a GeneChip® Scanner 3000, generating CEL files for each array.

Exon-level and gene level expression values were derived from the CEL file probe-level hybridization intensities using Partek Genomics Suite 6.2, (Partek Incorporated, Saint Louis, MO, USA). Data analysis with this software was performed with the GeneChip® array core meta probe sets as well as the extended meta probe sets.

Differentially expressed genes between conditions, *e.g.* NMIBC *versus* MIBC and MIBC *versus* NBl, are calculated using Anova (ANalysis Of Variance), a T-test for more than two groups. The target identification is biased since clinically well-defined risk groups were analyzed. The markers are categorized based on their role in cancer biology. For the identification of markers the non-muscle invasive bladder cancer (NMIBC) group (N=48), the muscle invasive bladder cancer (MIBC) group (N=49), the bladder cancer metastasis (BC-Meta) group (N=5) and the normal bladder(NBl) group (N=12) were compared.

Based on the GeneChip® microarrays expression analysis data, the most differentially expressed genes between NBl and NMIBC/MIBC (diagnostic genes) and also the most differentially expressed genes between the NMIBC and MIBC (prognostic genes) were selected.
In total, a group of 46 genes of interest were selected which will be further elucidated in example 2 and listed in **Table 2.** Based on the selected 18 genes in example 2, the GeneChip® expression data for these genes are shown in **Table 1.**

### Table 1: GeneChip® Microarray data showing the expression characteristics of 18 targets characterizing bladder cancer tissue, based on the analysis of 12 well annotated NBl, 48 NMIBC, 49 MIBC and 5 BC-Meta tissue specimens.

**Table 1A:**

| Gene | Gene | Gene | **up in MIBC vs NMIBC** | | up in MIBC vs NBl | |
|---|---|---|---|---|---|---|
| symbol | Name | assignment | Fold-Change | P-value | Fold-Change | P-value |
| INHBA | inhibin, beta A | NM_002192 | **10.1** | 3.3E-12 | 5.7 | 1.7E-8 |
| CTHRC1 | collagen triple helix repeat containing 1 | NM_138455 | **4.5** | 5.1E-22 | 2.4 | 7.9E-6 |
| CHI3L1 | chitinase 3-like 1 (cartilage glycoprotein-39) | NM_001276 | **13.5** | 1.5E-19 | 6.8 | 4.1E-7 |
| COL10A1 | collagen, type X, alpha 1 | NM_000493 | **3.7** | 1.0E-16 | 3.8 | 1.3E-9 |
| FAP | fibroblast activation protein, alpha | NM_004460 | **6.3** | 2.5E-17 | 3.5 | 1.3E-5 |
| TC2526896* | Transcript cluster 2526896, N/A** | N/A** | **7.6** | 1.1E-15 | 7.8 | 5.6E-9 |
| ASPN | Asporin | NM_017680 | **7.6** | 1.1E-16 | 2.0 | 2.7E-2 |
| TC2526893* | Transcript cluster 2526893, N/A** | N/A** | **4.2** | 1.2E-12 | 4.2 | 1.8E-7 |
| ADAMTS12 | ADAM metallopeptidase with thrombospondin type 1 motif, 12 | NM_016568 | **4.3** | 8.2E-21 | 3.8 | 1.9E-10 |
| IGF2BP2 | insulin-like growth factor 2 mRNA binding protein 2 | NM_006548 | **4.0** | 1.8E-12 | 2.7 | 2.5E-4 |
| PDCD1LG2 | programmed cell death 1 ligand 2 | NM_025239 | **5.6** | 1.2E-13 | 1.8 | 7.1E-2 |
| SFRP4 | secreted frizzled-related protein 4 | NM_003014 | **6.6** | 1.6E-12 | 2.9 | 3.5E-3 |
| KRT6A | keratin 6A | NM_005554 | **6.1** | 2.3E-07 | 4.6 | 2.6E-3 |

| | | | | | | |
|---|---|---|---|---|---|---|
| *data based on the GeneChip® extended meta probesets **N/A = there are no assigned mRNA sequences for this transcript cluster. | | | | | | |

**Table 1B:**

| Gene | Gene | Gene | **up in MIBC vs NBl** | | up in MIBC vs NMIBC | |
|---|---|---|---|---|---|---|
| symbol | Name | assignment | Fold-Change | P-value | Fold-Change | P-value |
| TPX2 | TPX2, microtubule-associated, homolog (Xenopus laevis) | NM_012112 | **18.4** | 1.6E-19 | 2.6 | 3.9E-07 |
| CCNB2 | cyclin B2 | NM_004701 | **10.0** | 3.6E-19 | 1.6 | 5.6E-04 |
| ANLN | anilin, actin binding protein | NM_018685 | **16.3** | 1.8E-18 | 3.1 | 2.0E-09 |
| FOXM1 | forkhead box M1 | NM_202002 | **8.5** | 5.5E-18 | 2.4 | 2.2E-09 |
| CDC20 | cell devision cycle 20 homolog | NM_001255 | **29.0** | 6.4E-18 | 2.4 | 6.4E-05 |

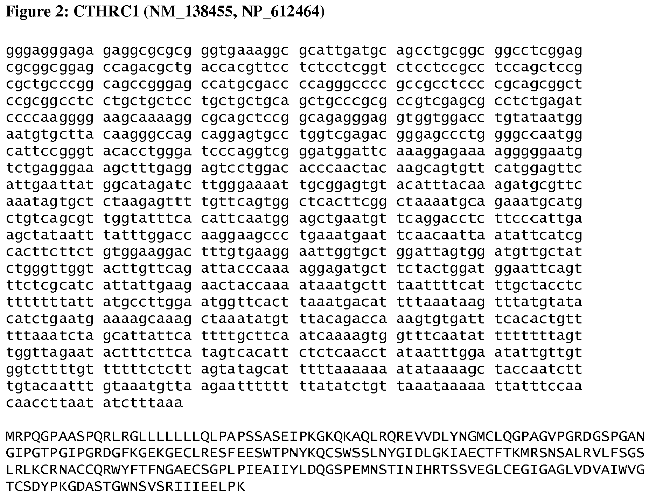

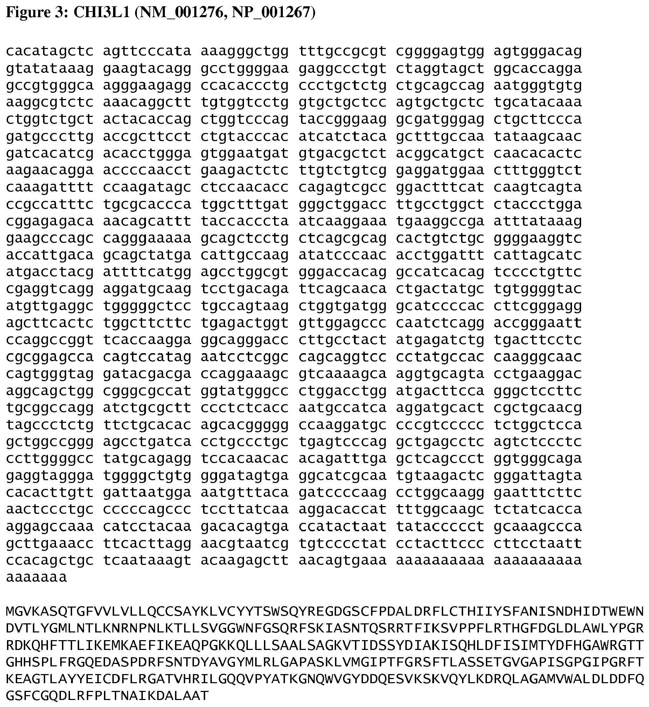

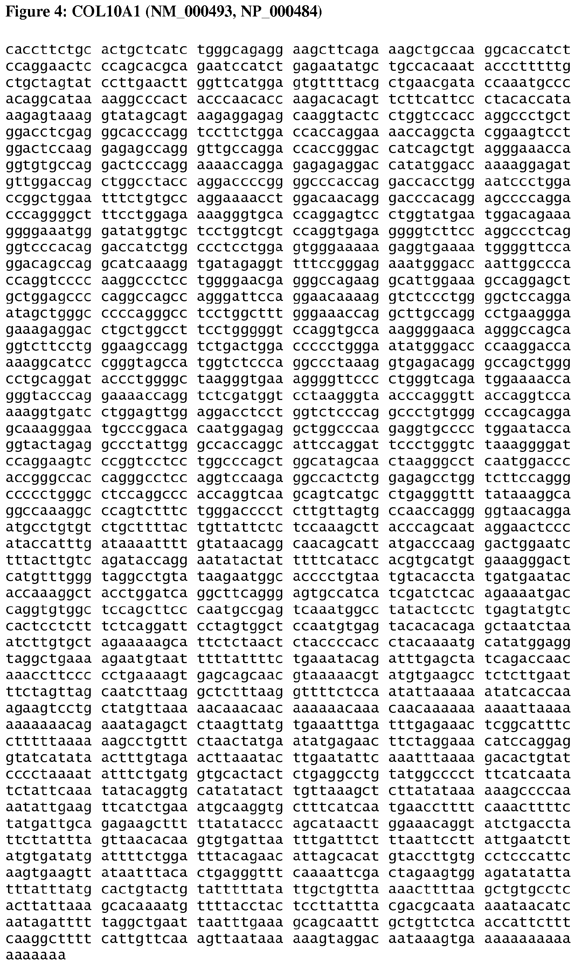

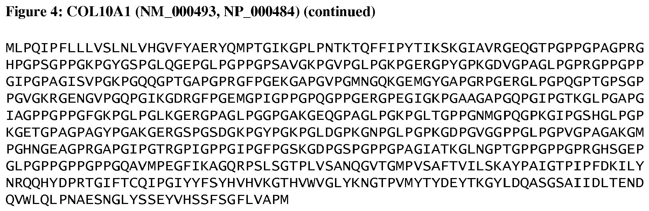

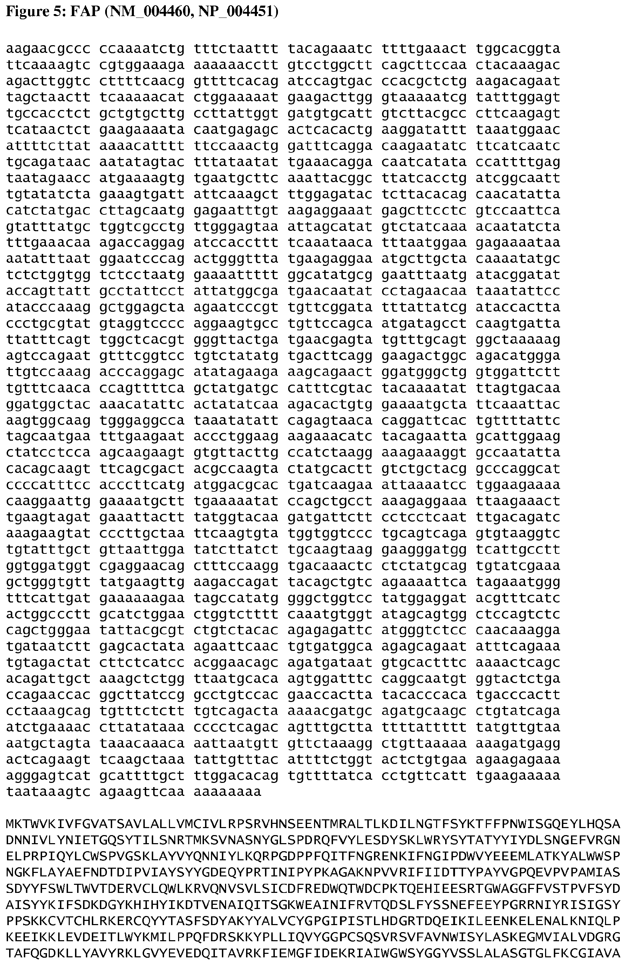

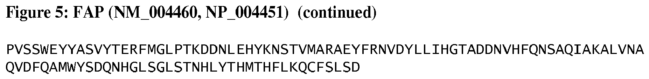

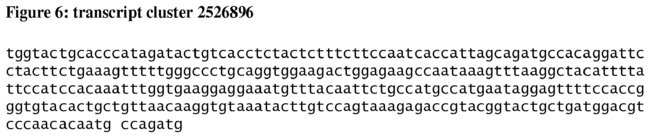

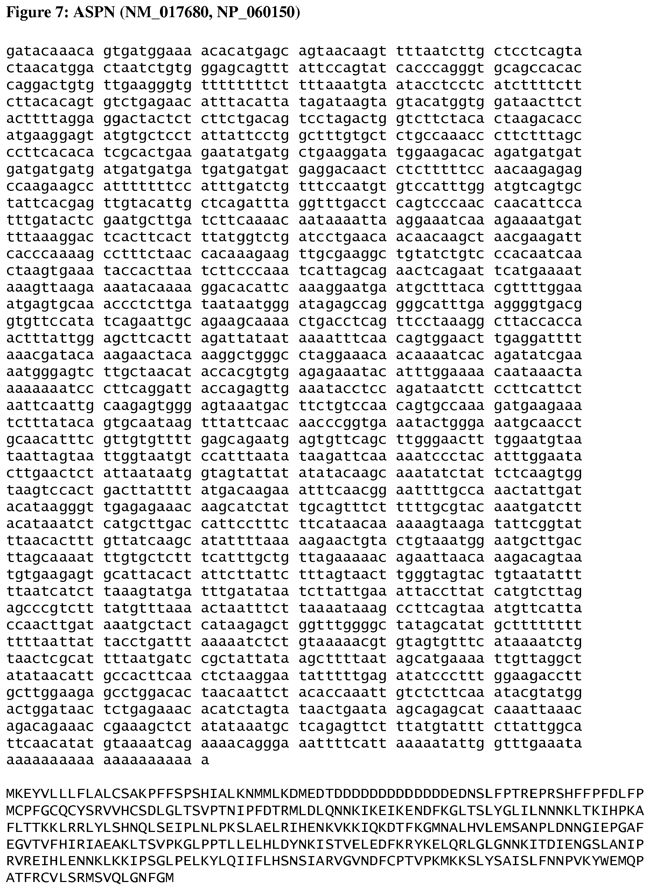

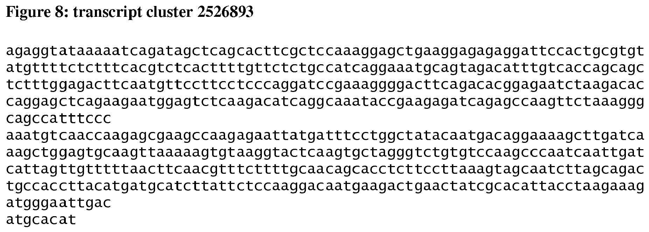

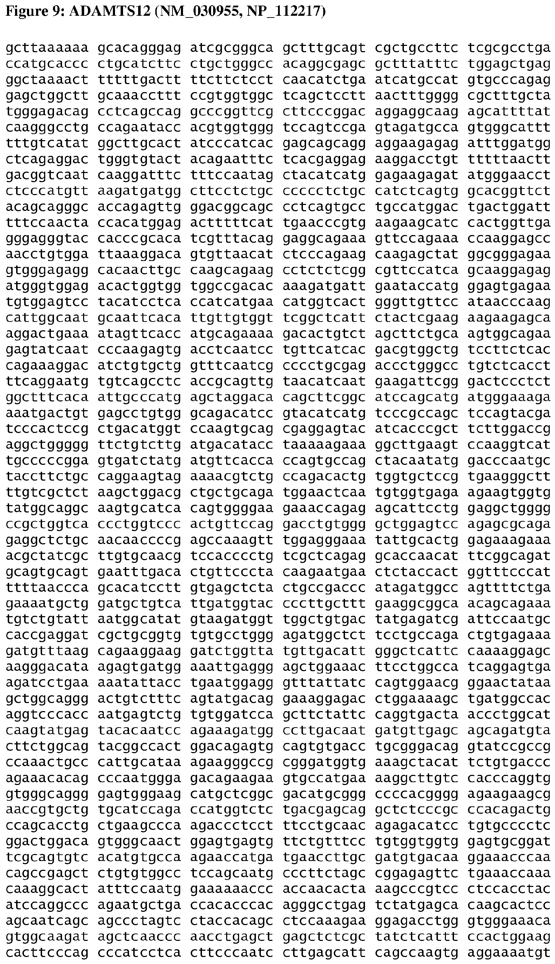

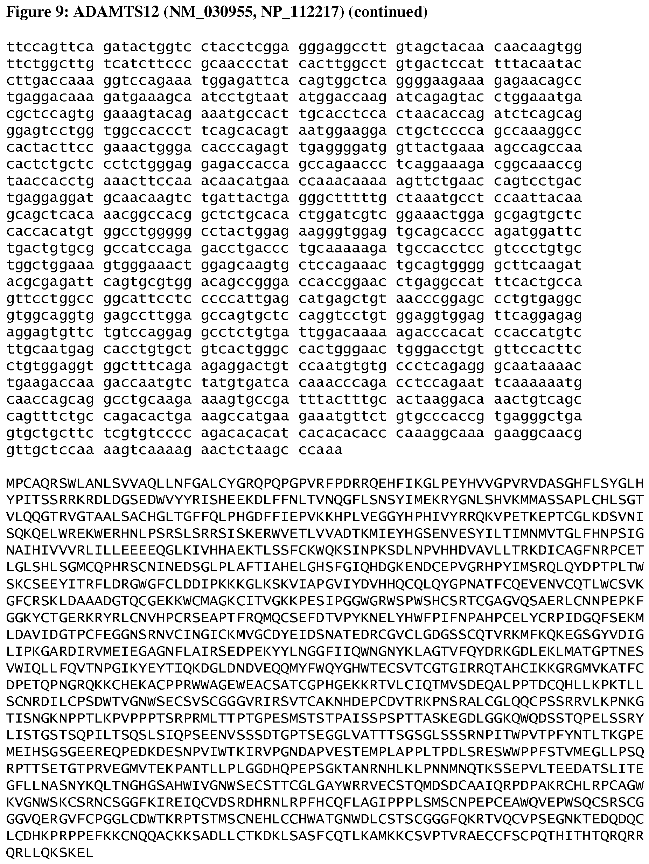

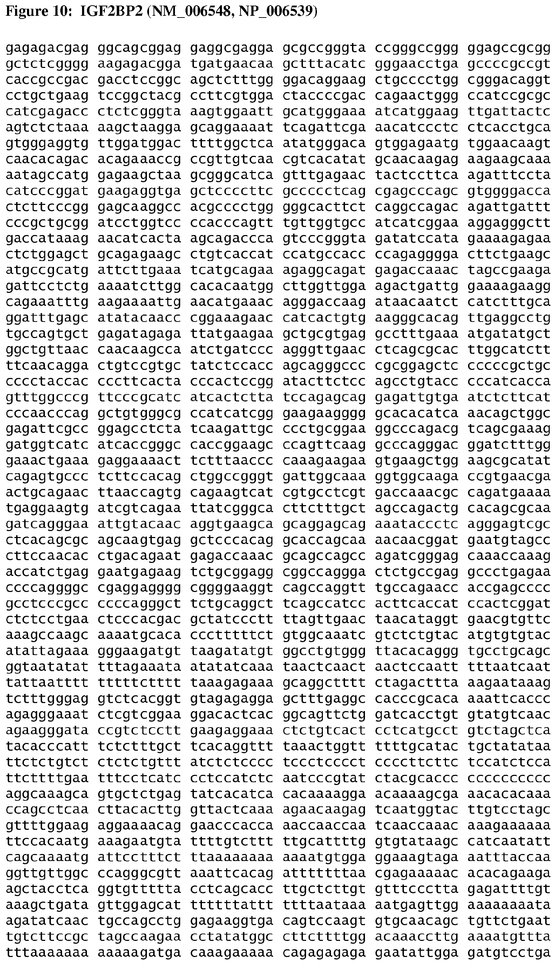

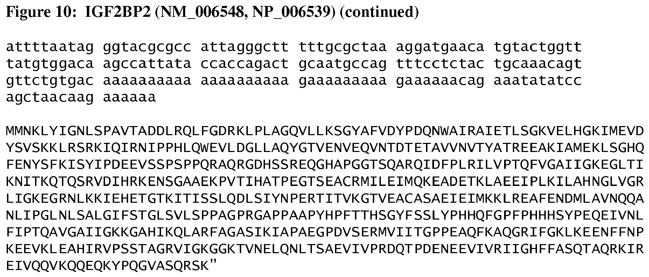

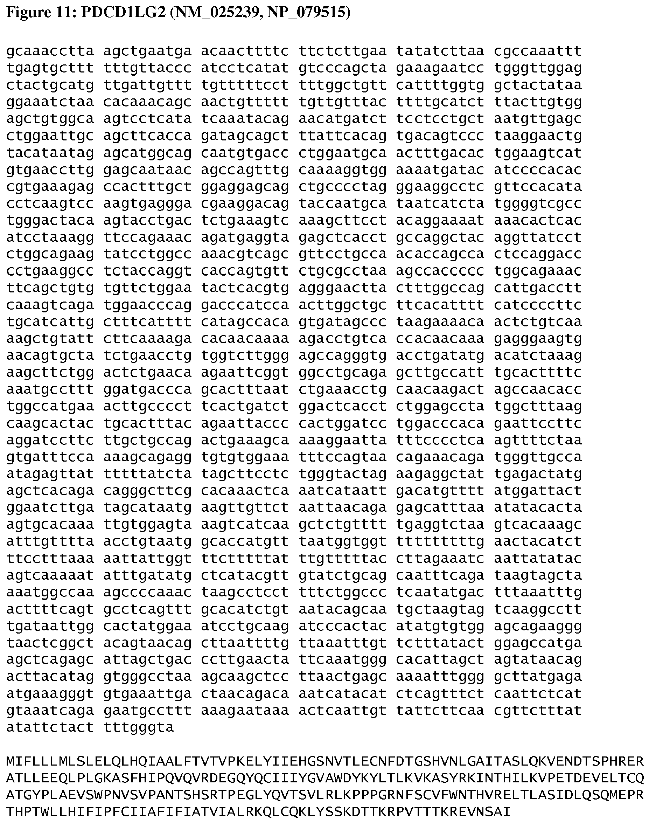

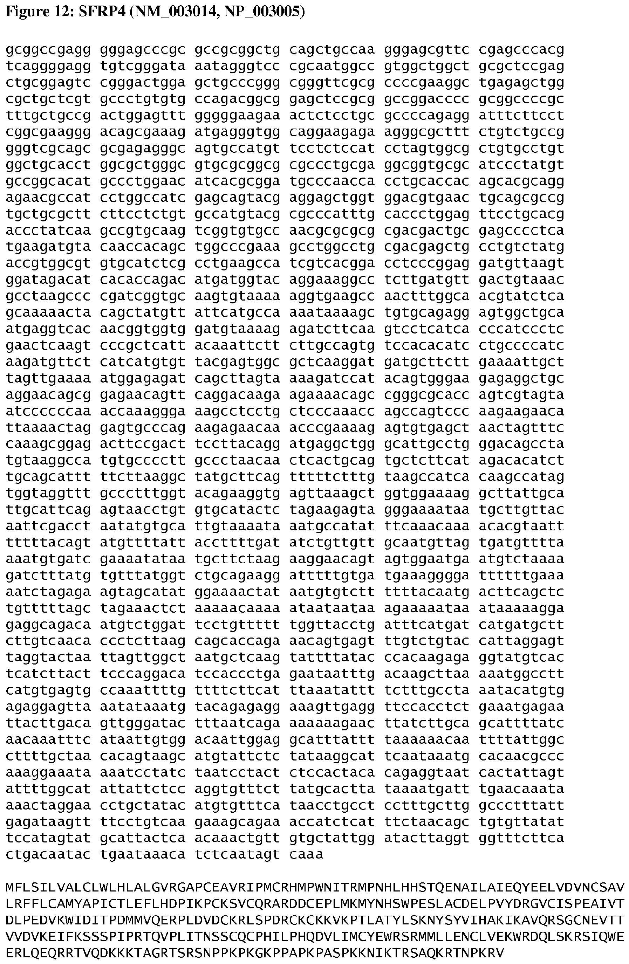

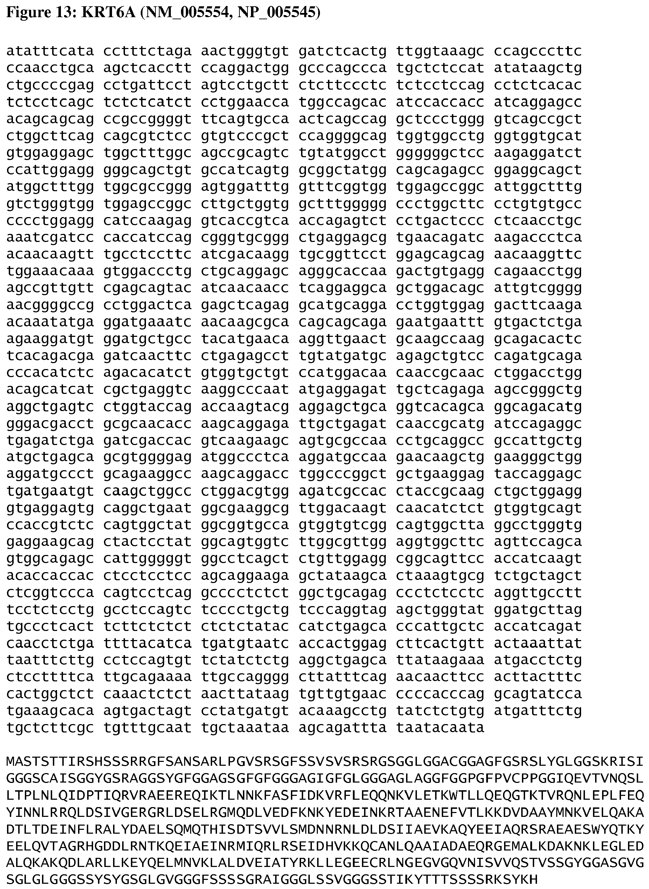

As can be clearly seen in **Table 1A** an up regulation of expression of INHBA (**Figures 1**), CTHRC1 (**Figures 2**), CHI3L1 (**Figures 3**), COL10A1 (**Figures 4**), FAP (**Figures 5**), transcript cluster 2526896 (**Figures 6**), ASPN (**Figures 7**), transcript cluster 2526893(**Figures 8**), ADAMTS12 (**Figures 9**), IGF2BP2 (**Figures 10**), PDCD1LG2 (**Figures 11**), SFRP4 (**Figure 12**), KRT6A (**Figure 13**) was associated with MIBC and as such has prognostic value. Eleven out of thirteen were identified using the core probe sets, two were identified using the extended probe sets and have no assigned mRNA sequence and gene symbol.

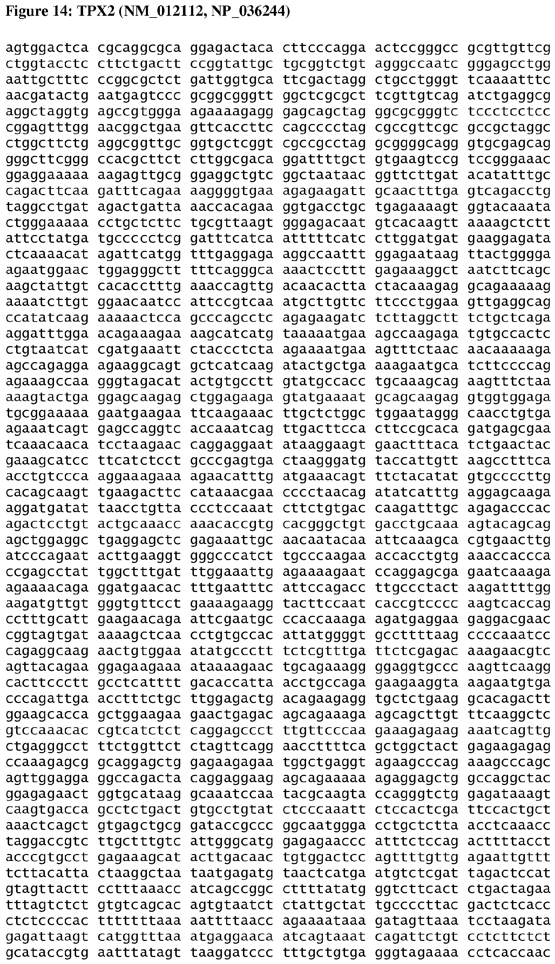

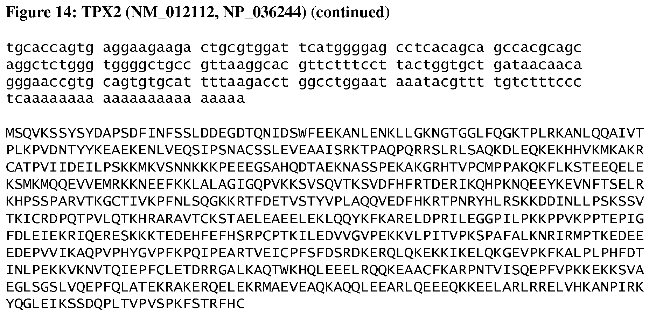

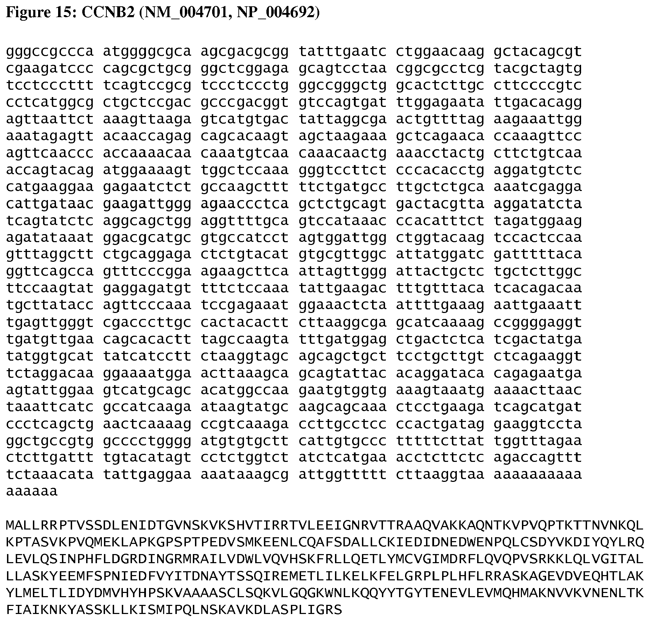

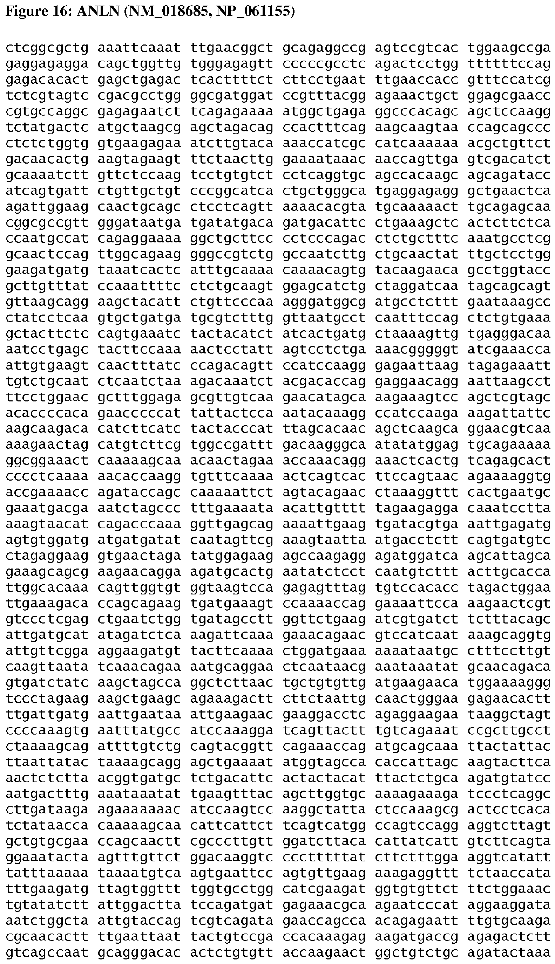

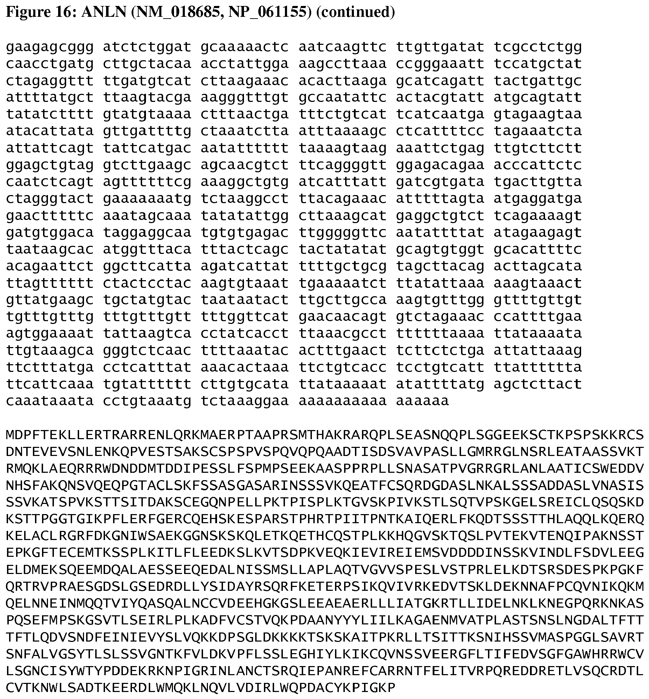

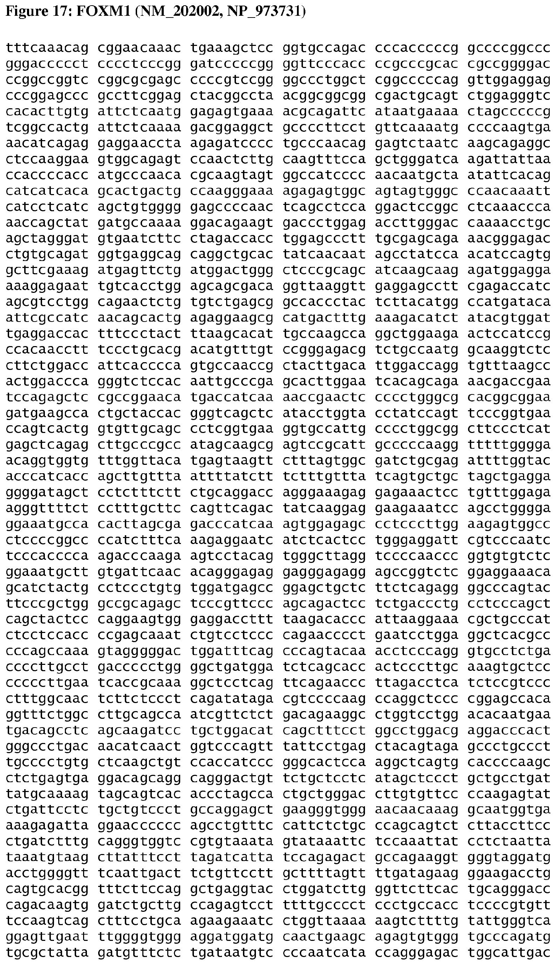

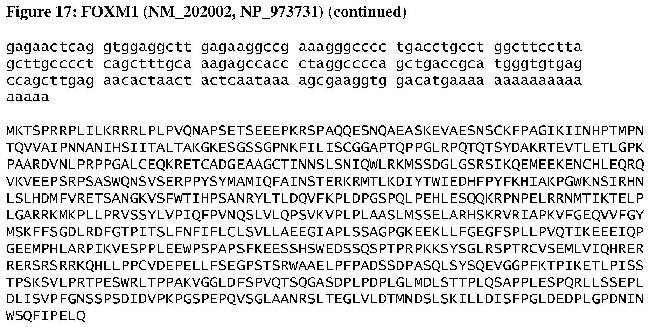

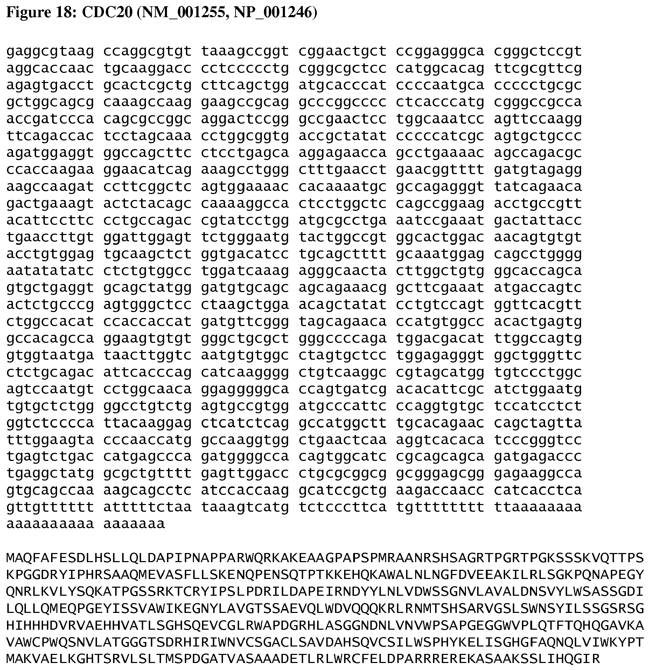

As can be clearly seen in **Table 1B** an up regulation of expression of TPX2 (**Figure 14**), CCNB2 (**Figure 15**), ANLN (**Figure 16**), FOXM1 (**Figure 17**) and CDC20 (**Figure 18**) was associated with the presence bladder cancer and as such has diagnostic value.

### Example 2

Using the gene expression profile (GeneChip® Human Exon 1.0 ST Array, Affymetrix) on 114 tissue specimens of normal bladder(NBl), non-muscle invasive bladder cancer (NMIBC), muscle invasive bladder cancer (MIBC) and bladder cancer metastasis (BC-Meta) several genes were found to be differentially expressed. The expression levels of 46 of these differentially expressed genes, together with the expression level of a housekeeping gene (GAPDH) and reference gene (TBP) were validated using the TaqMan® Low Density arrays (TLDA, Applied Biosystems). In **Table 2** an overview of the validated genes is shown.

**Table 2: Gene expression assays used for TLDA analysis**

| Gene symbol | Accesion nr. | Assay number |
|---|---|---|
| LOXL2 | NM_002318 | Hs00158757_m1 |
| INHBA | NM_002192 | Hs01081598_m1 |
| ADAMTS12 | NM_030955 | Hs00229594_m1 |
| CTHRC1 | NM_138455 | Hs00298917_m1 |
| SULF1 | NM_001128205 | Hs00290918_m1 |
| CHI3L1 | NM_001276 | Hs00609691_m1 |
| MMP11 | NM_005940 | Hs00968295_m1 |
| OLFML2B | NM_015441 | Hs00295836_m1 |
| CD109 | NM_133493 | Hs00370347_m1 |
| COL10A1 | NM_000493 | Hs00166657_m1 |
| NID2 | NM_007361 | Hs00201233_m1 |
| LOX | NM_002317 | Hs00942480_m1 |
| ADAMTS2 | NM_014244 | Hs01029111_m1 |
| FAP | NM_004460 | Hs00990806_m1 |
| GREM1 | NM_013372 | Hs01879841_s1 |
| WISP1 | NM_003882 | Hs00365573_m1 |
| ITGA11 | NM_001004439 | Hs00201927_m1 |
| ASPN | NM_017680 | Hs00214395_m1 |
| NTM | NM_001144058 | Hs00275411_m1 |
| PRR11 | NM_018304 | Hs00383634_m1 |
| BMP8A | NM_181809 | Hs00257330_s1 |
| SLC12A8 | NM_024628 | Hs00226405_m1 |
| SFRP4 | NM_003014 | Hs00180066_m1 |
| KRT6A | NM_005554 | Hs01699178_g1 |
| PDCD1LG2 | NM_025239 | Hs00228839_m1 |
| BCAT1 | NM_001178094 | Hs00398962_m1 |
| IGF2BP2 | NM_006548 | Hs01118009_m1 |
| TPX2 | NM_012112 | Hs00201616_m1 |
| CCNB2 | NM_004701 | Hs00270424_m1 |
| PLK1 | NM_005030 | Hs00153444_m1 |
| ANLN | NM_018685 | Hs01122612_m1 |
| AURKA | NM_198433 | Hs01582072_m1 |
| FOXM1 | NM_202002 | Hs01073586_m1 |
| CDC20 | NM_001255 | Hs00426680_mH |
| ECT2 | NM_018098 | Hs00216455_m1 |
| PLXNA1 | NM_032242 | Hs00413698_m1 |
| BUB1 | NM_004336 | Hs01557701_m1 |
| CKAP2 | NM_018204 | Hs00217068_m1 |
| TOP2A | NM_001067 | Hs00172214_m1 |
| TTK | NM_003318 | Hs01009870_m1 |
| CYB561D1 | NM_001134404 | Hs00699482_m1 |
| HMGB3 | NM_005342 | Hs00801334_s1 |
| SKP2 | NM_005983 | Hs01021864_m1 |
| FAPP6 | NM_001130958 | Hs01031183_m1 |
| FAM107A | NM_007177 | Hs00200376_m1 |
| NTRK3 | NM_001007156 | Hs00176797_m1 |
| TBP | NM_003194 | Hs00427620_m1 |
| GAPDH | NM_002046 | Hs99999905_m1 |

The validation with TLDA analysis was performed with 66 bladder tissue samples. Among these, 64 samples were newly selected and isolated, 2 normal bladder samples had been used before in the identification step with the GeneChip® Human Exon 1.0 ST Array.

Bladder cancer specimens in the following categories were used: Normal bladder (NBl, n=7), non-muscle invasive bladder cancer (NMIBC, n=29), muscle invasive bladder cancer (MIBC, n=27) and bladder cancer metastasis (BC-Meta, n=3).

To determine whether the identified biomarkers for bladder cancer could be used in a kit for specific detection in urine, 16 urinary sediments from patients suffering from bladder cancer (8 NMIBC and 8 MIBC) were included in the TLDA analysis and validation.

All tissue samples were snap frozen and cryostat sections were stained with hematoxylin and eosin (H.E.). These H.E.-stained sections were classified by a pathologist. Tumor areas were dissected. RNA was extracted from 10 µm thick serial sections that were collected from each tissue specimen at several levels. Tissue was evaluated by HE-staining of sections at each level and verified microscopically. Total RNA was extracted with TRIpure® (Roche, Indianapolis, IN, CA, USA) according to the manufacturer's instructions. Total RNA was purified using the RNeasy mini kit (Qiagen, Valencia, CA, USA).

The 16 urine samples of patients with bladder cancer were immediately cooled to 4°C and were processed within 48h after collection to guarantee good sample quality. The urine, EDTA stabilized, was centrifuged at 4°C and 1.800xg for 10 minutes. The obtained urinary sediment were washed twice with icecold buffered sodium chloride solution. On centrifugation at 4°C and 1.000xg for 10 minutes, the sediments were snap frozen in liquid nitrogen and stored at - 70°C. RNA was extracted from the urinary sediments using a modified TriPure reagent protocol. After the chloroform extraction, GlycoBlue was added to the aquous phase to precipitate the RNA using isopropanol. Total RNA from the sediments was used to generate amplified sense-strand cDNA using the Whole Transcriptome Expression kit according to the manufacturers protocol.

RNA quantity and quality were assessed on a NanoDrop 1000 spectrophotometer (NanoDrop Technologies, Wilmington, DE, USA) and on an Agilent 2100 Bioanalyzer (Agilent Technologies Inc., Santa Clara, CA, USA).

Two µg total RNA was eliminated from genomic DNA and reverse transcribed using the Quantitect® Reverse Transcription Kit Qiagen gMBH, Hilden, D) according to the manufacturer's instructions. Gene expression levels were measured using the TaqMan® Low Density Arrays (TLDA; Applied Biosystems).

A list of assays used in this study is given in **Table 2.** Of the individual cDNAs, 3 µl is added to 50 µl Taqman® Universal Probe Master Mix (Applied Biosystems)and 47 µl milliQ. One hundred µl of each sample was loaded into 1 sample reservoir of a TaqMan® Array (384-Well Micro Fluidic Card) (Applied Biosystems). The TaqMan® Array was centrifuged twice for 1 minute at 280g and sealed to prevent well-to-well contamination. The cards were placed in the micro-fluid card sample block of an 7900 HT Fast Real-Time PCR System (Applied Biosystems). The thermal cycle conditions were: 2 minutes 50°C, 10 minutes at 94.5°C, followed by 40 cycles for 30 seconds at 97°C and 1 minute at 59.7°C.

Raw data were recorded with the Sequence detection System (SDS) software of the instruments. Micro Fluidic Cards were analyzed with RQ documents and the RQ Manager Software for automated data analysis. Delta cycle threshold (Ct) values were determined as the difference between the Ct of each test gene and the Ct of glyceraldehyde 3-phosphate dehydrogenase (GAPDH) (endogenous control gene).

Furthermore, gene expression values were calculated based on the comparative threshold cycle (Ct) method, in which a normal bladder RNA sample was designated as a calibrator to which the other samples were compared.

For the validation of the differentially expressed genes found by the GeneChip® Human Exon 1.0 ST array, 66 bladder tissue specimens and 16 urinary sediments from bladder cancer patients were used in Taqman Low Density Arrays (TLDAs). In these TLDAs, expression levels were determined for the 48 genes of interest. The bladder tissue specimens were put in order from normal bladder, bladder cancer with low to high T-stage and finally bladder cancer metastasis.

Both GeneChip® Human Exon 1.0 ST array and TLDA data were analyzed using scatter- and box plots.

After analysis of the data a list of genes, shown in **Table 3,** was derived the expression of which is indicative for establishing the presence, or absence, of bladder tumour in a human individual suspected of suffering from bladder cancer comprising and, accordingly, indicative for bladder cancer and prognosis thereof.

**Table 3: List of genes identified**

| **Gene Symbol** | **Gene description** | **Figure** |
|---|---|---|
| INHBA | inhibin, beta A | 1 |
| CTHRC1 | collagen triple helix repeat containing 1 | 2 |
| CHI3L1 | chitinase 3-like 1 (cartilage glycoprotein-39) | 3 |
| COL10A1 | collagen, type X, alpha 1 | 4 |
| FAP | fibroblast activation protein, alpha | 5 |
| TC2526896* | transcript cluster 2526896, N/A** | 6 |
| ASPN | Aspirin | 7 |
| TC2526893* | transcript cluster 2526893, N/A** | 8 |
| ADAMTS12 | ADAM metallopeptidase with thrombospondin type 1 motif, 12 | 9 |
| IGF2BP2 | insulin-like growth factor 2 mRNA binding protein 2 | 10 |
| PDCD1LG2 | programmed cell death 1 ligand 2 | 11 |
| SFRP4 | secreted frizzled-related protein 4 | 12 |
| KRT6A | keratin 6A | 13 |
| TPX2 | TPX2, microtubule-associated, homolog (Xenopus laevis) | 14 |
| CCNB2 | cyclin B2 | 15 |
| ANLN | anilin, actin binding protein | 16 |
| FOXM1 | forkhead box M1 | 17 |
| CDC20 | cell devision cycle 20 homolog | 18 |

| | | |
|---|---|---|
| *data based on the GeneChip® extended meta probesets **N/A = there are no assigned mRNA sequences for this transcript cluster. | | |

Below detailed GeneChip® Human Exon 1.0 ST array data and TLDA validation data is presented for the 16 genes and only GeneChip® array data for the two transcript clusters, based on the groups normal bladder(NBl), non-muscle invasive bladder cancer (NMIBC), muscle invasive bladder cancer (MIBC) and bladder cancer metastasis (BC-Meta). For the identification of markers the non-muscle invasive bladder cancer (NMIBC) group, the muscle invasive bladder cancer (MIBC) group, the bladder cancer metastasis (BC-Meta) group and the normal bladder(NBl) group were compared.

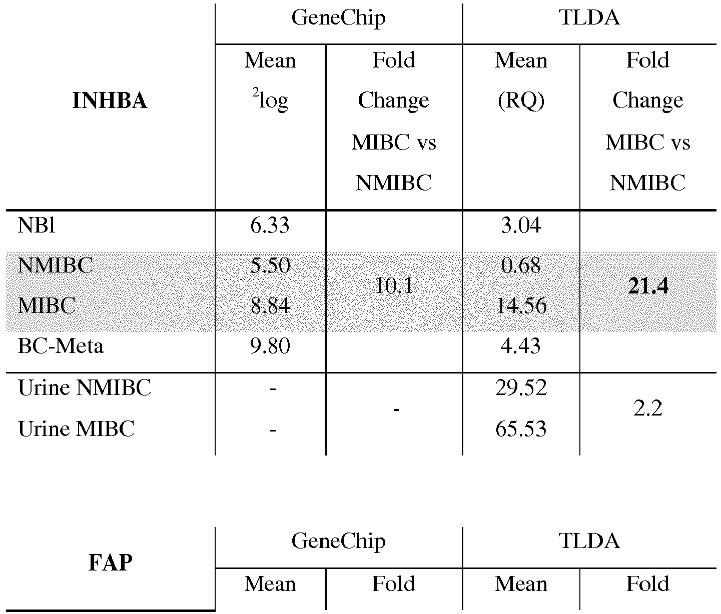

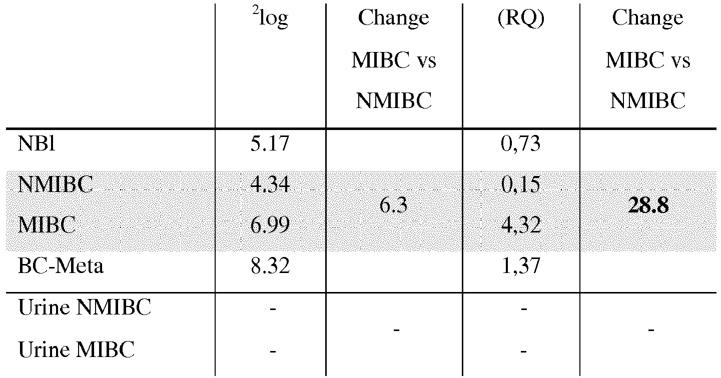

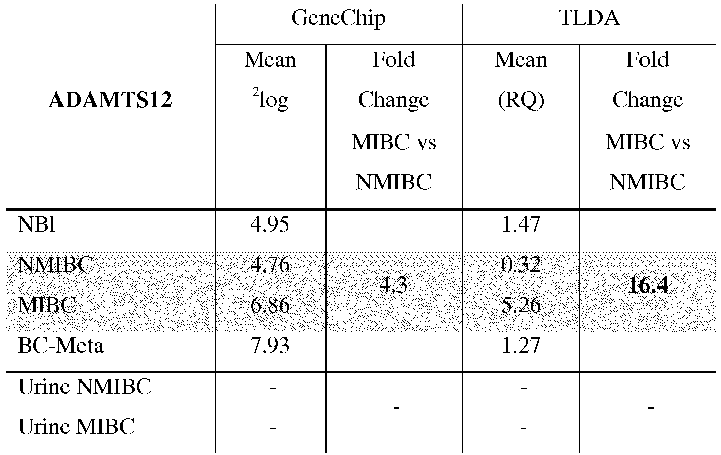

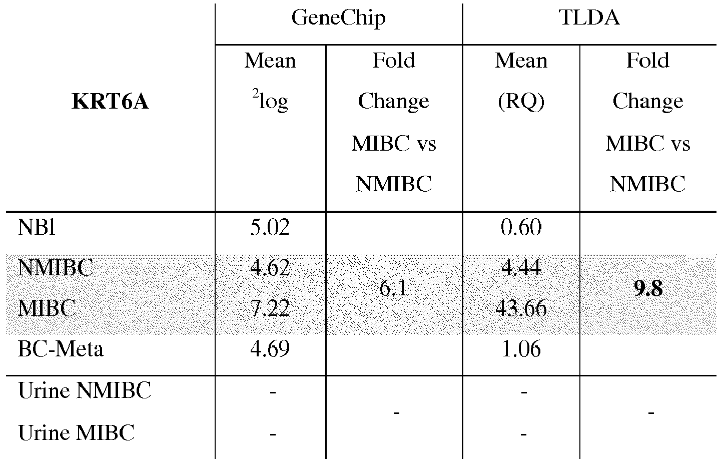

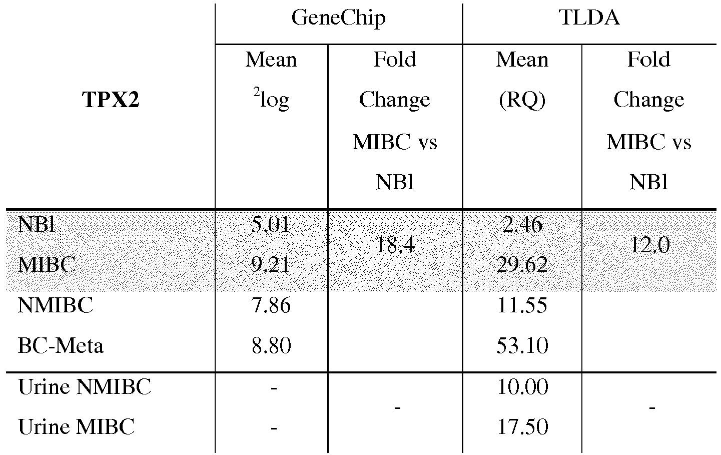

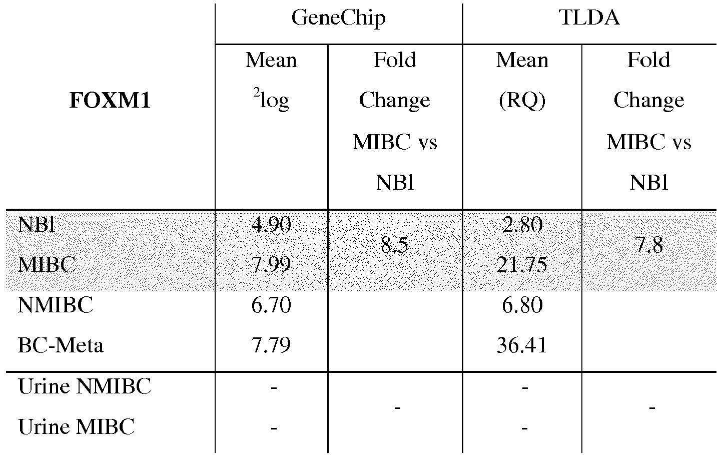

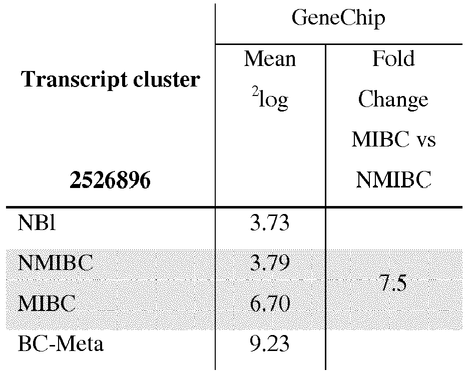

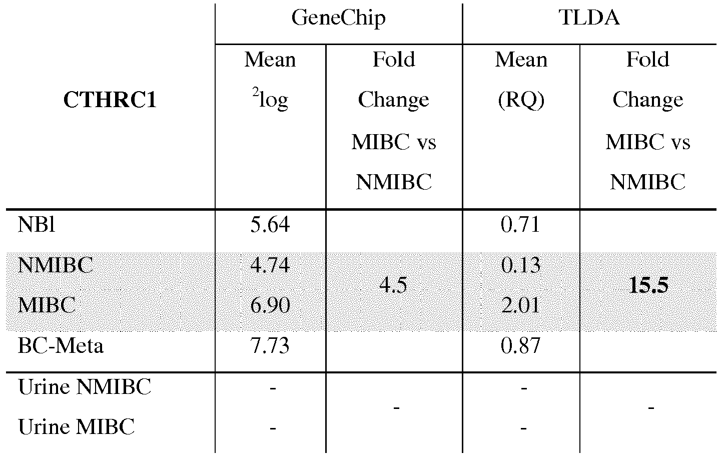

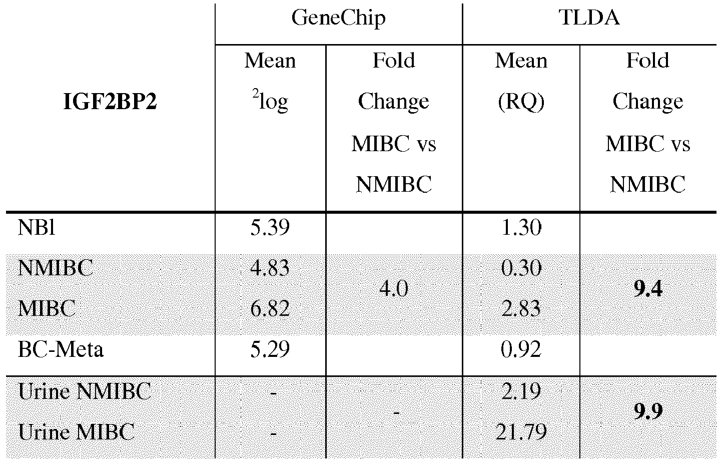

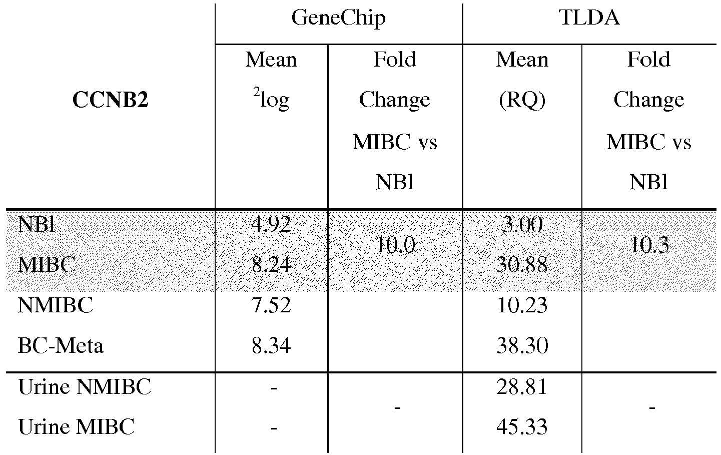

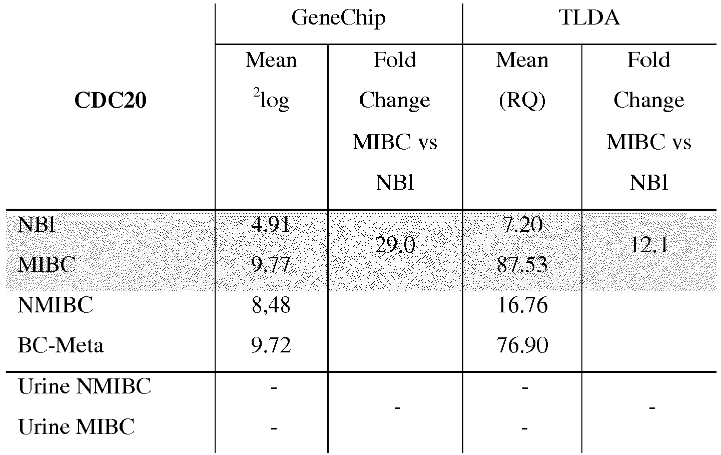

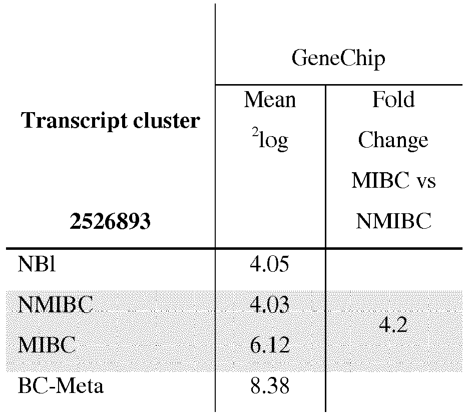

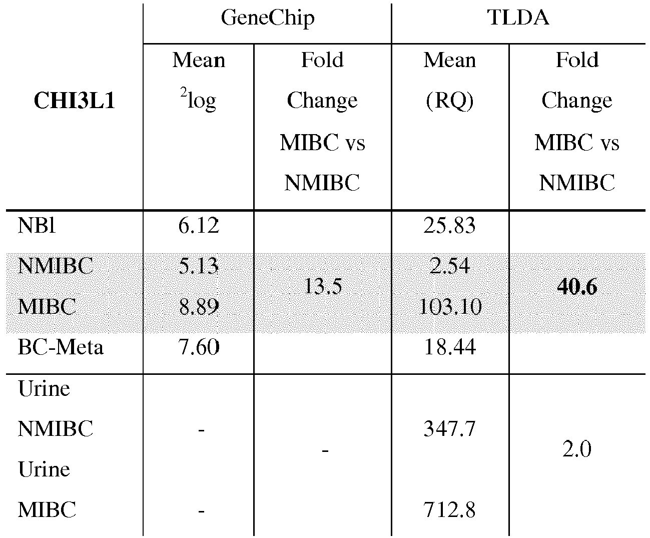

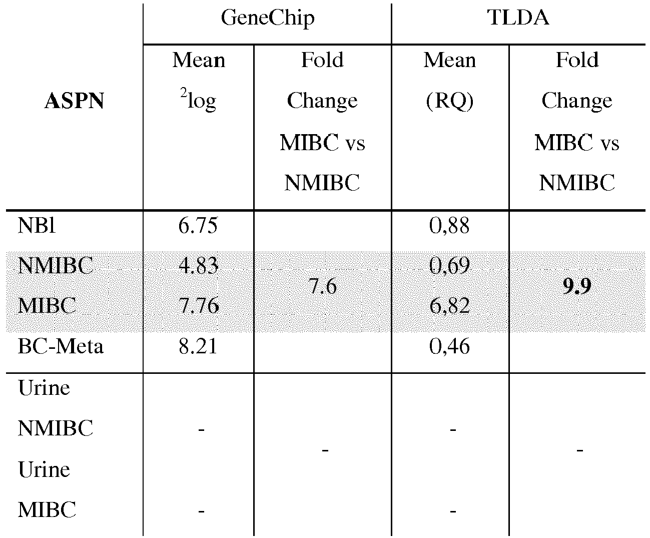

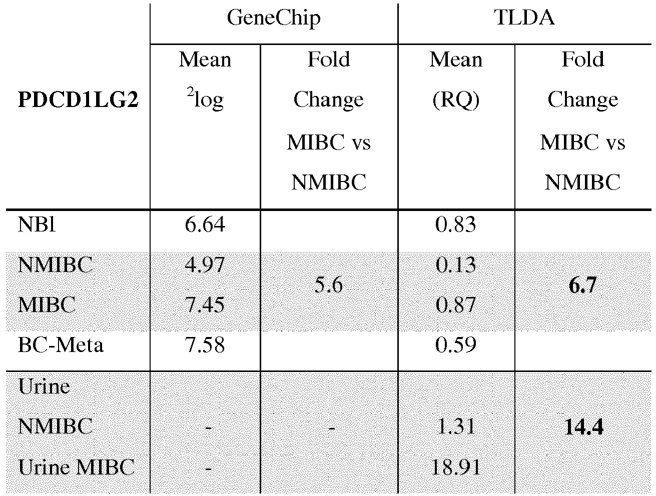

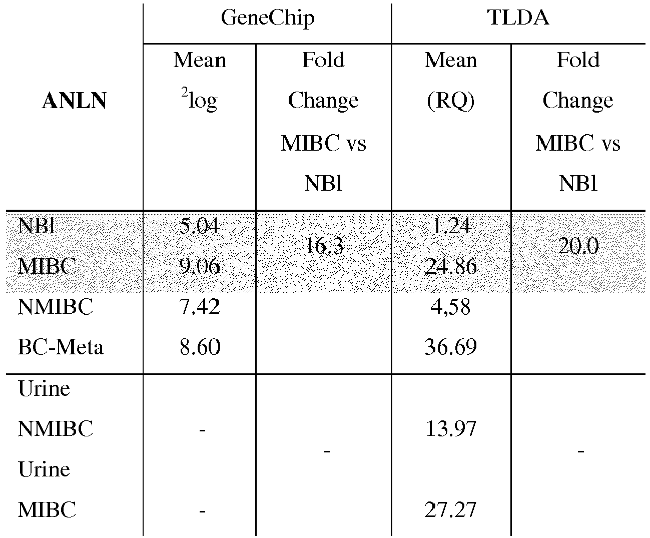

### Example 3:

The identified genes mentioned in example 2 and listed in **Table 3** were used for further validation and selection in a larger cohort of patient samples. For 17 of the 18 identified genes and for the control gene TBP used for normalization, fluorescence based real-time qPCR assays were designed and established according the MIQE guidelines. The performance of transcript clusters 2526896 and 2526893 were very similar. Therefore, no qPCR assay was established for transcript cluster 2526893. PCR products were cloned in either the pCR2.1-TOPO cloning vector (Invitrogen). Calibration curves with a wide linear dynamic range (10 - 1,000,000 copies) were generated using serial dilutions of the plasmids. The amplification efficiency of the primer pair was determined using the calibration curve and was >1.85. Control samples with known template concentrations were used as a reference. Two µl of each cDNA sample were amplified in a 20 µl PCR reaction containing optimized amounts of forward primer and reverse primer, 2 pmol of hydrolysis probe and 1x Probes Master mix (Roche, Cat No. 04902343001). The following amplification conditions were used: 95°C for 10 minutes followed by 50 cycles at 95°C for 10 seconds, 60°C for 30 seconds and a final cooling step at 40°C for 55 seconds (LightCycler LC480, Roche). The crossing point (Cp) values were determined using the Lightcycler 480 SW 1.5 software (Roche). The Cp values of the samples were converted to concentrations by interpolation in the generated calibration curve. The assay performance of the real-time PCR experiments was evaluated during in-study validation. The reference control samples had an inter- and intra-assay variation < 30%.

Total RNA was extracted from bladder tissue and urinary sediments and used for reverse transcription to generate cDNA. In total 211 bladder tissue specimen and 100 urinary sediments were used. The group of 206 bladder tissue specimen consisted of 10 normal bladders, 124 NMIBC, 72 MIBC. The group of 100 urinary sediments consisted of urinary sediments from 15 healthy controls (defined as normal), and from 65 patients with NMIBC, and 18 patients with MIBC.

Statistical analyses were performed with SPSS® version 20.0. All data were log-transformed prior to statistical analysis as a transformation to a normal distribution. Two-tailed P values of 0.05 or less were considered to indicate statistical significance. The nonparametric Mann Whitney test (for continuous variables) was used to test if biomarker levels were significantly correlated with the presence of BCa and/or BCa prognosis (muscle invasiveness, metastasis).
The assay results for the 17 selected biomarkers are shown in **Tables 4-7.**

**Table 4: Absolute and relative expression of the 17 biomarkers in NBl and BCa tissue**

| | copy numbers | | | copy numbers | | | relative exp pression¹ | | | |
|---|---|---|---|---|---|---|---|---|---|---|
| | NBl | | | BCa (NMIBC+MIBC) | | | NBl N=10 | BCa N=196 | Fold Change | P-value |
| | N=10 | | | N=196 | | | | | | |
| Biomarker | Mean | Median | Range | Mean | Median | Range | Mean | Mean | | MW² |
| CTHRC1 | 1041 | 1263 | 1-1810 | 3129 | 835 | 1-42200 | 917.8 | 1891.7 | 2.1 | 0.29 |
| IGF2BP2 | 203 | 130 | 69-651 | 544 | 85 | 0-5750 | 182.1 | 248.8 | 1.4 | 0.74 |
| ADAMTS12 | 91 | 74 | 24-259 | 472 | 103 | 1-4180 | 75.5 | 275.8 | 3.7 | 0.31 |
| INHBA | 411 | 437 | 30-869 | 3472 | 386 | 1-61500 | 400.5 | 1895.9 | 4.7 | 0.38 |
| SFRP4 | 231 | 92 | 55-896 | 1365 | 26 | 1-37300 | 201.7 | 911.5 | 4.5 | 0.18 |
| FAP | 568 | 403 | 146-1440 | 1239 | 270 | 1-12500 | 469.2 | 738.0 | 1.6 | 0.52 |
| CHI3L1 | 81 | 52 | 1-339 | 1468 | 158 | 1-21700 | 104.5 | 883.2 | 8.5 | 0.45 |
| COL10A1 | 275 | 205 | 58-585 | 2991 | 984 | 1-88200 | 225.6 | 1881.5 | 8.3 | 0.56 |
| ASPN | 548 | 205 | 23-3500 | 608 | 210 | 1-15700 | 397.8 | 372.0 | -1.1 | 0.8 |
| ANLN | 92 | 35 | 1-412 | 1297 | 772 | 17-8000 | 56.9 | 521.8 | 9.2 | <0.05 |
| TPX2 | 73 | 28 | 1-325 | 1768 | 964 | 1-13000 | 45.8 | 715.0 | 15.6 | <0.05 |
| FOXM1 | 57 | 16 | 1-251 | 806 | 397 | 1-5650 | 32.9 | 271.3 | 8.2 | <0.05 |
| CCNB2 | 156 | 47 | 1-669 | 2160 | 1485 | 1-11100 | 93.8 | 811.1 | 8.6 | <0.05 |
| CDC20 | 185 | 54 | 1-795 | 2727 | 1650 | 1-22000 | 122.0 | 1057.5 | 8.7 | <0.05 |
| KRT6A | 1983 | 26 | 1-19500 | 32220 | 132 | 1-1470000 | 1386.7 | 12483.6 | 9.0 | 0.48 |
| PDCD1LG2 | 288 | 223 | 119-536 | 570 | 336 | 1-3440 | 262.1 | 298.0 | 1.1 | 0.14 |
| TC2526896 | 3 | 1 | 1-11 | 110 | 1 | 1-1920 | 1.9 | 63.8 | 33.6 | 0.67 |
| TBP | 1230 | 1058 | 492-2820 | 2890 | 2585 | 77-9320 | - | - | - | - |

| | | | | | | | | | | |
|---|---|---|---|---|---|---|---|---|---|---|
| Relative expression¹: ratio (copy numbers biomarker/copy number TBP)*1000 MW²: Mann-Whitney test | | | | | | | | | | |

**Table 5: Absolute and relative expression of the 17 biomarkers in NMIBC and MIBC tissue**

| | copy numbers | | | copy numbers | | | relative expression¹ | | | | |
|---|---|---|---|---|---|---|---|---|---|---|---|
| | NMIBC | | | MIBC | | | NMIBC N=124 | MIBC N=72 | Fold Change | P-value | P-value |
| | N=124 | | | N=72 | | | | | | | |
| Biomarker | Mean | Median | Range | Mean | Median | Range | Mean | Mean | | MW² | T-test |
| CTHRC1 | 898 | 585 | 24-5360 | 6972 | 4625 | 1-42200 | 364.6 | 4521.7 | 12.4 | <0.05 | 3.5E-20 |
| IGF2BP2 | 206 | 38 | 0-1950 | 1126 | 611 | 1-5750 | 74.2 | 549.4 | 7.4 | <0.05 | 1.0E-20 |
| ADAMTS12 | 124 | 65 | 1-1880 | 1070 | 515 | 1-4180 | 48.2 | 667.9 | 13.9 | <0.05 | 1.4E-19 |
| | 529 | 230 | 1-7440 | 8540 | 3775 | 29-61500 | 183.0 | 4846.0 | 26.5 | <0.05 | 1.1E-22 |
| SFRP4 | 76 | 1 | 1-2300 | 3567 | 509 | 1-37300 | 41.6 | 2367.6 | 56.9 | <0.05 | 3.3E-23 |
| FAP | 323 | 167 | 1-2510 | 2817 | 1915 | 39-12500 | 135.2 | 1776.3 | 13.1 | <0.05 | 9.6E-34 |
| CHI3L1 | 475 | 37 | 1-21700 | 3177 | 1420 | 15-14800 | 170.6 | 2110.5 | 12.4 | <0.05 | 2.6E-30 |
| COL10A1 | 1087 | 804 | 1-4670 | 6271 | 2040 | 1-88200 | 337.4 | 4540.6 | 13.5 | <0.05 | 2.3E-10 |
| ASPN | 223 | 134 | 1-1450 | 1271 | 551 | 1-15700 | 105.7 | 830.6 | 7.9 | <0.05 | 2.2E-18 |
| ANLN | 1001 | 582 | 17-6930 | 1806 | 1380 | 25-8000 | 318.5 | 871.9 | 2.7 | <0.05 | 2.6E-16 |
| TPX2 | 1358 | 579 | 1-8760 | 2474 | 1620 | 1-13000 | 430.5 | 1205.1 | 2.8 | <0.05 | 3.2E-10 |
| FOXM1 | 723 | 336 | 1-5650 | 948 | 515 | 1-3660 | 196.1 | 400.8 | 2.0 | <0.05 | 4.6E-07 |
| CCNB2 | 2075 | 1275 | 17-11100 | 2306 | 1575 | 1-7360 | 637.3 | 1110.4 | 1.7 | <0.05 | 6.2E-07 |
| CDC20 | 2062 | 1210 | 1-15300 | 3873 | 2445 | 1-22000 | 642.0 | 1773.0 | 2.8 | <0.05 | 3.7E-10 |
| KRT6A | 2257 | 71 | 1-181000 | 83822 | 2320 | 1-1470000 | 721.9 | 32739.8 | 45.4 | <0.05 | 1.3E-11 |
| PDCD1LG2 | 477 | 292 | 1-3200 | 728 | 413 | 1-3440 | 185.4 | 492.0 | 2.7 | <0.05 | 6.3E-10 |
| TC2526896 | 14 | 1 | 1-606 | 276 | 70 | 1-1920 | 4.4 | 166.7 | 37.9 | <0.05 | 1.1E-16 |
| TBP | 3354 | 3250 | 129-9320 | 2090 | 1675 | 77-9260 | - | - | - | - | - |

| | | | | | | | | | | | |
|---|---|---|---|---|---|---|---|---|---|---|---|
| Relative expression¹: ratio (copy numbers biomarker/copy number TBP)*1000 MW²: Mann-Whitney test | | | | | | | | | | | |

**Table 6: Expression levels of the 17 biomarkers in normal bladder and BCa urine samples**

| | copy numbers | | | copy numbers | | | Fold Change | P-value |
|---|---|---|---|---|---|---|---|---|
| | NBl | | | BCa (NMIBC+MIBC) | | | | |
| | N=15 | | | N=85 | | | | |
| Biomarker | Mean | Median | Range | Mean | Median | Range | | MW¹ |
| CTHRC1 | 1764 | 1030 | 58-5140 | 17901 | 10200 | 12-190000 | 10.1 | <0.005 |
| IGF2BP2 | 9994 | 6160 | 94-34600 | 135158 | 36700 | 1040-4000000 | 13.5 | <0.005 |
| ADAMTS12 | 1 | 1 | 1-1 | 110 | 1 | 1-1490 | 110.0 | 0.037 |
| INHBA | 10487 | 4220 | 1-70300 | 373104 | 89400 | 64-6070000 | 35.6 | <0.005 |
| SFRP4 | 24 | 1 | 1-210 | 181 | 50 | 1-1520 | 7.5 | 0.007 |
| FAP | 8 | 1 | 1-106 | 758 | 107 | 1-21000 | 94.8 | <0.005 |
| CHI3L1 | 53324 | 24600 | 2220-368000 | 754615 | 234000 | 2900-5380000 | 14.2 | <0.005 |
| COL10A1 | 1296 | 160 | 1-5630 | 7700 | 6130 | 1-43300 | 5.9 | <0.005 |
| ASPN | 122 | 1 | 1-632 | 75 | 1 | 1-1000 | 0.6 | 0.881 |
| ANLN | 2283 | 832 | 1-10700 | 37092 | 13600 | 25-454000 | 16.2 | <0.005 |
| TPX2 | 946 | 521 | 1-3120 | 28382 | 8860 | 1-310000 | 30.0 | <0.005 |
| FOXM1 | 231 | 209 | 1-649 | 12502 | 4000 | 16-165000 | 54.1 | <0.005 |
| CCNB2 | 1951 | 1290 | 12-10000 | 30857 | 16000 | 1120-240000 | 15.8 | <0.005 |
| CDC20 | 3490 | 2600 | 189-11100 | 40674 | 15800 | 280-61200 | 11.7 | <0.005 |
| KRT6A | 140105 | 70000 | 1230-762000 | 70419 | 27200 | 13-576000 | 0.5 | 0.362 |
| PDCD1LG2 | 1869 | 877 | 1-8650 | 52224 | 16300 | 1-679000 | 27.9 | <0.005 |
| TC2526896 | 2876 | 2840 | 557-6590 | 4356 | 3100 | 73-28600 | 1.5 | 0.382 |
| TBP | 29783 | 21100 | 1160-94400 | 199980 | 161000 | 6000-950000 | - | |

**Table 7: Expression levels of the 17 biomarkers in NMIBC and MIBC urine samples**

| | copy numbers | | | copy numbers | | | Fold Change | P-value |
|---|---|---|---|---|---|---|---|---|
| | NMIBC | | | MIBC | | | | |
| | N=66 | | | N=19 | | | | |
| Biomarker | Mean | Median | Range | Mean | Median | Range | | MW¹ |
| CTHRC1 | 13991 | 9860 | 12-76100 | 31483 | 11400 | 1190-190000 | 2.3 | 0.143 |
| IGF2BP2 | 71976 | 31500 | 1040-1740000 | 365297 | 85250 | 2290-4000000 | 5.1 | 0.087 |
| ADAMTS12 | 71 | 1 | 1-790 | 253 | 1 | 1-1490 | 3.6 | 0.070 |
| INHBA | 288446 | 79500 | 64-3420000 | 667174 | 101000 | 13200-6070000 | 2.3 | 0.451 |
| SFRP4 | 153 | 13 | 1-919 | 276 | 123 | 1-1520 | 1.8 | 0.085 |
| FAP | 427 | 100 | 1-8390 | 1905 | 352 | 1-21000 | 4.5 | 0.023 |
| CHI3L1 | 689779 | 204000 | 2900-5050000 | 979837 | 394000 | 28300-5380000 | 1.4 | 0.117 |
| COL10A1 | 7596 | 6170 | 1-43300 | 8060 | 6130 | 409-28200 | 1.1 | 0.587 |
| ASPN | 54 | 1 | 1-436 | 151 | 1 | 1-1000 | 2.8 | 0.203 |
| ANLN | 31291 | 12450 | 25-454000 | 57243 | 21300 | 350-273000 | 1.8 | 0.083 |
| TPX2 | 16665 | 7180 | 1-188000 | 69081 | 14200 | 232-310000 | 4.1 | 0.017 |
| FOXM1 | 7218 | 3465 | 16-76800 | 30299 | 10600 | 101-156000 | 4.2 | 0.008 |
| CCNB2 | 22387 | 14900 | 1120-161000 | 60280 | 27300 | 1130-240000 | 2.7 | 0.014 |
| CDC20 | 22898 | 12700 | 396-184000 | 102422 | 23700 | 280-612000 | 4.5 | 0.013 |
| KRT6A | 6226 | 27650 | 166-500000 | 99574 | 26200 | 13-576000 | 16.0 | 0.780 |
| PDCD1LG2 | 38805 | 16200 | 1-679000 | 98840 | 22600 | 1900-477000 | 2.5 | 0.207 |
| TC2526896 | 4294 | 3185 | 73-27300 | 4571 | 2810 | 274-28600 | 1.1 | 0.609 |
| TBP | 189411 | 167000 | 6000-596000 | 236691 | 142000 | 9730-950000 | 1.2 | |

| | | | | | | | | |
|---|---|---|---|---|---|---|---|---|
| MW¹: Mann-Whitney test | | | | | | | | |

In **Table 4** the expression data of the 17 selected biomarkers in tissue are shown for the groups NBl and BCa total (NMIBC+MIBC). The difference (Fold-Change) between the groups and P-value provide information about/determine the diagnostic performance of the markers. In **Table 5** the data in tissue are shown for the groups NMIBC and MIBC and thereby provide information about the prognostic performance of the biomarkers. In **Tables 6 and 7** the data in the urine samples are shown.

### Summary results examples 1, 2 and 3

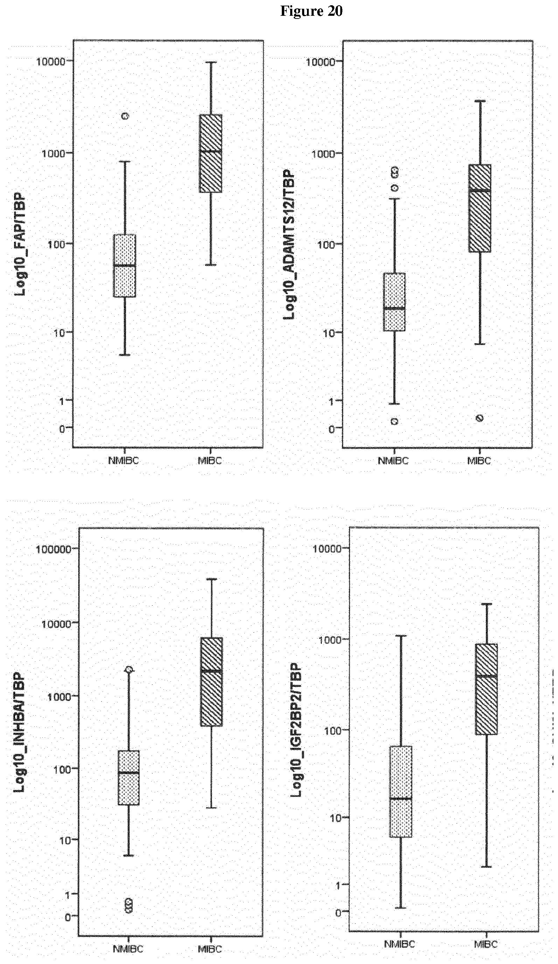

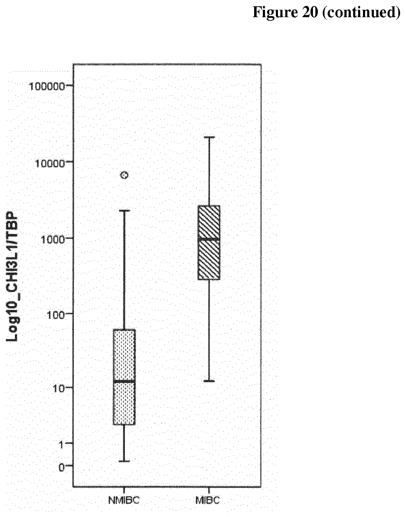

INHBA (**Figures 1****,** **20****; Tables 1, 4-7**): The present GeneChip® Human Exon 1.0 ST Array data, TLDA validation and qPCR assay data showed that INHBA was highly and significantly up-regulated in tissue from MIBC and BC-meta compared to NMIBC tissue. INHBA could also be detected in urine and was highly and significantly up-regulated in urine from BCa patients vs. normal urine. Therefore, INHBA has prognostic value in tissue of patients with BCa and diagnostic value in the detection of BCa in urine.

CTHRC1 (**Figure 2****, Tables 1, 4-7**): The present GeneChip® Human Exon 1.0 ST Array data, TLDA validation and qPCR assay data showed that CTHRC1 was highly and significantly up-regulated in tissue from MIBC and BC-meta compared to NMIBC. CTHRC1 could also be detected in urine and was significantly and highly up-regulated in urine from BCa patients vs. normal urine. Therefore, CTHRC1 has prognostic value in tissue of patients with BCa and diagnostic value in the detection of BCa in urine.

CHI3L1 (**Figures 3****,** **20****: Tables 1, 4-7**): The present GeneChip® Human Exon 1.0 ST Array data, TLDA validation and qPCR assay data showed that CHI3L1 was highly and significantly up-regulated in tissue from MIBC compared to NMIBC. CHI3L1 could also be detected in urine and was significantly and highly up-regulated in urine from BCa patients vs. normal urine. Therefore, CHI3L1 has prognostic value in tissue of patients with BCa and diagnostic value in the detection of BCa in urine.

COL10A1 (**Figures 4****, Tables 1, 4-7**): The present GeneChip® Human Exon 1.0 ST Array data, TLDA validation and qPCR assay data showed that COL10A1 was highly and significantly up-regulated in MIBC and BC-meta compared to NMIBC. COL10A1 could also be detected in urine and was significantly up-regulated in urine from BCa patients vs. normal urine. Therefore, COL10A1 has prognostic value in tissue of patients with BCa and diagnostic value in the detection of BCa in urine.

FAP (**Figures 5****,** **20****,** **21****; Tables 1, 4-7**): The present GeneChip® Human Exon 1.0 ST Array data, TLDA validation and qPCR assay data showed that FAP was highly and significantly up-regulated in tissue from MIBC and BC-meta compared to NMIBC. FAP could also be detected in urine and was significantly and highly up-regulated in urine from BCa patients vs. normal urine and significantly up-regulated in urine from MIBC patients vs. NMIBC patients. Therefore, FAP has prognostic value in urine and in tissue of patients with BCa and diagnostic value in the detection of BCa in urine.

Transcript cluster 2526896 (**Figures 6****, Tables 1, 4-7**): The present GeneChip® Human Exon 1.0 ST Array data, and qPCR assay data showed that transcript cluster TC2526896 was highly and significantly up-regulated in tissue from MIBC and BC-meta compared to NMIBC. Therefore, transcript cluster 2526896 has prognostic value in tissue of patients with BCa.

ASPN (**Figure 7****, Tables 1, 4-7**): The present GeneChip® Human Exon 1.0 ST Array data, TLDA validation and qPCR assay data showed that ASPN was highly and significantly up-regulated in tissue from MIBC compared to NMIBC. Therefore, ASPN has prognostic value in tissue of patients with BCa.

Transcript cluster 2526893 (**Figure 8**): The present GeneChip® Human Exon 1.0 ST Array data showed that transcript cluster 2526893 was highly up-regulated in tissue from MIBC and BC-meta compared to NMIBC. Therefore, transcript cluster 2526893 has prognostic value in tissue of patients with BCa.

ADAMTS12 (**Figures 9****,** **20****; Tables 1, 4-7**): The present GeneChip® Human Exon 1.0 ST Array data, TLDA validation and qPCR assay data showed that ADAMTS12 was highly and significantly up-regulated in tissue from MIBC and BC-meta compared to NMIBC. Low copy numbers of ADAMTS12 could also be detected in urine. ADAMTS12 was significantly up-regulated in urine from BCa patients vs. normal urine and significantly up-regulated in urine from MIBC patients vs. NMIBC patients. Therefore, ADAMTS12 has prognostic value in urine and tissue of patients with BCa and diagnostic value in the detection of BCa in urine.

IGF2BP2 (**Figures 10****,** **20****; Tables 1, 4-7**): The present GeneChip® Human Exon 1.0 ST Array data, TLDA validation and qPCR assay data showed that IGF2BP2 was highly and significantly up-regulated in tissue from MIBC compared to NMIBC. IGF2BP2 could also be detected in urine and was significantly and highly up-regulated in urine from BCa patients vs. normal urine. Therefore, IGF2BP2 has prognostic value in tissue of patients with BCa and diagnostic value in the detection of BCa in urine.

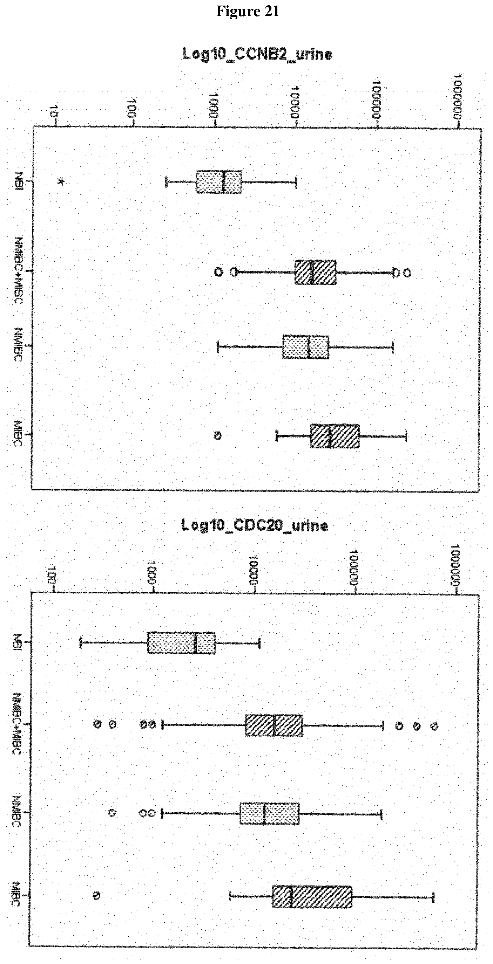

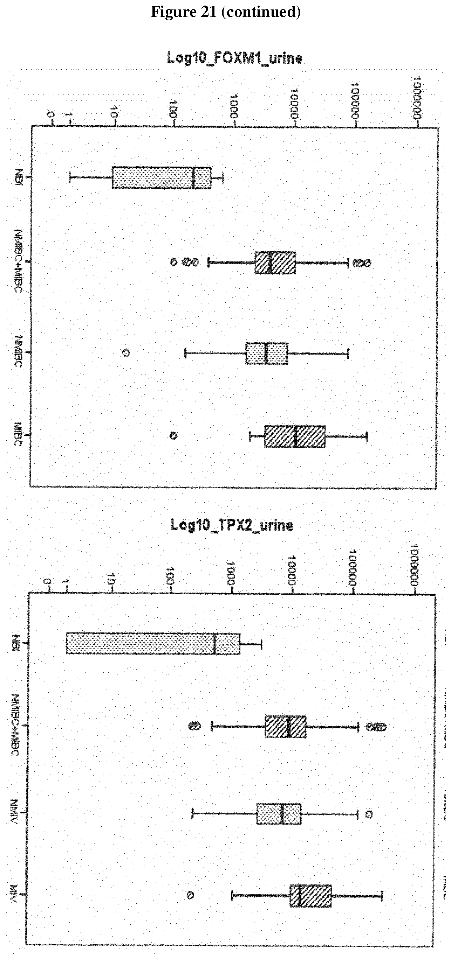

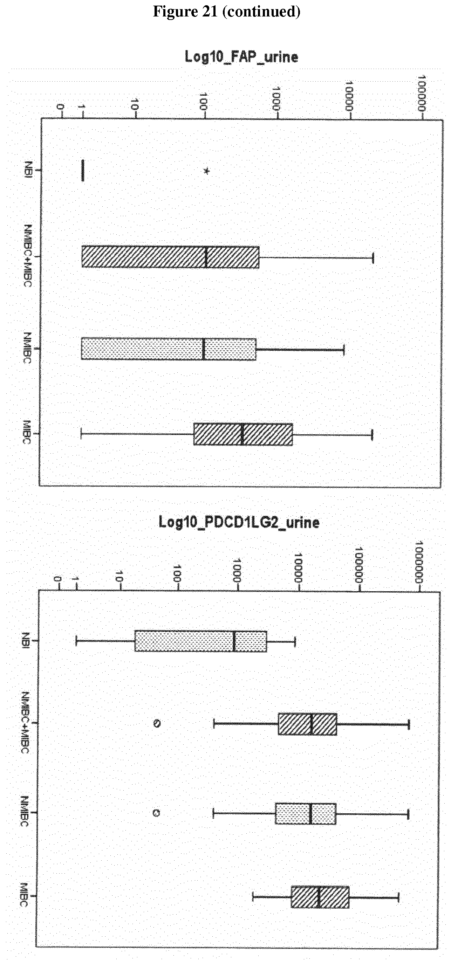

PDCD1LG2 (**Figures 11****,** **21****; Tables 1, 4-7**): The present GeneChip® Human Exon 1.0 ST Array data, TLDA validation and qPCR assay data showed that PDCD1LG2 was significantly up-regulated in tissue from MIBC and BC-meta compared to NMIBC. PDCD1LG2 could also be detected in urine and was significantly and highly up-regulated in urine from BCa patients vs. normal urine. Therefore, PDCD1LG2 has prognostic value in tissue of patients with BCa and diagnostic value in the detection of BCa in urine.

SFRP4 (**Figure 12****; Tables 1, 4-7**): The present GeneChip® Human Exon 1.0 ST Array data, TLDA validation and qPCR assay data showed that SFRP4 was highly and significantly up-regulated in tissue from MIBC and BC-meta compared to NMIBC. Low copy numbers of SFRP4 could also be detected in urine. SFRP4 was significantly up-regulated in urine from BCa patients vs. normal urine. Therefore, SFRP4 has prognostic value in tissue of patients with BCa and diagnostic value in the detection of BCa in urine.

KRT6A (**Figure 13****; Tables 1, 4-7**): The present GeneChip® Human Exon 1.0 ST Array data, TLDA validation and qPCR assay data showed that KRT6A was highly and significantly up-regulated in tissue from MIBC compared to NMIBC. Therefore, KRT6A has prognostic value in tissue of patients with BCa.

TPX2 (**Figures 14****,** **19****,** **21****; Tables 1, 4-7**): The present GeneChip® Human Exon 1.0 ST Array data, TLDA validation and qPCR assay data showed that TPX2 was highly and significantly up-regulated in tissue as well as in urine from patients with BCa compared to normal bladder and significantly up-regulated in tissue and urine from MIBC and BC-meta patients compared to NMIBC patients. Therefore, TPX2 has diagnostic value in tissue and in the detection of BCa in urine and has prognostic value in urine and in tissue of patients with BCa.

CCNB2 (**Figures 15**, **19****,** **21****; Tables 1, 4-7**): The present GeneChip® Human Exon 1.0 ST Array data, TLDA validation and qPCR assay data showed that CCNB2 was highly and significantly up-regulated in tissue as well as in urine from patients with BCa compared to NBl and significantly up-regulated in tissue and urine from MIBC and BC-meta patients compared to NMIBC patients. Therefore, CCNB2 has diagnostic value in tissue and in the detection of BCa in urine and has prognostic value in urine and in tissue of patients with BCa.

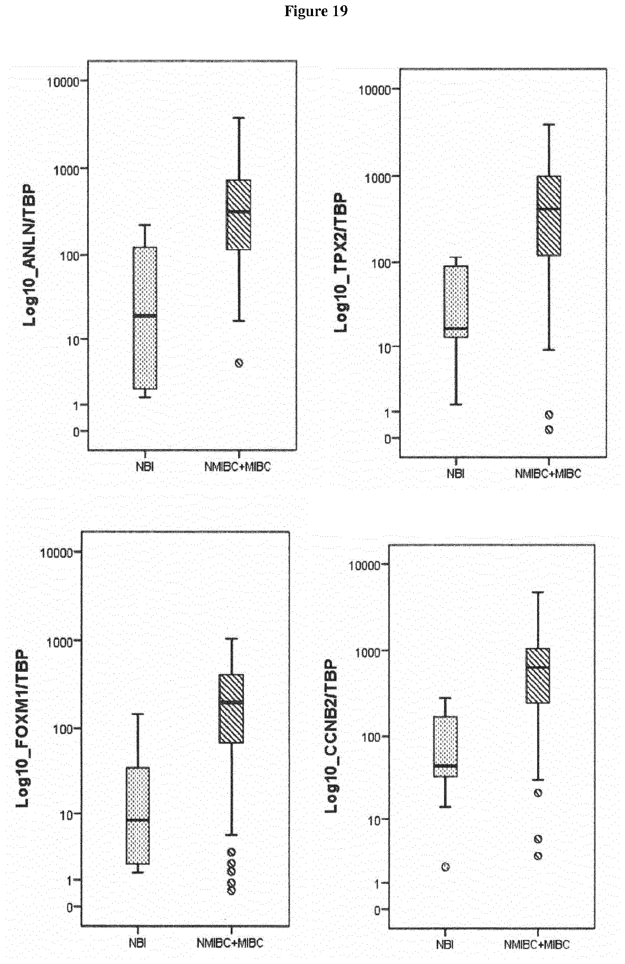

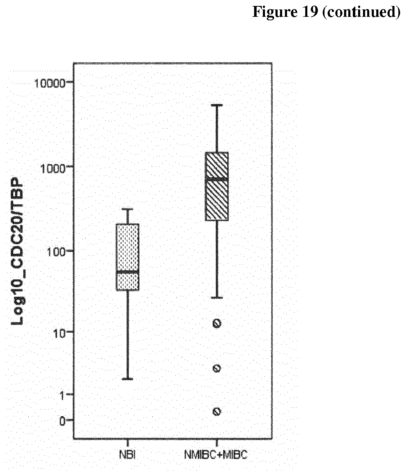

ANLN (**Figures 16****,** **19****; Tables 1, 4-7**): The present GeneChip® Human Exon 1.0 ST Array data, TLDA validation and qPCR assay data showed that ANLN was highly and significantly up-regulated in tissue as well as in urine from patients with BCa compared to NBl and significantly up-regulated in tissue from MIBC and BC-meta patients compared to NMIBC patients. Therefore, ANLN has diagnostic value in tissue and in the detection of BCa in urine and has prognostic value in tissue of patients with BCa.

FOXM1 (**Figures 17****,** **19****,** **21****; Tables 1, 4-7**): The present GeneChip® Human Exon 1.0 ST Array data, TLDA validation and qPCR assay data showed that FOXM1 was highly and significantly up-regulated in tissue as well as in urine from patients with BCa compared to NBl and significantly up-regulated in tissue and urine from MIBC and BC-meta patients compared to NMIBC patients. Therefore, FOXM1 has diagnostic value in tissue and in the detection of BCa in urine and has prognostic value in urine and in tissue of patients with BCa.

CDC20 (**Figures 18****,** **19****,** **21****; Tables 1, 4-7**): The present GeneChip® Human Exon 1.0 ST Array data, TLDA validation and qPCR assay data showed that CDC20 was highly and significantly up-regulated in tissue as well as in urine from patients with BCa compared to NBl and significantly up-regulated in tissue and urine from MIBC and BC-meta patients compared to NMIBC patients. Therefore, CDC20 has diagnostic value in tissue and in the detection of BCa in urine and has prognostic value in urine and in tissue of patients with BCa.

### Example 4: Selection of the best candidate biomarkers

Based on the highest up-regulation in BCa vs NBl and MIBC vs. NMIBC, lowest P-value and high copy numbers the best performing diagnostic and prognostic individual biomarkers in tissue and urine were identified. The five best performing individual biomarkers for the detection of BCa in tissue were identified and are shown in boxplots in **Figure 19****:** ANLN, TPX2, FOXM1, CCNB2 and CDC20.

The five best performing individual biomarkers that could distinguish MIBC tissue from NMIBC tissue were identified and are shown in boxplots **in** **Figure 20****:** IGF2BP2, INHBA, ADAMTS12 FAP and CHI3L1.

The six best performing individual biomarkers for the detection of BCa in urine were identified and are shown in a boxplot in **Figure 21****:** FAP, TPX2, CCNB2, CDC20, FOXM1 and PDCD1LG2. The first five genes could also significantly distinguish MIBC from NMIBC in urine.

Given that the nature of these tumors is very heterogeneous, it is likely that combination of markers can identify different patients and have additional diagnostic and/or prognostic value to eachother. For the identification of the best combinations of biomarkers for the diagnosis of BCa in urine and/or tissue and for the best combinations of markers that had prognostic value by distinguishing MIBC from NMIBC in tissue and/or urine the method of binary logistic regression analysis was performed. All data were log-transformed prior to statistical analysis as a transformation to a normal distribution. Binary logistic regression analysis (stepwise forward) was performed with the 17 biomarkers in order to find regression models and marker combinations for predicting the presence of bladder cancer (NMIBC and MIBC) in urine or for predicting whether BCa is muscle invasive or not. The statistical significant level for all tests was set at P=0.05.

As example two possible identified combinations of biomarkers are described, one for predicting the occurrence of BCa based on the expression of the markers in urine and one for predicting the occurence of muscle invasive disease based on the expression of the markers in tissue.

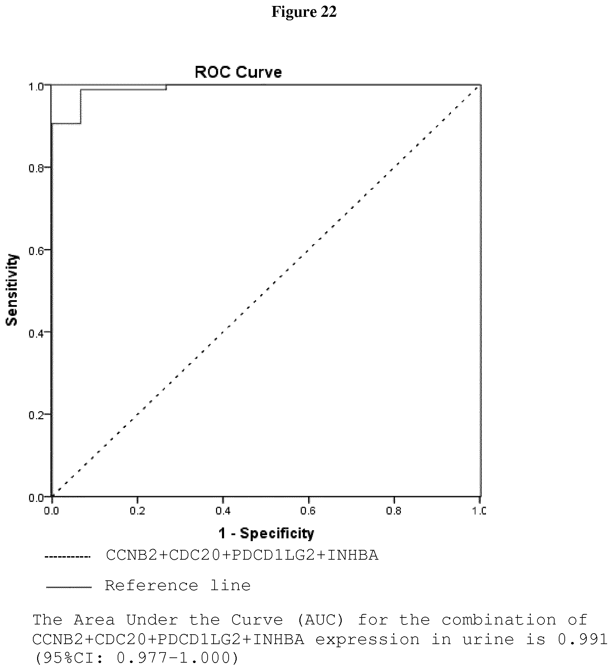

In urine, CCNB2 is a key predictor and predicts that 66.7% of healthy controls have no cancer and that 96.5% from the cancer patients do have cancer. With the addition of CDC20 and PDCD1LG2 the new model predicts that 80% of the healthy controls have no cancer and 96.5% of the cancer patients are correctly classified. When INHBA is added to this model the model model predicts that 93.3% of the healthy controls have no cancer and that 98.8% of the cancer patients are correctly calssified. This four biomarker model is higly significant (P=1.9E-13) showing that the biomarkers can predict the presence of bladdercancer in urine well. To visualize the performance of the biomarker combinations a ROC curve is shown (**Figure 22**).

In a ROC curve, the true positive rate to detect BCa or MIBC (sensitivity) is plotted in function of the false positive rate (i.e. positives in the control group, 1-specificity) for different cut-off points. Each point on the curve represents a sensitivity/specificity pair corresponding to a particular decision threshold. The Area Under the Curve (AUC) of the ROC curve is a measure how well a parameter can distinguish between two groups and is maximum 1.0 (all samples correctly classified). The AUC for the combination of CCNB2, CDC20, PDCD1LG2 and INHBA expression is 0.991 (95%CI: 0.977-1.000).

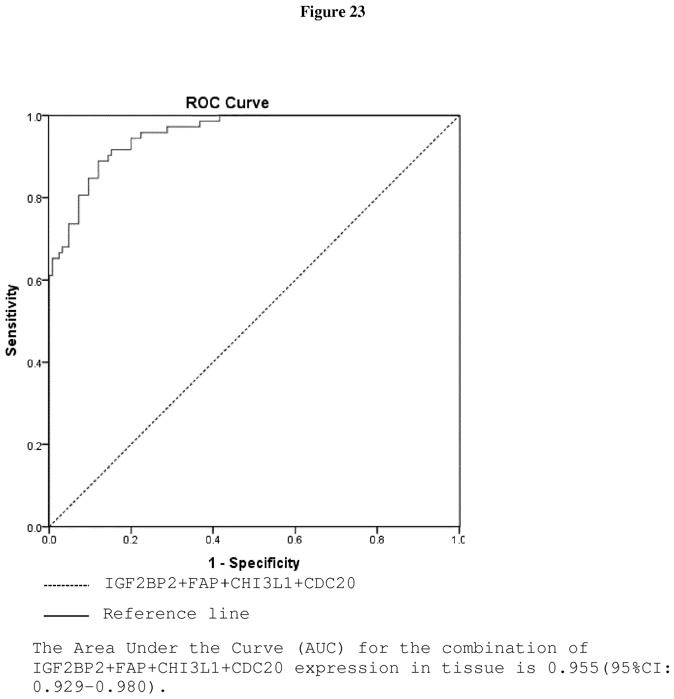

In tissue, FAP is a key predictor and predicts that 87% of the NMIBC are NMIBC and that 80.3% of the MIBC specimen are correctly classified. When CDC20 and CHI3L1 are added 89.3 of the NMIBC are correctly classified and 83.1% of the MIBC are correctly classified. The addition of IGF2BP2 leads to the correct classification of 90.2% of the NMIBC and 83.1% of the MIBC. This four marker model is higly significant (P=4.9 x10-32) showing that the biomarkers can predict the occurence of muscle invasive disease well. The Area Under the Curve (AUC) for the combination of IGF2BP2+FAP+CHI3L1+CDC20 expression is 0.955 (95%CI: 0.929-0.980). See **Figure 23**.

Based on the binary logistic regression model the following genes and combinations were identified. For predicting the occurrence of BCa (diagnosis) based on the detection and quantification expression of the markers in tissue: at least ANLN combined with one or more markers from the list: IGF2BP2, FAP, CTHRC1, CCNB2, COL10A1 and/or TPX2.
For predicting the occurence of muscle invasive disease (prognosis) based on the expression of the markers in tissue: at least FAP, combined with one or more markers from the list: CDC20, CHI3L1, IGF2BP2, INHBA, ADAMTS12, CCNB2 and/or ANLN or at least CHI3L1, combined with one or more markers from the list: CDC20, FAP, IGF2BP2, INHBA, ADAMTS12, CCNB2 and/or ANLN.

For predicting the occurrence of BCa based on the expression of the markers in urine at least CCNB2, combined with one or more markers from the list: CDC20, PDCD1LG2, TPX2, SFRP4, COL10A1, INHBA and/or TC2526896 or at least PDCD1LG2, combined with one or more markers from the list: CCNB2, CDC20, TPX2, SFRP4, COL10A1, INHBA and/or CTHRC1

For predicting the occurence of muscle invasive disease based on the expression of the markers in urine at least FAP, combined with one or more from the list FOXM1, CCNB2, CDC20 and/or TC2526896

### CONCLUSIONS:

The present invention relates to biomarkers and their diagnostic and prognostic uses for bladder cancer. The biomarkers can be used alone or in combination. The invention provides methods for diagnosing bladder cancer in a subject, comprising measuring the levels of a single or a plurality of biomarkers in a biological sample derived from a subject suspected of having bladder cancer. Differential expression of one or more biomarkers in the biological sample is compared to one or more biomarkers in a healthy control sample indicates that a subject has cancer. Furthermore, the invention provides methods for determing classification of tumors according to the aggressiveness or establishing the prediction of prognosis and disease outcome for a human individual suffering from bladder cancer, comprising measuring the levels of a single or a plurality of biomarkers in a biological sample derived from a subject having bladder cancer. Differential expression of one or more biomarkers in the biological sample is compared to one or more biomarkers in a NMIBC control sample that indicates that a subject has an aggressive type of bladder cancer.

Based on the results obtained and described in examples 1, 2,3 and 4, the following observations can be made:
1) Given that the biological sample is urine, the identified best performing individual biomarkers for diagnosis of BCa were: FAP, TPX2, CCNB2, CDC20, FOXM1 and PDCD1LG2. The first five markers could also significantly distinguish MIBC from NMIBC in urine and therefore had prognostic value.
2) The best combinations of biomarkers for predicting the occurrence of BCa based on the expression of the markers in urine contain at least CCNB2, combined with one or more markers from the list: CDC20, PDCD1LG2, TPX2, SFRP4, COL10A1, INHBA and/or TC2526896; or contain at least: PDCD1LG2, combined with one or more markers from the list: CCNB2, CDC20, TPX2, SFRP4, COL10A1, INHBA and/or CTHRC1;
3) The best combination of biomarkers for predicting the occurrence of muscle invasive disease based on the expression of the markers in urine contains at least FAP, combined with one or more from the list FOXM1, CCNB2, CDC20 and/or TC2526896;
4) Given that the biological sample is tissue, the identified best performing individual biomarkers for diagnosis of BCa were: ANLN, TPX2, FOXM1, CCNB2 and CDC20;
5) The identified best performing individual biomarkers that could distinguish MIBC tissue from NMIBC tissue were: IGF2BP2, INHBA, ADAMTS12 FAP and CHI3L1;
6) The best combination of biomarker s for predicting the occurrence of BCa based on the expression of the markers in tissue contains at least ANLN combined with one or more markers from the list: IGF2BP2, FAP, CTHRC1, CCNB2, COL10A1 and/or TPX2;
7) The best combinations of biomarkers for predicting the occurrence of muscle invasive disease based on the expression of the markers in tissue contain at least FAP, combined with one or more markers from the list: CDC20, CHI3L1, IGF2BP2, INHBA, ADAMTS12, CCNB2 and/or ANLN or at least CHI3L1, combined with one or more markers from the list: CDC20, FAP, IGF2BP2, INHBA, ADAMTS12, CCNB2 and/or ANLN

### CLAUSES

1. Method, preferably an *in vitro* method, for establishing the presence, or absence, of a bladder tumour in a human individual; or establishing the prediction of prognosis and disease outcome for a human individual suffering from bladder cancer comprising:
   a) determining the expression of one or more genes chosen from the group consisting of CCNB2, ADAMTS12, ASPN, CDC20, COL10A1, CTHRC1, FAP, SFRP4, FOXM1, KRT6A, ANLN, CHI3L1, TPX2, IGF2BP2, INHBA, PDCD1LG2, transcript cluster 2526893, and transcript cluster 2526896 in a sample originating from said human individual; and
   b) establishing up regulation of expression of said one or more genes as compared to expression of said respective one or more genes in a sample originating from said human individual not comprising tumour cells or tissue, or from an individual, or group of individuals, not suffering from bladder cancer; and
   c) establishing the presence, or absence, of a bladder tumour based on the established up- or down regulation of said one or more genes; or establishing the prediction of prognosis and disease outcome for a human individual suffering from bladder cancer based on the established up- or down regulation of said one or more genes.
2. Method according to clause 1, wherein establishing the presence, or absence, of bladder cancer in a human individual preferably includes diagnosis, prognosis and/or prediction of disease survival.
3. Method according to clause 1 or clause 2, wherein the method is an *ex vivo* or *in vitro* method.
4. Method according to clause 3, wherein expression analysis is performed on a sample selected from the group consisting of body fluid, saliva, lymph, blood, urine, tissue sample and a transurethral resection of a bladder tumour (TURBT), preferably blood, urine, urine desiment, and samples of, derived or originating from TURBT specimens.
5. Method according to any of the clauses 1 to 4, wherein determining the expression comprises determining mRNA expression of the one or more genes, preferably by Northern blot hybridisation or amplification based techniques, preferably PCR, real time PCR, or NASBA.
6. Method according to any of the clauses 1 to 5, wherein expression analysis comprises high-throughput array chip analysis.
7. Method according to any of the clauses 1 to 5, wherein expression analysis comprises determining protein levels of the said genes, preferably by matrix-assisted laser desorption-ionization time-of-flight mass spectrometer (MALDI-TOF).
8. Use of expression analysis of one or more genes selected from the group consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and transcript cluster 2526896 for establishing the presence, or absence, of a bladder tumour or establishing the prediction of prognosis and disease outcome for an individual patient suffering from bladder cancer.
9. Kit of parts for establishing the presence, or absence, of a bladder tumour and establishing the prediction of prognosis and disease outcome for an individual patient suffering from bladder cancer said kit of parts comprises:
   - expression analysis means for determining the expression of one or more genes chosen from the group consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and transcript cluster 2526896;
   - instructions for use.

## Claims

1. Method for establishing the presence, or absence, of a bladder tumour in a human individual; or establishing the prediction of prognosis and disease outcome for a human individual suffering from bladder cancer comprising:
a) determining the expression of one or more genes chosen from the group consisting of ANLN, TPX2, FOXM1, CCNB2, CDC20, ADAMTS12,ASPN, COL10A1, CTHRC1, FAP, SFRP4, , KRT6A, CHI3L1, , IGF2BP2, INHBA, PDCD1LG2, transcript cluster 2526893, and transcript cluster 2526896 in a sample originating from said human individual wherein said sample is a tissue sample or a transurethral resection of a bladder tumour (TURBT); and
b) establishing up regulation of expression of said one or more genes as compared to expression of said respective one or more genes in a sample originating from said human individual not comprising tumour cells or tissue, or from an individual, or group of individuals, not suffering from bladder cancer; and
c) establishing the presence, or absence, of a bladder tumour based on the established up- or down regulation of said one or more genes; or establishing the prediction of prognosis and disease outcome for a human individual suffering from bladder cancer based on the established up- or down regulation of said one or more genes.

2. Method according to claim 1, wherein establishing the presence, or absence, of bladder cancer in a human individual preferably includes diagnosis, prognosis and/or prediction of disease survival.

3. Method according to claim 1 or claim 2, wherein the method is an *ex vivo* or *in vitro* method.

4. Method according to any of the claims 1 to 3, wherein determining the expression comprises determining mRNA expression of the one or more genes, preferably by Northern blot hybridisation or amplification based techniques, preferably PCR, real time PCR, or NASBA.

5. Method according to any of the claims 1 to 4, wherein expression analysis comprises high-throughput array chip analysis.

6. Method according to any of the claims 1 to 4, wherein expression analysis comprises determining protein levels of the said genes, preferably by matrix-assisted laser desorption-ionization time-of-flight mass spectrometer (MALDI-TOF).
